# EUROPÄISCHE PATENTANMELDUNG

(11) **EP 1 108 779 A2**
(43) Veröffentlichungstag der Anmeldung: **20.06.2001**
(21) Anmeldenummer: 00127369.7
(22) Anmeldetag: 13.12.2000
(51) Int. Cl.: C12N 9/64, C12N 15/55

(54) **Raumform und Kristallform von Polypeptiden, die eine Sub-Domäne des Calpains enthalten**

(30) Priorität: 14.12.1999 DE 19960225
(71) Anmelder: Max-Planck-Gesellschaft zur Förderung der Wissenschaften e.V., 80539 München (DE); proteros biostructures GbmH, 82152 Martinsried (DE)
(72) Erfinder: Strobl, Stefan, Dr., 82152 Planegg-Martinsried (DE); Fernandez-Catalan, Carlos, Dr., 28039 Madrid (ES); Bode, Wolfram, Prof. Dr., 82131 Gauting (DE); Huber, Robert, Prof. Dr., 82110 Germering (DE); Suzuki, Koichi, Prof. Dr., Tokyo 168-0064 (JP)
(74) Vertreter: Graf von Stosch, Andreas, Dr. rer. nat.

(57) **Zusammenfassung**

Die vorliegende Erfindung betrifft Raumstrukturen und Kristallform mindestens eines Polypeptids pro asymmetrischer Einheit, wobei mindestens ein Polypeptid in der asymmetrischen Einheit mindestens eine an der Katalyse beteiligte (Sub)-Domäne eines Proteins aus der Familie der neutralen Ca-aktivierten Cystein-Proteinasen (Calpaine) aufweist. Weiterhin betrifft die vorliegende Erfindung Verfahren zur Identifizierung eines Liganden für eine neutrale Ca-aktivierte Cystein-Proteinase.

## Beschreibung

Die vorliegende Erfindung betrifft Raumstrukturen und Kristallform mindestens eines Polypeptids pro asymmetrischer Einheit, wobei mindestens ein Polypeptid in der asymmetrischen Einheit mindestens eine an der Katalyse beteiligte (Sub)-Domäne eines Proteins aus der Familie der neutralen Ca-aktivierten Cystein-Proteinasen (Calpaine) aufweist. Weiterhin betrifft die vorliegende Erfindung Verbindungen, insbesondere Liganden, mit der Eigenschaft als Substrat, Pseudosubstrat, Aktivator oder Inhibitor einer neutralen Ca-aktivierten Cystein-Proteinase (Calpaine) zu wirken, und Verfahren zur Identifizierung einer solchen Verbindung bzw. eines solchen Liganden für eine neutrale Ca-aktivierte Cystein-Proteinase. Im Rahmen der vorliegenden Erfindung wird auch die Verwendung derartiger Liganden bzw. Verbindungen, die als Inhibitoren und/oder Aktivatoren der katalytischen Aktivität einer neutralen Ca-aktivierten Cystein-Proteinase wirken, als Wirkstoff in Arzneimitteln bzw. zur Herstellung eines Arzneimittels offenbart. Schließ-lich gehören auch Verfahren zur Herstellung einer Kristallform mit mindestens einem Polypeptid, das mindestens eine an der Katalyse beteiligte (Sub)-Domäne eines Proteins aus der Familie der neutralen Ca-aktivierten Cystein-Proteinasen aufweist, zum Gegenstand der vorliegenden Erfindung. Ein weiterer Erfindungsgegenstand der vorliegenden Erfindung sind Verfahren, die das Modellieren von Calpainen unbekannter Struktur unter Verwendung von Strukturkoordinaten einer erfindungsgemäßen Raumstruktur oder Kristallform erlauben.

Die sogenannten Calpaine gehören zu einer Familie von in-trazellulären, Ca-abhängigen Cystein-Proteinasen, die sowohl mehrere Gewebe-spezifische Isoformen (n-Calpaine) als auch zwei ubiquitär auftretende Isozyme (µ- und m-Calpain) umfassen. Calpain gehört in die Enzymklasse EC 3.4.22.17, wobei es sich um ein Enzym, das als Heterodimer vorliegt, aufgebaut aus einer großen katalytischen und einer kleinen regulatorischen Untereinheit (Ono et al., Biochem. Biophys. Res. Com. 245, 289-294, 1998) handelt. Entsprechende Untersuchungen haben ergeben, daß die große Untereinheit ein Molekulargewicht von ungefähr 80.000, die kleine Untereinheit von ungefähr 30.000 Dal-ton hat.

Ohno et al. (Nature 312, 566-570, 1984) haben die molekulare Primärstruktur von µ-/m-Calpain aus dem Hühnchen durch cDNA-Klonierung beschrieben. Dabei wurde die große katalytische Untereinheit des Calpain-Heterodimers in Domänen mit den Bezeichnungen I, II, III und IV, die kleine regulatorische Untereinheit in zwei Domänen mit den Bezeichnungen V und VI unterteilt. Jede der beiden Untereinheiten weist eine Calmodulin-artige Ca²⁺-Bindungsdomäne auf, die jeweils am C-Terminus zu finden ist (Domänen IV und VI). Die Domäne II der großen Untereinheit, die wiederum in zwei (Sub)-Domänen zerfällt (IIa und IIb) läßt Sequenzähnlichkeiten zu katalytisch aktiven Domänen anderer Cystein-Proteinasen, wie z.B. Papain und Cathepsinen, erkennen. Gleichwohl besteht, abgesehen von drei Aminosäureresten im aktiven Zentrum der katalytischen (Sub)-Domänen von Calpainen, keine ausgeprägte Sequenzhomologie zu anderen Cystein-Proteinasen, weswegen die Calpaine als evolutionär separierte, eigenständige Familie innerhalb der Großfamilie der Cystein-Proteinasen angesehen werden (Berti und Stora, J. Mol. Biol. 246, 273-283, 1995). Eine regulatorische Wirkung auf die katalytische Aktivität von Calpain wurde den Calmodulin-artigen Calcium-Bindungsdomänen an den jeweiligen C-Termini der katalytischen bzw. der regulatorischen Untereinheit zugeschrieben (Suzuki et al., Biol. Chem. Hoppe-Seyler, 376, 523-529, 1995).

Die proteolytische Funktion von Calpainen ist für die Zellphysiologie von höchster Bedeutung. Den ubiquitär und konstitutiv exprimierten µ- und m-Calpainen bspw. wurde eine zentrale Rolle bei der Regulierung zellulärer Funktionen zugeschrieben. Dagegen scheinen Gewebe-spezifischen Calpain-Homologe (etwa n-Calpaine) für die jeweilige Gewebeentwicklung, -funktion und den Gewebebe-stand von zentraler Bedeutung zu sein (Sorimachi et al., J. Biol. Chem. 264, 20106-20111, 1989). So gibt es etwa Hinweise darauf, daß muskelspezifisches Calpain an der Entstehung von Muskeldystrophie beteiligt ist (Richard et al., Cell, 81, 27-40, 1995). Weiterhin scheint die Bildung von Plaques bei Alzheimer-Patienten auf eine Deregu-lierung von µ-Calpain und seines physiologischen Inhibitors Calpastatin zurückzuführen zu sein (Saito et al., PNAS USA, 90, 2628-2632, 1993). Derart wird das für die Alzheimer-Krankheit charakteristische, abnormale Prozes-sieren von Transmembran-Amyloid-Precursor-Protein, das schließlich zur Selbstaggregation von β-Amyloidpeptiden führt, auf extra- und intrazelluläre proteolytische Akt-vitäten zurückgeführt, wobei eine verlorengegangene Balance zwischen intaktem und autolysiertem µ-Calpain ursächlich sein mag. Da auch an der Kataraktbildung Calpain offenbar beteiligt ist (David et al., J. Biochem. 268, 1937-1940, 1993), sollten Ergebnisse dreidimensionaler Strukturaufklärungen, bspw. anhand entsprechender Raum- oder Kristallformen, nähere Einblicke in die Funktionsweise und die Art der Regulation von Calpainen schaffen.

Diverse Versuche zur Überexpression, Kristallisierung und/oder Röntgenstrukturanalyse zeigen das diesbezügliche Interesse an der Aufklärung der Struktur-/Funktions-beziehung bei Calpainen. So etwa haben Blanchard et al. (Nature Structural Biology, Vol. 4, No. 7, 532-538, 1997) die Calcium-Bindungsdomäne der kleinen Untereinheit von Ratten-Calpain (Domäne VI) kloniert, exprimiert, gereinigt und schließlich kristallisiert (Graham-Siegenthaler et al., J. Biochem., 269, 30457-30460, 1994). Diese isolierte Domäne VI liegt in Lösung als Homodimer vor und wurde in der Raumgruppe C222₁ kristallisiert. Die Kristalle weisen zwei Monomere pro asymmetrischer Einheit auf. Zwar wurde durch die Strukturaufklärung der Domäne VI deutlich, daß ein Faltungsmuster von fünf EF-Händen, einem charakteristischen α-Helices aufweisenden Supersekun-därstrukturmuster, pro Monomer vorliegt, von denen drei wiederum Calcium bei physiologischen Calcium-Konzentrationen binden, doch erlaubt die bei Blanchard et al. dargestellte Struktur nicht, einen funktionellen oder strukturellen Zusammenhang zwischen der Calcium-Bindung und deren Wirkung auf die katalytischen (Sub)-Domänen in der großen Untereinheit von Calpain aufzuzeigen. Die Strukturkoordinaten der von Blanchard et al. gelösten Kristallstruktur sind in der PDB-Datenbank (Brookhaven, USA) unter den Bezeichnungen 1AJ5 und 1DVI hinterlegt.

Lin et al. (Nature Structural Biology, Vol. 4, No. 7, 539-547, 1997) beschreiben gleichfalls die Kristallisierung der Domäne VI, d.h. der Calcium-Bindungsdomäne der kleinen Untereinheit von Schweine-Calpain, bei einer Strukturauflösung von 1,9 Å. Auch diese Untersuchung beschränkt sich damit auf die Strukturaufklärung der Calcium-Bindungsdomäne und läßt keine Aussage über die Struktur der katalytischen Untereinheit bzw. deren Wirkung auf den Regulationsmechanismus der katalytischen Untereinheit selbst zu. Lin et al. haben ihre Kristallstrukturen unter den Bzeichnungen 1ALV und 1ALW in der PDB-Datenbank (Brookhaven, USA) hinterlegt.

Schließlich wurde gezeigt, daß sich m-Calpain der Ratte in zwei Kristallformen, P1 und P2₁, kristallisieren läßt (Hosfield et al., Acta Crystallographica Section D, Bio-logical Crystallography, D55, 1484-1486, 1999). Das verwendete rekombinante Ratten-m-Calpain unterscheidet sich geringfügig vom natürlichen Enzym. Der im aktiven Zentrum residierende native Aminosäurerest Cys105 wurde im rekombinantem Protein zu einem Serin mutiert, wodurch die Aktivität des Enzyms ausgeschaltet und damit die Autodegra-dation vermieden wurde. Zudem wurden die große Untereinheit mit 14 Aminosäureresten am C-Terminus, einschließ-lich eines Histidin-Tags, versehen. Zwar berichten Hosfield et al. über eine röntgenkristallographische Datensammlung mit Auflösungen für die erhaltenen Kristalle von bis zu 2,6 bzw. 2,15 Å, doch offenbaren die Autoren keine Kristallformen, also keine strukturellen Ergebnisse der röntgenkristallographischen Datenerhebung. Zudem wurden die Untersuchungen von Hosfield et al. ausschließlich für m-Calpain der Ratte, nicht aber für humanes m-Calpain durchgeführt.

Masumoto et al. (J. Biochem. 124, 957-961, 1998) beschreiben zwar eine Überproduktion von rekombinantem humanen Calpain in aktiver Form in einem Baculovirus-Expressionssystem und dessen Reinigung und Charakterisierung, doch können die Autoren weder über eine Kristallisierung noch über eine Strukturaufklärung des überexpri-mierten humanen m-Calpains berichten.

Aufgabe der vorliegenden Erfindung ist es daher, Raum-und vorzugsweise Kristallformen von Calpainen zur Verfügung zu stellen, die eine Struktur-/Funktionsuntersuchung möglich machen. Hierzu bedarf es einer 3D-Strukturaufklärung eines Polypeptids oder eines Komplexes, das (der) mindestens eine an der Katalyse des nativen Calpains beteiligte (Sub)-Domäne aufweist, ggf. auch weitere katalytische und/oder regulatorische (Sub)-Domänen einer oder beider Untereinheiten. Eine weitere Aufgabe der vorliegenden Erfindung ist es, solche Verbindungen zur Verfügung zu stellen, die als Substrate, Pseu-dosubstrate, Aktivatoren, oder Inhibitoren einer neutralen Ca-aktivierten Cystein-Proteinase wirken können. Darüber hinaus hat die vorliegende Erfindung die Aufgabe, Verfahren zur Identifizierung einer Verbindung, die als Agonist oder Antagonist bzw. Substrat, Pseudosubstrat, Aktivator oder Inhibitor für ein oder mehr Calpain(e) fungieren kann, zur Verfügung zu stellen. Daneben liegt der vorliegenden Erfindung auch die Aufgabe zugrunde, Verfahren zur Herstellung einer Kristallform mit mindestens einem Polypeptid, das mindestens eine an der Katalyse beteiligte (Sub)-Domäne eines Proteins aus der Familie der Calpaine aufweist, bereitzustellen. Neben derartigen Kristallisierungsverfahren hat die vorliegende Erfindung auch die Aufgabe, Kristalle zur Verfügung zu stellen, die die vorgenannten Polypeptide in symmetrischer Anordnung aufweisen. Schließlich stellt sich vorliegend die Aufgabe, solche Verfahren zur Verfügung zu stellen, die dazu dienen, dreidimensionale Strukturen von bisher strukturell nicht aufgeklärten Proteinen (Polypeptiden) oder Komplexen mit struktureller Verwandt-schaft zu Calpainen bekannter Raum- bzw. Kristallform zu ermitteln, und Verfahren bereitzustellen, die es erlauben, für derart modellierte dreidimensionale Protein-strukturen Agonisten oder Antagonisten in Form von Pseu-do-Substraten, Substraten, Aktivatoren oder Inhibitoren bereitzustellen.

Die vorgenannten Aufgaben werden durch die Ansprüche 1, 25, 35, 41, 43, 44, 45, 49 und 50 gelöst.

Gemäß Anspruch 1 werden Raumformen zur Verfügung gestellt, die dreidimensionale Struktur mindestens eines Polypeptids pro asymmetrischer Einheit darstellen, wobei mindestens eines dieser Polypeptide pro asymmetrischer Einheit mindestens eine an der Katalyse der proteolytischen Calpain-Reaktion eines Proteins aus der Familie der neutralen Ca-aktivierten Cystein-Proteinasen (Calpaine) beteiligte (Sub)-Domäne aufweist. Unter einer Raumform wird hierbei die dreidimensionale Struktur eines Moleküls oder Molekülkomplexes, also die atomare räumliche Anordnung der Atome des Moleküls, das dreidimensionale Erscheinungsbild eines Moleküls im vorliegenden Fall also mindestens eines Polypeptids der vorgenannten Art, verstanden, so wie sie sich nach einer Strukturanalyse mit den einschlägigen Methoden der Strukturaufklärung ergibt. Hierbei wären insbesondere die Verfahren der Röntgenstrukturanalyse von Kristallen und der Strukturaufklärung durch nuklear-magnetische Resonanzspektroskopie (NMR-Spektroskopie) zu nennen. Die Raumform entspricht damit dem dreidimensionalen Erscheinungsbild des untersuchten Moleküls/Molekülkomplexes, d.h. seiner durch die Strukturkoordinaten jedes Atoms des Moleküls/Molekülkomplexes wiedergegebenen Raumform. In einem bevorzugten erfin-dungsgemäßen Fall, nämlich bei Vorliegen des mindestens einen Polypeptids vorgenannter Art in einem Kristall, wird die Raumform der Kristallform des mindestens einen Polypeptids entsprechen. Die Kristallform ist insoweit auch das spezifische Erscheinungsbild des kristallisierten mindestens einen Polypeptids in einem Kristall, so wie sie als Ergebnis einer Röntgenstrukturanalyse an entsprechenden Kristallen erhalten wird. Strukturkoordinaten für die Atome des mindestens einen Polypeptids mit mindestens einer an der Katalyse einer Calpain-Reaktion beteiligten (Sub)-Domäne geben die Raumform eines solchen durch NMR-Spektroskopie oder Röntgenkristallographie strukturaufgeklärten Moleküls/Molekülkomplexes wieder.

Während eine erfindungsgemäße Raumform mindestens eines solchen Polypeptids mittels NMR-Strukturanalyse in Lösung aufgeklärt werden kann, ist es für die Methode der Röntgenstrukturanalyse eine Voraussetzung, daß die zu untersuchenden Moleküle kristallisiert vorliegen. Ein derartiger Kristall ist dabei durch Einheitszellen gekennzeichnet, die die zu untersuchenden Moleküle/Molekülkomplexe in einer charakteristischen Anordnung präsentieren. Auf Grund von Symmetrie-Gesetzmäßigkeiten gibt es eine begrenzte Anzahl solcher Anordnungen, die als Raumgruppen bezeichnet werden.

Erfindungsgemäß ist vorgesehen, daß das bevorzugt kristallisierte Polypeptid mindestens eine an der Katalyse beteiligte (Sub)-Domäne eines Proteins aus der Familie der neutralen Ca-aktivierten Cystein-Proteinasen enthält. Nach der Standardnomenklatur (im wesentlichen nach Sorimachi et al., Biochem. J., 1997, 721-732, mit geringfügigen Modifikationen der Erfinder, s. Figur 9 und dazu gehörige Beschreibung) handelt es sich bei diesen katalytischen Calpain-(Sub)-Domänen um die beiden (Sub)-Domänen IIa und IIb, aus der großen Untereinheit des physiologisch aus zwei Untereinheiten zusammengsetzten Calpains. Erfindungsgemäß kann demnach die Raumform oder bevorzugt die Kristallform ein Polypeptid darstellen, das aus-schließlich eine der (Sub)-Domäne IIa oder ausschließlich eine der (Sub)-Domäne IIb entsprechende Aminosäuresequenz aufweisen oder aber auch eine oder beide der vorgenannten (Sub)-Domänen in Kombination mit beliebigen Aminosäuresequenzen an den jeweiligen Termini der katalytischen (Sub)-Domänen, also als rekombinantes Protein, bspw. mit Domänen andere Proteine, enthalten kann. Bevorzugt ist dabei eine erfindungsgemäße Raumform, insbesondere als Kristallform, eines Polypeptids, das die Domänen I, IIa, IIb, III und IV enthält. Ganz besonders bevorzugt ist die Raumform, insbesondere als Kristallform, eines Komplexes beider Calpain-Untereinheiten, d. h. sowohl der 30 kDa als auch der 80 kDa Untereinheit.

Zwar sind vorliegendenfalls solche Raumformen oder bevorzugt Kristallformen von Polypeptiden, die mindestens eine katalytische (Sub)-Domäne eines Calpains mit seiner nativen Aminosäuresequenz enthalten, bevorzugt, doch werden erfindungsgemäß auch solche Raum- oder bevorzugt Kristallformen offenbart, die auf nicht-nativen Calpain-Aminosäuresequenzen beruhen, also Derivate der nativen Sequenzen, darstellen. Hierbei wird es sich insbesondere um Derivate der katalytischen (Sub)-Domänen IIa und/oder IIb handeln, die insbesondere konservative Substituti-on(en) gegenüber den nativen Sequenzen aufweisen. Als konservative Substitutionen werden Substitutionen bezeichnet, bei denen mindestens eine Aminosäure durch eine andere Aminosäure aus der gleichen Klasse ersetzt ist. So etwa können Threonin durch Serin, Lysin durch Arginin (positiv geladene Aminosäuren), Leucin durch Isoleucin, Alanin oder Valin (aliphatische Aminosäuren) bzw. jeweils vice versa substituiert sein. Die nativ auftretenden 20 Aminosäuren werden dabei nach ihren chemischen oder physikalischen Eigenschaften in Klassen eingeteilt. So etwa werden bspw. Aminosäuren mit positiv oder negativ geladenen Seitenketten, mit aromatischen Seitenketten, aliphatischen Seitenketten, Seitenketten mit Hydroxyl- oder Aminogruppengruppen in entsprechende, jeweilige Klassen gruppiert.

Ein Polypeptid in einer erfindungsgemäßen Kristallform kann aber auch Aminosäuren aufweisen, die nativ nicht oder jedenfalls nativ nicht typischerweise auftreten.

Aber auch Raum- oder bevorzugt Kristallformen von Polypeptiden mit mindestens einer Deletion und/oder Insertion gegenüber der jeweiligen nativen Aminosäuresequenz einer oder beider katalytischer (Sub)-Domänen oder einer regulatorischen Domäne eines Calpains werden im Rahmen der vorliegenden Erfindung zur Verfügung gestellt. Insbesondere schließt die vorliegende Erfindung Derivate ein, die mindestens eine Insertion und/oder Deletion in sog. Schleifen("Loop")-Strukturen der katalytischen (Sub)-Domäne(n) aufweisen.

Bevorzugt handelt es sich bei den beanspruchten Raumformen, vorzugsweise als Kristallformen, um dreidimensionale Strukturen von Polypeptiden, wobei die in denselben enthaltene katalytische (Sub)-Domäne(n) aus Isozymen aus der Familie der ubiquitär exprimierten Calpaine oder aus Isozymen der Familie der gewebespezifisch exprimierten Calpaine (n-Calpaine) entstammt/en. Besonders bevorzugt sind wiederum Raum-/Kristallformen für Polypeptide oder Komplexe aus zwei oder mehr Polypeptiden, die die beiden Untereinheiten mit einer Auswahl, ganz besonders bevorzugt aber mit allen katalytischen und regulatorischen Domänen enthalten. Ganz besonders bevorzugt sind solche dreidimensionalen Raumformen, insbesondere Kristallformen, als Ergebnis kristallographischer Untersuchungen, wenn sie mindestens eine katalytische (Sub)-Domäne von Proteinen aus der Gruppe der m- oder µ-Calpaine enthalten. In einer ganz besonders bevorzugten Ausführungsform entspricht die Aminosäuresequenz der in der Raum- bzw. Kristallform vorliegenden zumindest einen katalytischen Calpain-(Sub)-Domäne einer korrespondierenden nativen Aminosäuresequenz aus Eukaryonten-Zellen, insbesondere aus Zellen von Ver-tebraten, vor allem aus Säugetierzellen, und hier wiederum insbesondere aus humanen Zellen, oder Derivaten, bspw. Substitutions-, Deletions- und/oder Insertionsderivaten, derselben.

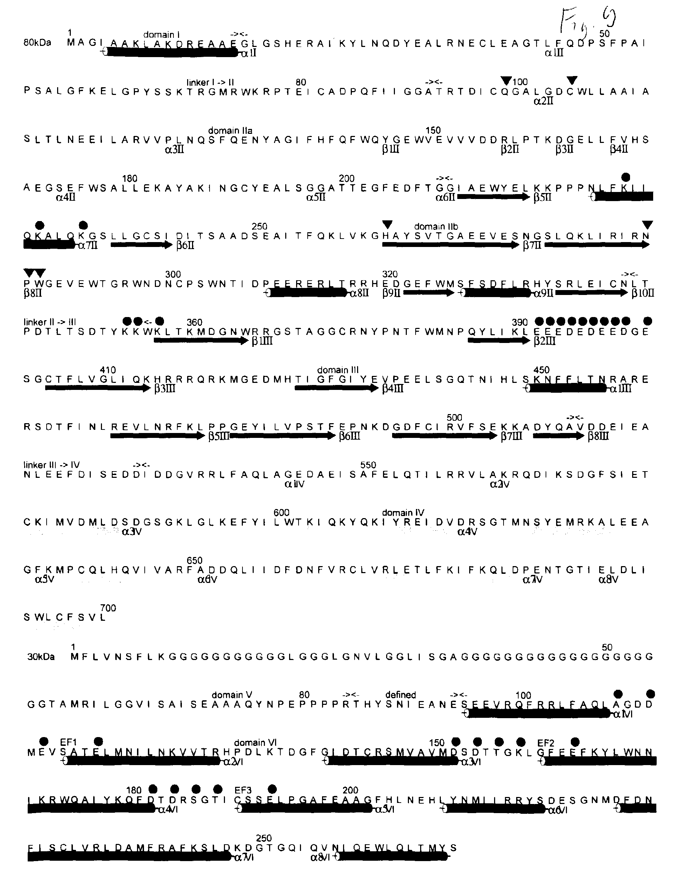

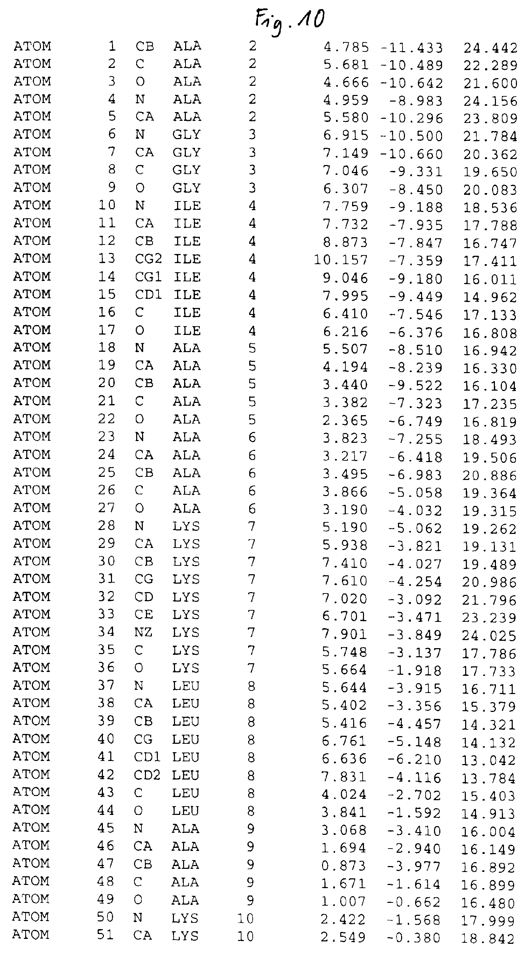

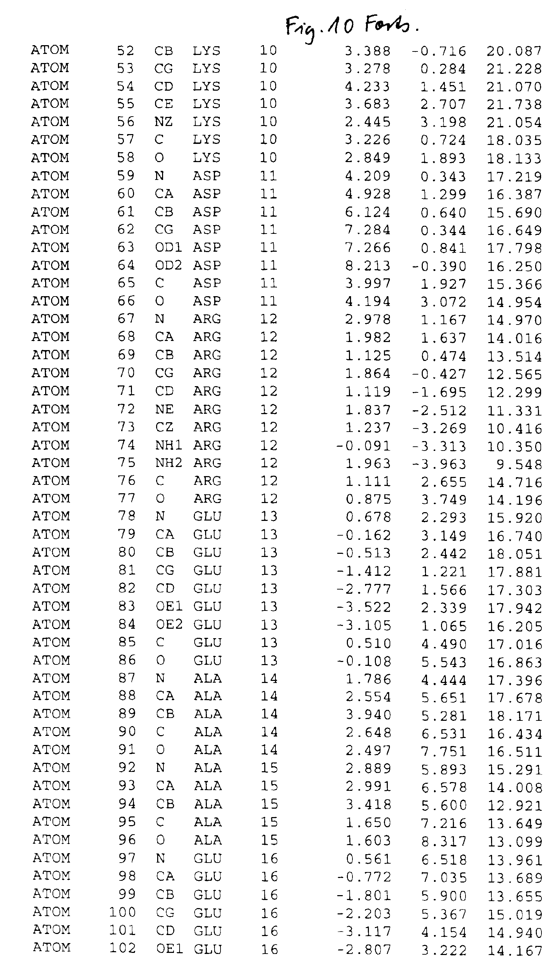

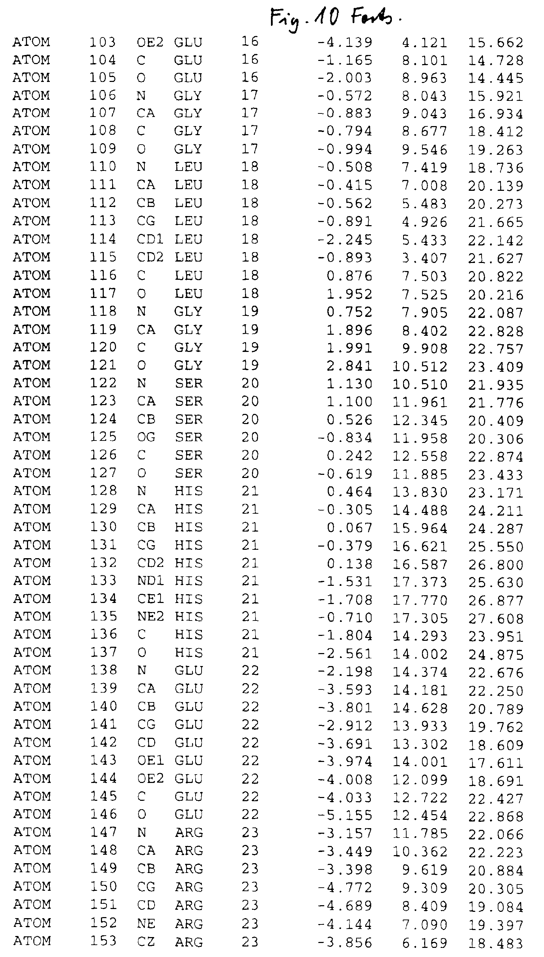

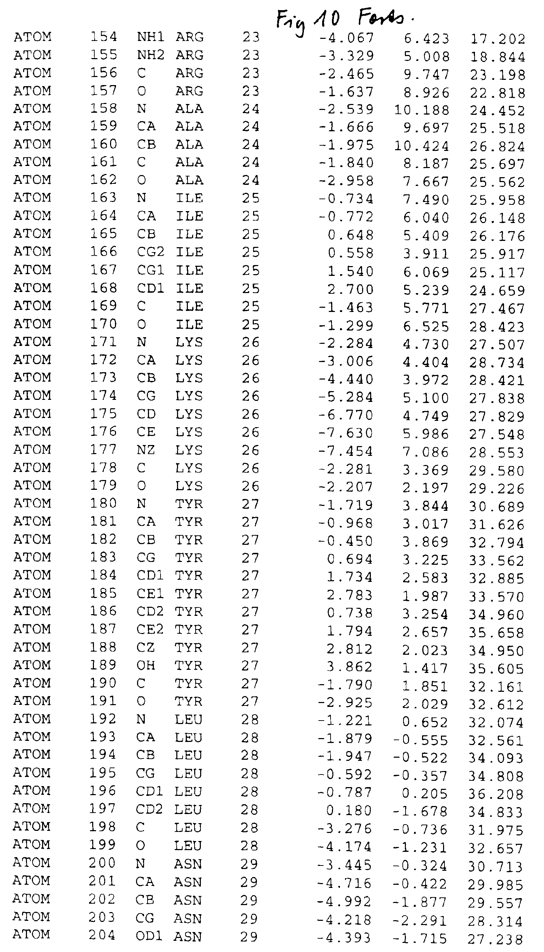

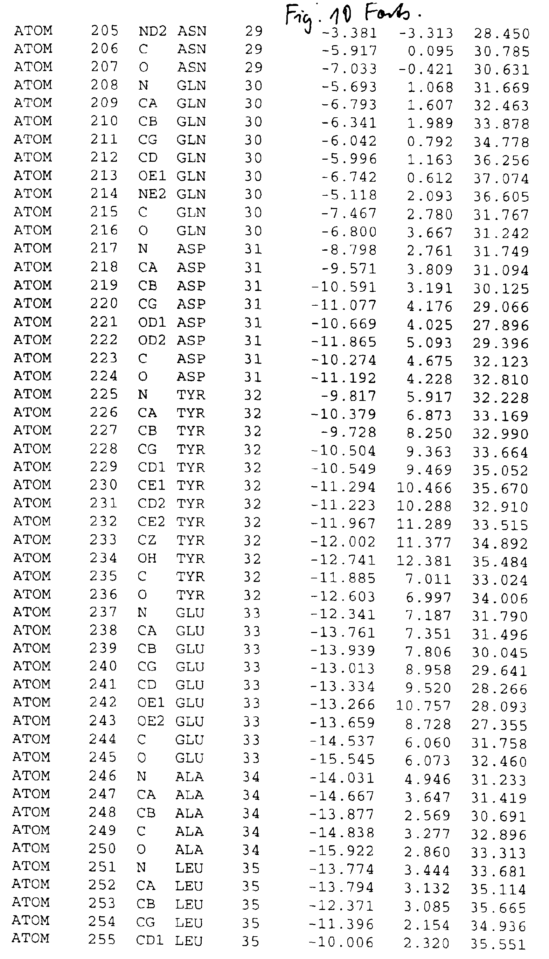

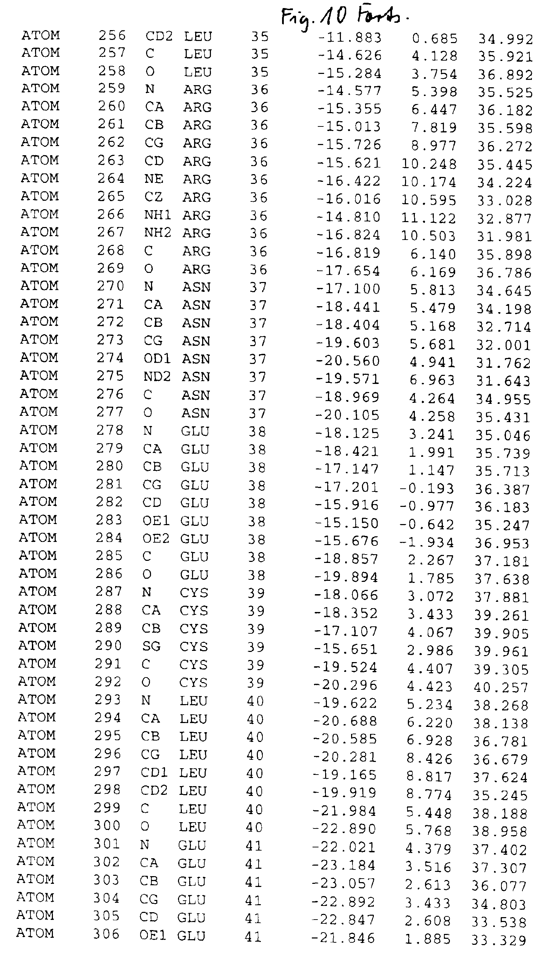

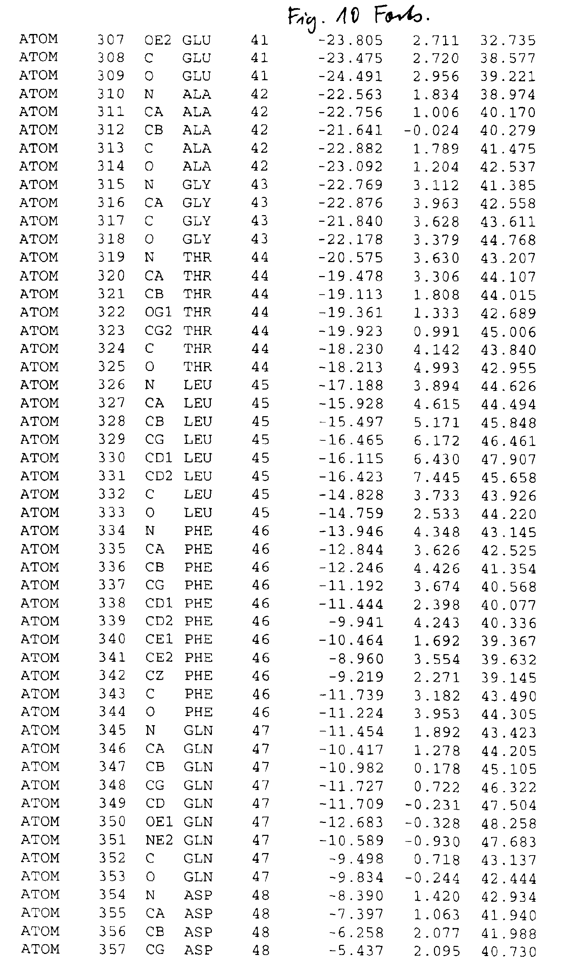

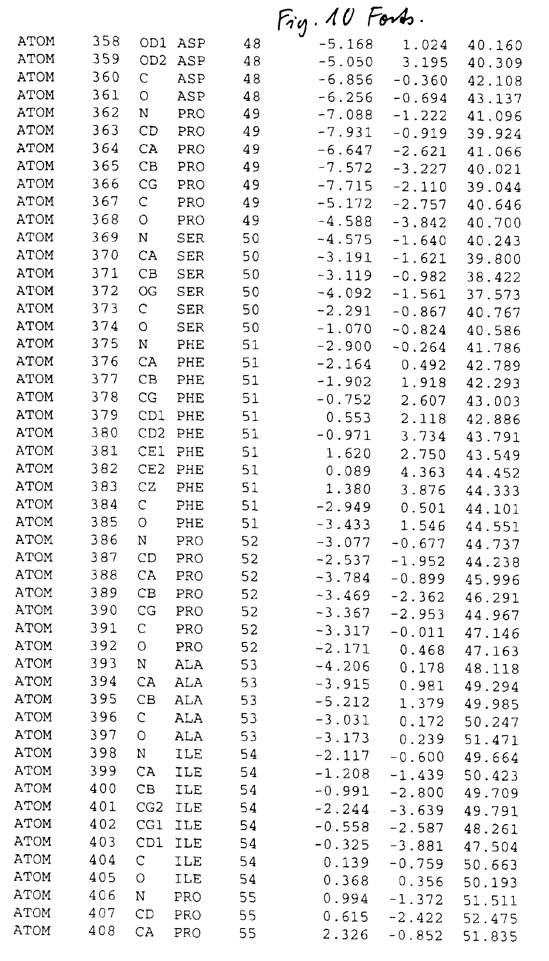

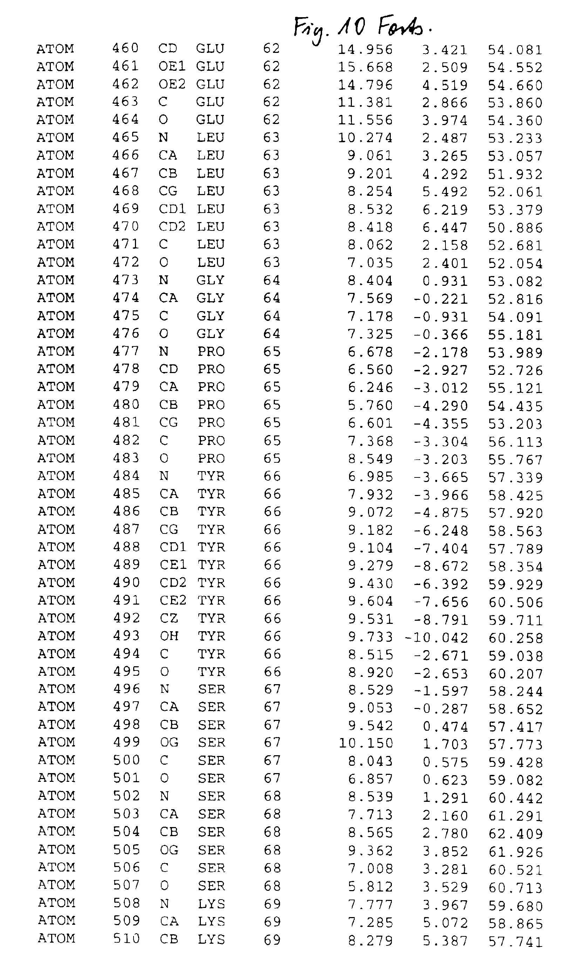

Vorzugsweise wird erfindungsgemäß eine Raum- oder Kristallform eines solchen Polypeptids zur Verfügung gestellt sein, das die Aminosäuresequenz gemäß Fig. 3, 4, 5 X und/oder Fig. 6, insbesondere die in den Figuren 4 und 6 enthaltenen Sub-Domänen IIa (von T93 bis G209) und/oder IIb (von G210 bis N342) oder auch Derivate einer oder beider der vorgenannten (Sub)-Domänen-Aminosäure-sequenzen, enthält. Darüber hinaus wird eine bevorzugte Raum- oder Kristallform eine dreidimensionale Struktur eines Polypeptids oder eines Komplexes mit mindestens einem Polypeptid widerspiegeln, das eine Aminosäuresequenz gemäß Fig. 9 oder ein Derivat derselben aufweist.

In einer weiteren bevorzugten Ausführungsform werden durch die vorliegende Erfindung solche Raumformen, vorzugsweise als Kristallformen, offenbart, die neben dem mindestens einen Polypeptid mit einer der zuvor erfin-dungsgemäß beschriebenen Aminosäuresequenzen mindestens eine weitere Komponente aufweisen. Bei dieser weiteren Komponente kann es sich um ein oder mehr gleiche oder verschiedene Metallion(en) handeln, vorzugsweise um Alkali- und/oder Erdalkalimetall-Ionen, vor allem um Calcium-Ionen. In dem bevorzugten Fall einer Kristallform kann es sich bei der zusätzlichen Komponente aber auch um ein oder mehr identische oder verschiedene Schwermetall-Ion(en) handeln, die typischerweise in räumlicher Nachbarschaft zu Cystein- oder Histidinresten der dreidimensional aufgefalteten mindestens einen Polypeptidsequenz angesiedelt sind. Ganz besonders bevorzugt sind dabei solche Kristallformen, bei denen die Schwermetall-Ionen, vorzugsweise Gold- und/oder Quecksilber-Ionen, Wechselwirkungen mit einer oder mehr der folgenden Aminosäuren (nach der Nomenklatur gemäß Figur 9 für humanes m-Calpain) C105, C98, H169, C191, R420, C240, H334, C696 und/oder H908 (aus der kleinen Untereinheit) bzw. mit den zu den vorgenannten Aminosäuren strukturell und funktionell korrespondierenden Aminosäuren anderer Calpaine eingehen. Weiterhin ganz besonders bevorzugt sind Kristallformen, bei denen mindestens ein Gold- und/oder mindestens ein Quecksilber-Ion durch die Aminosäure C105, durch Anlagerung an die räumlich benachbarten Aminosäuren C98/H169, C191/R420, C240/H334 und/oder C696/H908 komplexiert wird (werden) (zur Nomenklatur s. vorangehend).

In einer weiteren bevorzugten Ausführungsform umfaßt die Raumform bzw. vorzugsweise Kristallform mindestens einen Liganden, der typischerweise nicht-kovalent, ggf. aber auch kovalent, an das (die) Polypeptid(e) in der asymmetrischen Einheit gebunden ist (sind). Bei diesem Liganden kann es sich um Agonisten oder Antagonisten von Calpain handeln, also um Substrat-, PseudoSubstrat-, Aktivator-oder Inhibitor-Moleküle. Eine Raumform, vorzugsweise in Kristallform, ist dann besonders bevorzugt, wenn der Ligand an die katalytische Domäne, insbesondere in das aktive Zentrum dieser Calpain-Domäne, oder an bzw. in die von den Sub-Domänen IIa und IIb gebildete Furche - für den Fall der katalytisch inaktiven Konformation - des mindestens einen Polypeptids bindet. Eine erfindungsgemä-ße Raumform kann aber auch zwei oder mehr Liganden aufweisen, z. B. einen an eine regulatorische Domäne (Domänen III und/oder IV) bindenden inhibitorischen Liganden und mindestens einen weiteren Liganden, der an eine oder beide katalytische(n) Sub-Domäne(n) der großen Untereinheit (IIa und/oder IIb) andockt.

Bei diesen funktionellen Liganden kann es sich um beliebige, insbesondere auch organisch- chemische Moleküle handeln, die durch ihre sterischen und/oder chemischen Eigenschaften an den Calpain-Komplex binden können. Bevorzugt allerdings sind Di- und/oder Oligopeptide, die gegebenenfalls durch chemische Modifikationen stabilisiert sind, oder um die Di- oder Oligopeptidanaloga, die beispielsweise im Bereich des aktiven Zentrums um Bin-dungsplätze mit den eigentlichen (Poly)-Peptidsubstrat-Molekülen konkurrieren, die aber aufgrund chemischer Veränderung nicht der Proteolyse unterliegen und damit das aktive Zentrum des Calpains blockieren. So etwa können die Amidbindungen eines normalerweise Substrateigenschaf-ten aufweisenden Di- und/oder Oligopeptids durch reduzierte Amidbindungen oder Pseudopeptidbindungen, bspw. Methylen- oder Acetylengruppen, modifiziert sein und sind damit nicht der Proteolyse zugänglich. In einer keineswegs abschließenden Aufzählung können Inhibitoren des aktiven Zentrums daher nicht proteolysierbare Peptidomime-tika der folgenden Peptide (im Ein-Buchstaben-Code) sein oder derartige nicht proteolysierbare Peptidsequenzen enthalten: YCTGVSAQVQK, RARELGLGRHE, AERELRRGQIL, PRDETDSKTAS, KYLATASTMDH, DHARHGPLPRH, STSRTP, SCPIKE, DTPLPV, STPDSP, PNGIPK, PPGGDRGAPKR, WFRGLNRIQTQ und/oder RGSGKDSHHPA.

Neben den erfindungsgemäßen Raumformen, insbesondere den dreidimensionalen Kristallformen, die eine Darstellung des jeweiligen strukturellen Aufbaus mindestens eines Polypeptids mit den vorgenannten Eigenschaften liefern, sind auch makroskopische Kristalle Gegenstand der vorliegenden Erfindung. Demnach werden erfindungsgemäß - als Basis für die Röntgenstrukturanalyse dienende-kristalline Anordnungen offenbart, die mindestens ein Polypeptid pro asymmetrischer Einheit aufweisen, wobei mindestens ein solches Polypeptid mindestens eine katalytische Sub-Domäne eines Calpains, also entweder die Sub-Domäne IIa und/oder die Sub-Domäne IIb, enthält. Bevorzugt sind dabei Kristalle, die in der asymmetrischen Einheit mindestens ein Polypeptid enthalten, das mindestens eine katalytische Sub-Domäne von humanem Calpain, insbesondere humanem m-Calpain, aufweist. Es wird in diesem Zusammenhang darauf hingewiesen, daß im Rahmen der vorliegenden Erfindung bevorzugte Ausführungsformen von er-findungsgemäßen Kristallen all jene makroskopischen Ar-rangements einschließen, bei denen mikroskopisch in der asymmetrischen Einheit Kristallformen entsprechen, wie sie erfindungsgemäß zuvor offenbart worden sind. Daher wird insoweit auf die vorhergehende Offenbarung Bezug genommen.

Erfindungsgemäße Kristalle können hinsichtlich ihrer symmetrischen Eigenschaften in allen denkbaren 65 enantio-morphen Raumgruppen vorliegen. Insbesondere kommen erfin-dungsgemäß Raumgruppen des triklinen, monoklinen, orthorhombischen, tetragonalen, trigonal/rhombohedralen, hexagonalen und kubischen Typs in Betracht.

Ganz besonders bevorzugt sind Kristalle, die in der asymmetrischen Einheit beide Calpain-Untereinheiten, nämlich die 30 kDa- und die 80 kDa-Untereinheit, enthalten. Dabei kann bspw. mindestens ein Polypeptid in der asymmetrischen Einheit mit der in Figur 4 dargestellten Aminosäuresequenz in einer erfindungsgemäßen Kristallform vorliegen oder diese enthalten. Ganz besonders bevorzugt sind dabei monokline Raumgruppen, insbesondere die Raumgruppe P2₁. Weiterhin bevorzugt sind erfindungsgemäße Kristalle mit Einheitszellen, deren asymmetrische Einheit Kristallformen mit mindestens einem Polypeptid-Heterodimer aufweist, wobei das eine Polypeptid (1) vorzugsweise eine Aminosäuresequenz entsprechend Figur 3 und das andere Polypeptid (2) vorzugsweise eine Aminosäuresequenz gemäß Figur 4 umfaßt. Insoweit entspricht das vorzugsweise in der asymmetrischen Einheit mikroskopisch als Kristallform vorliegende Heterodimer dem funktionellen physiologischen Calpain-Proteinkomplex mit der großen und der kleinen Untereinheit. Weiterhin bevorzugt sind erfindungsgemäß Kristalle, die eine monokline Einheitszelle mit den folgenden Zellkonstanten (ungefähre Abmessungen) a=64,9 Å, b=134,0 Å, c=78,0 Å und β=102,4° oder aber a=51,8 Å, b=171,4 Å, c=64,7 Å und β=94,8° aufweisen.

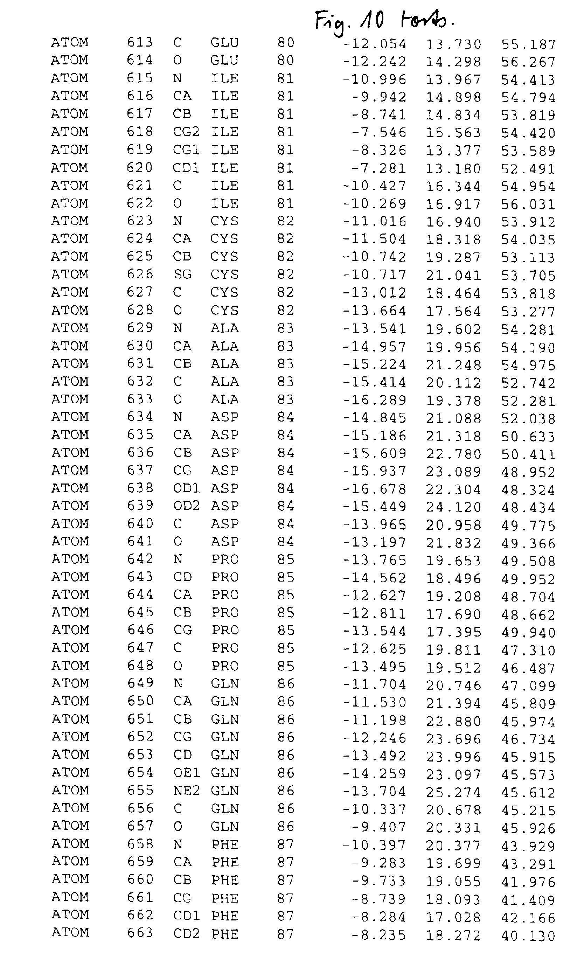

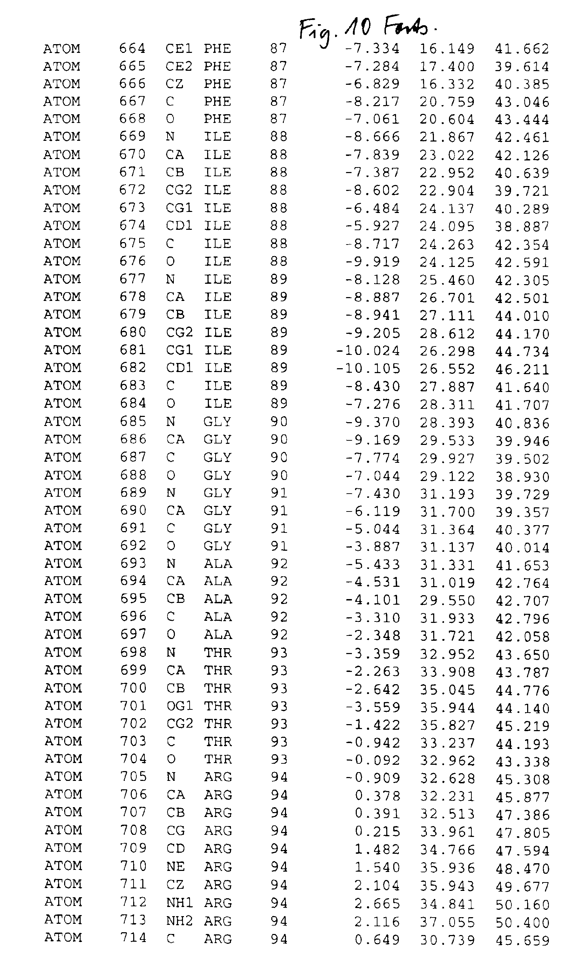

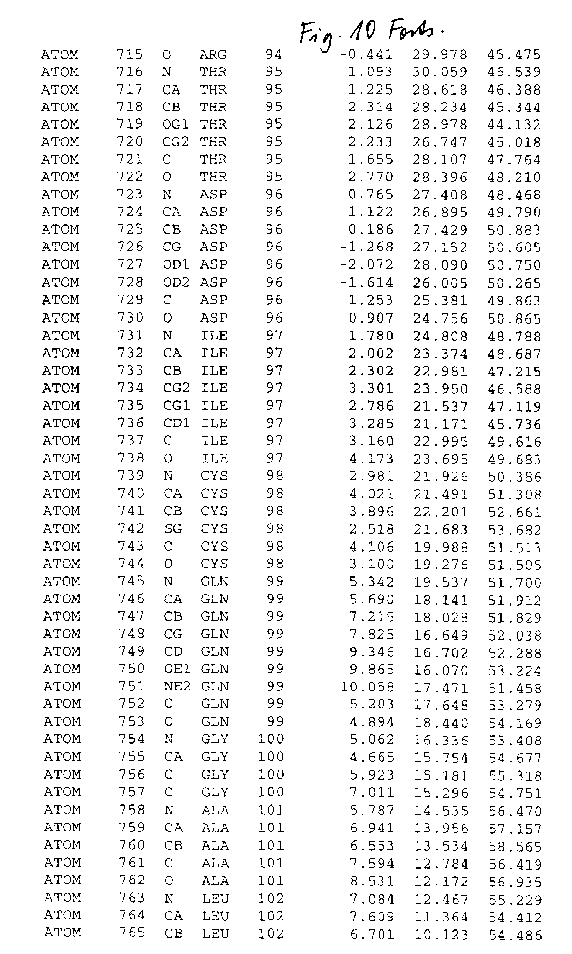

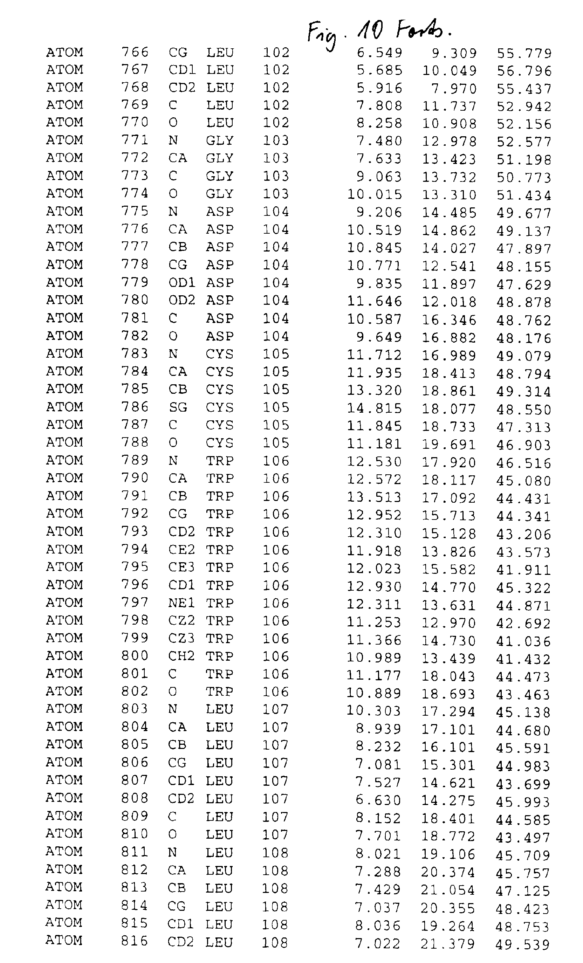

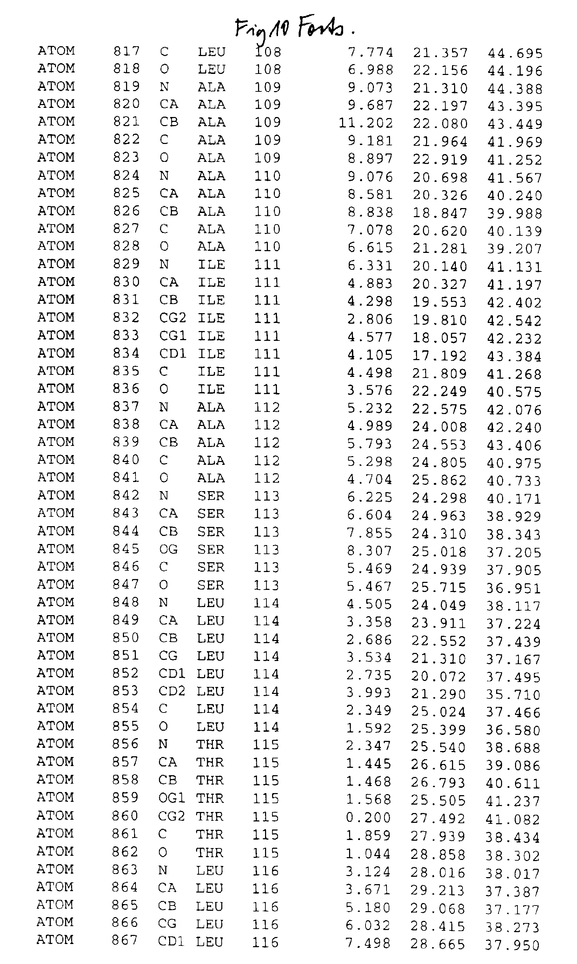

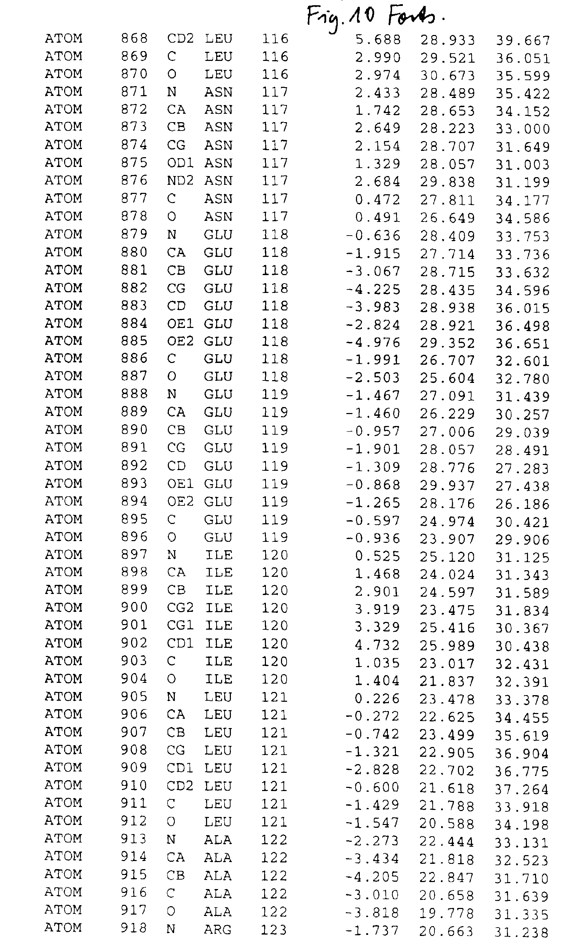

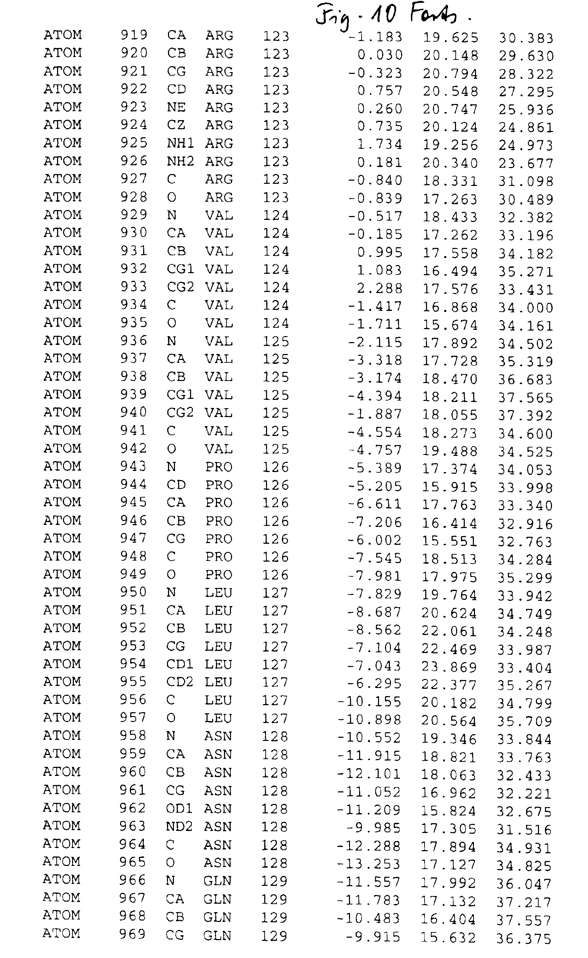

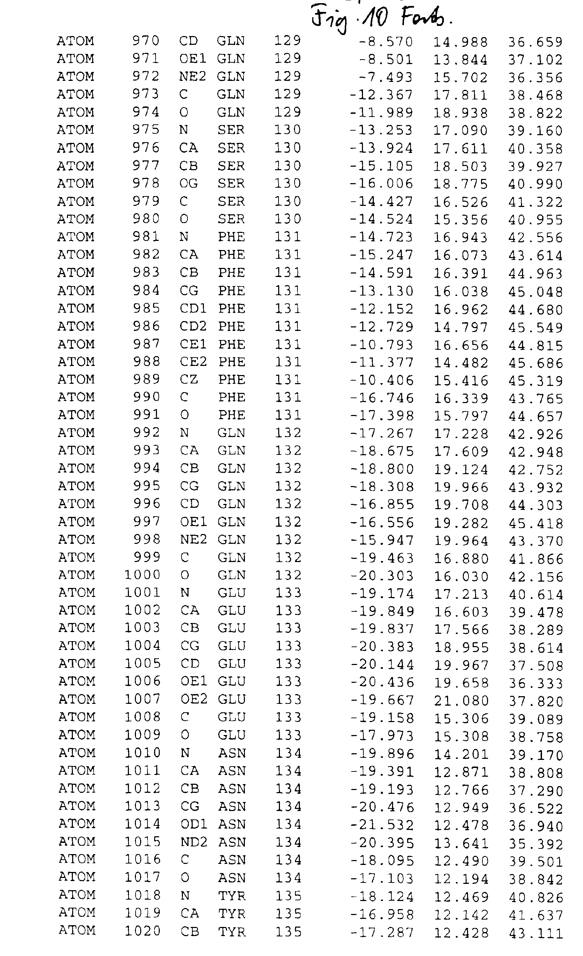

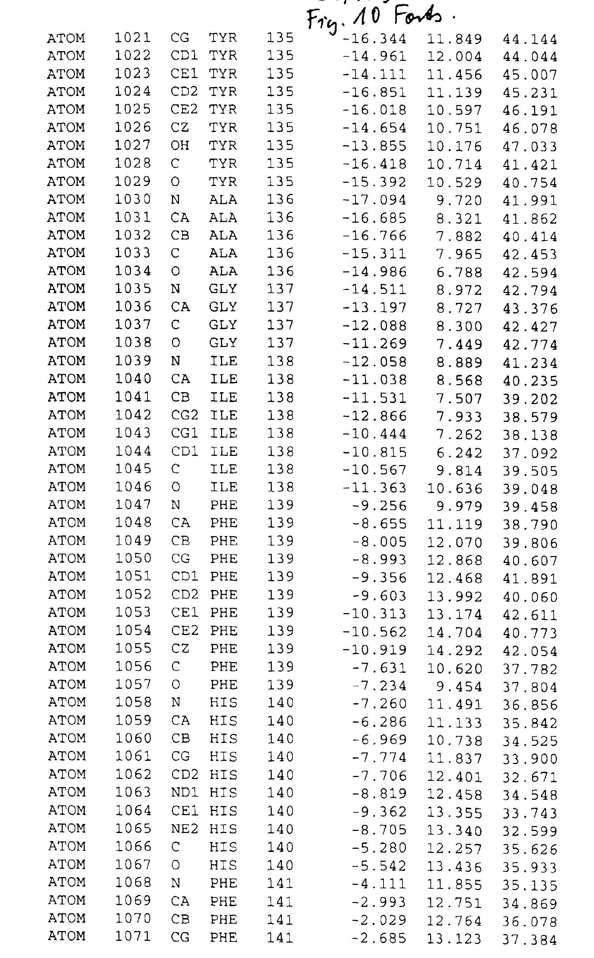

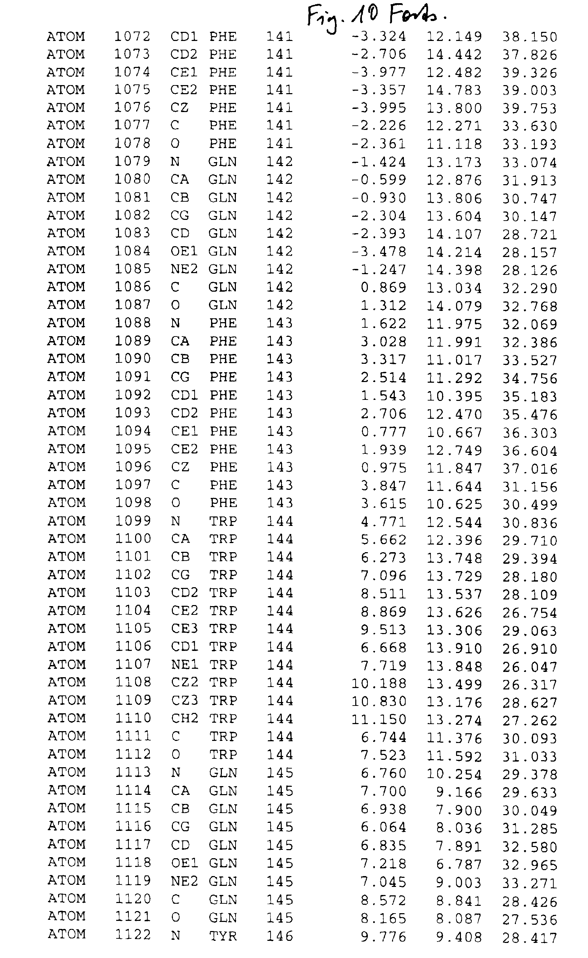

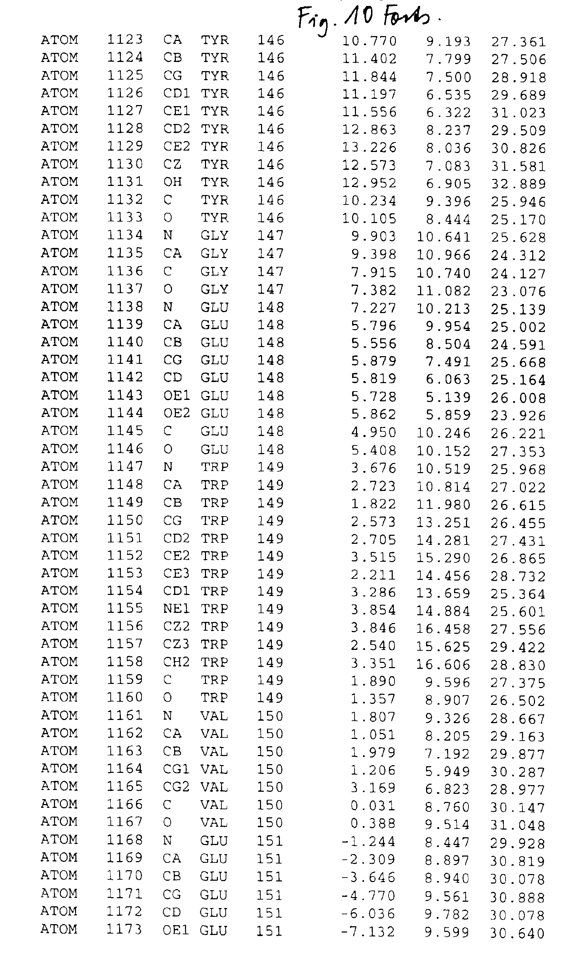

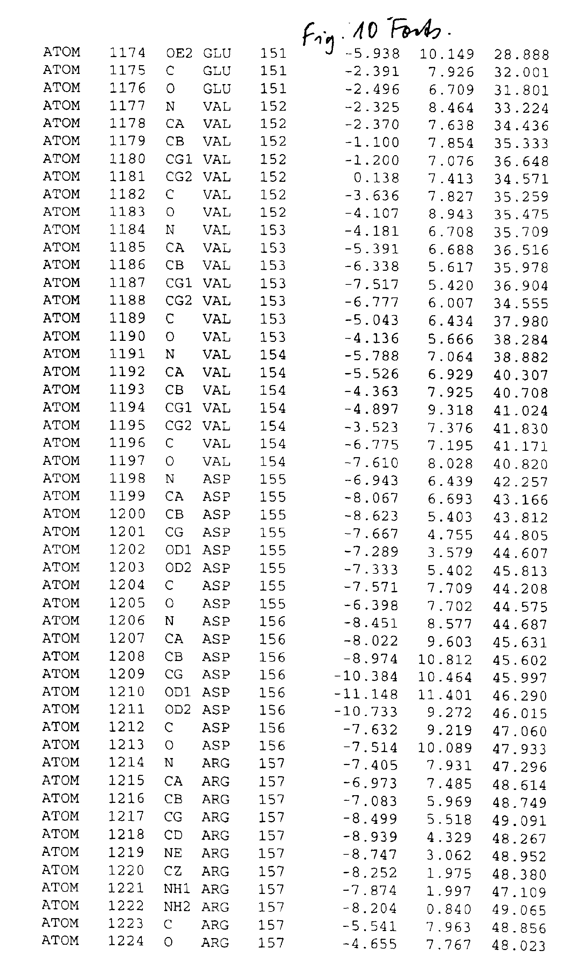

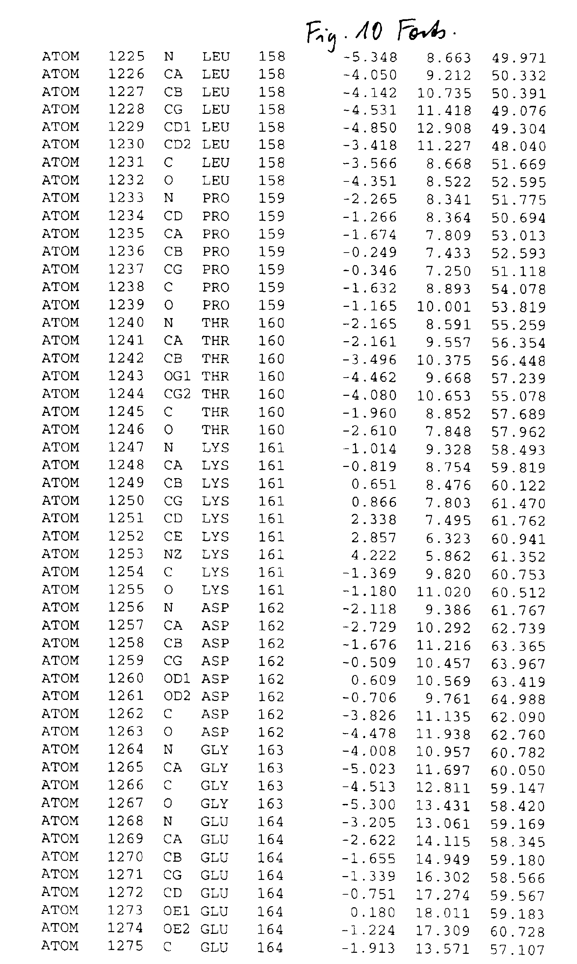

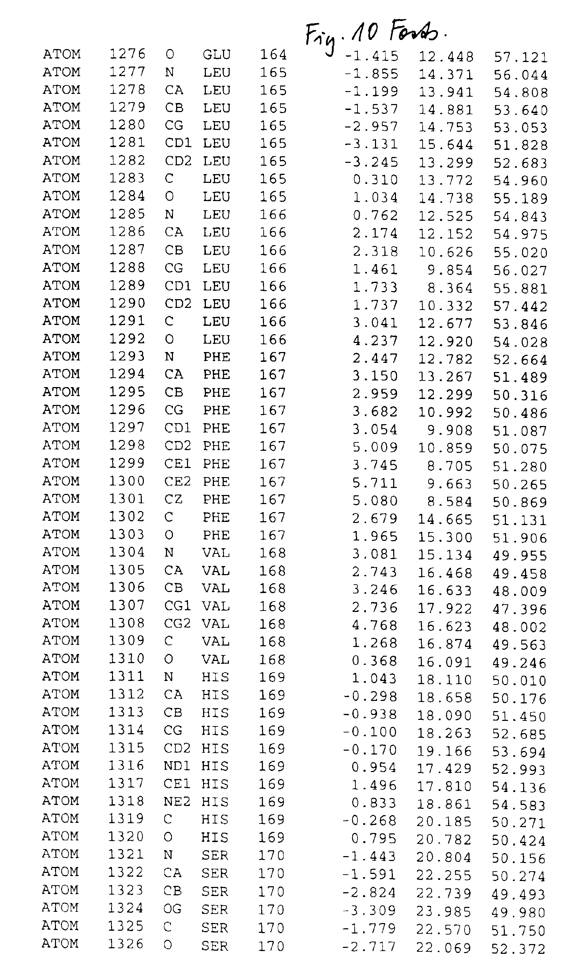

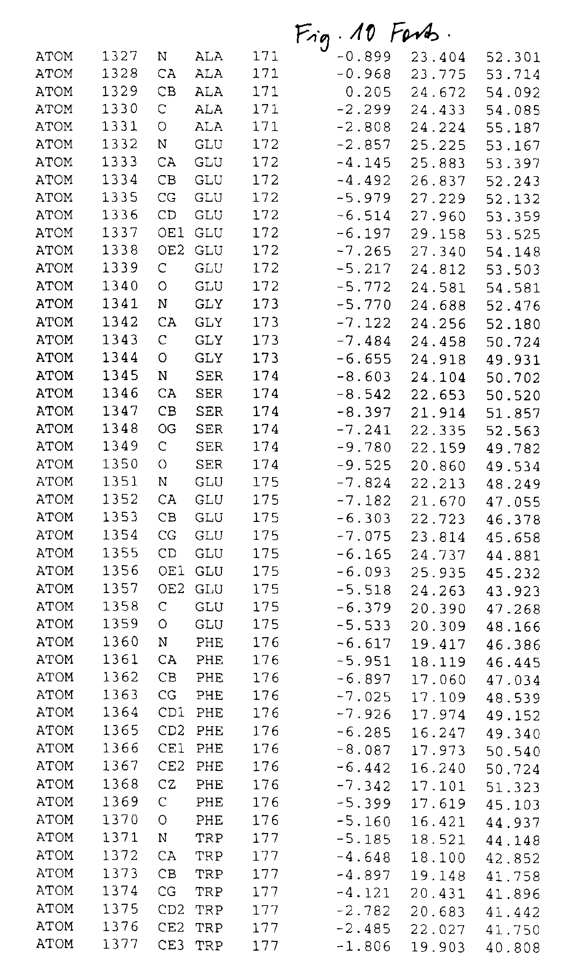

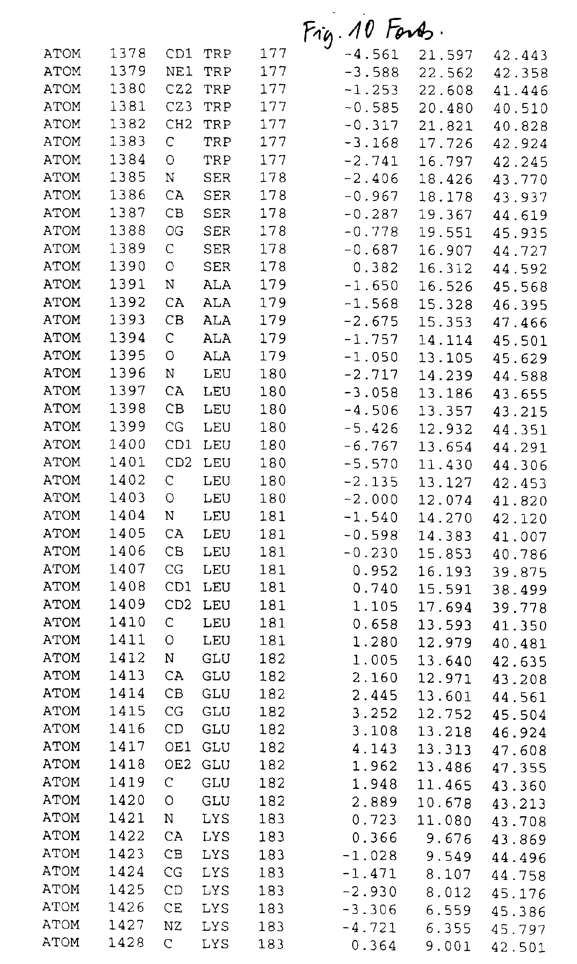

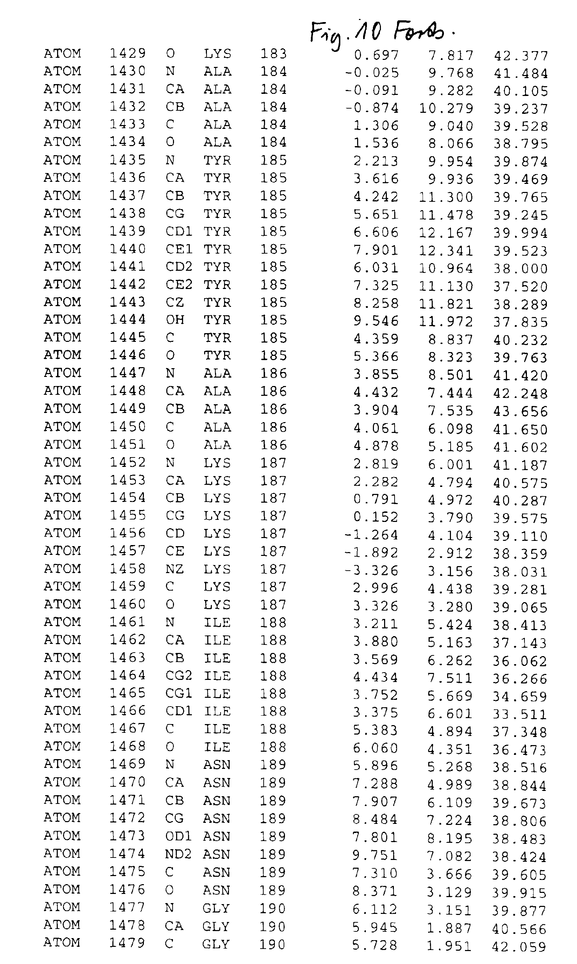

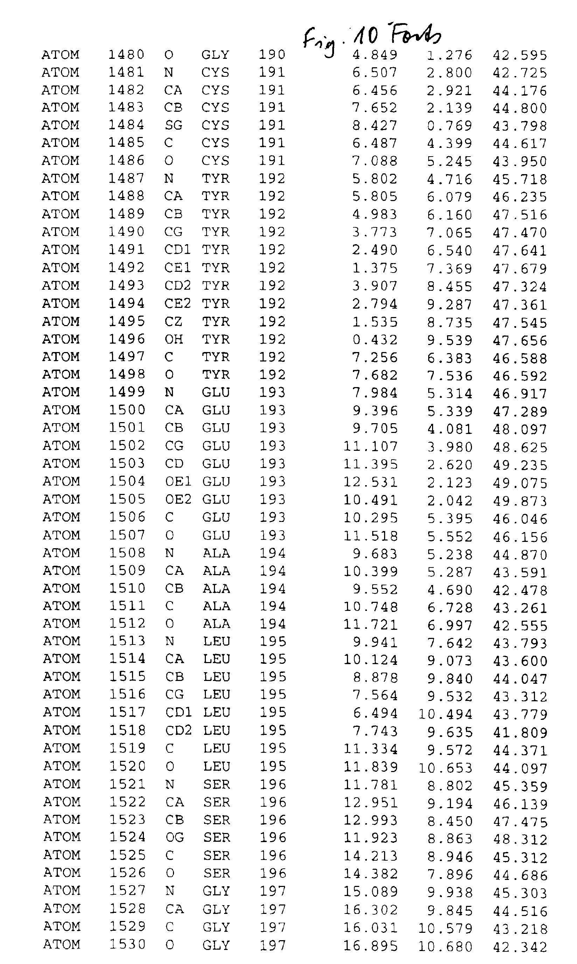

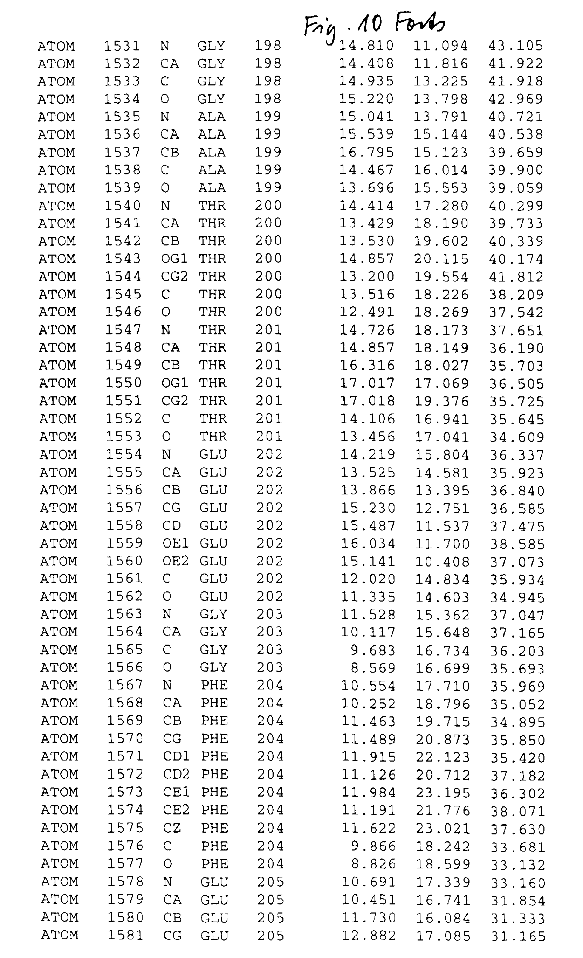

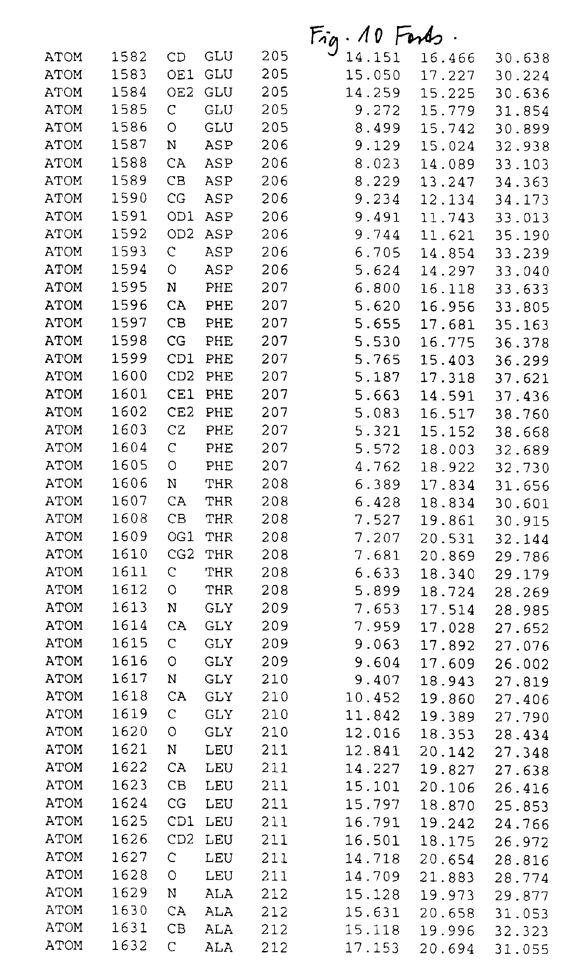

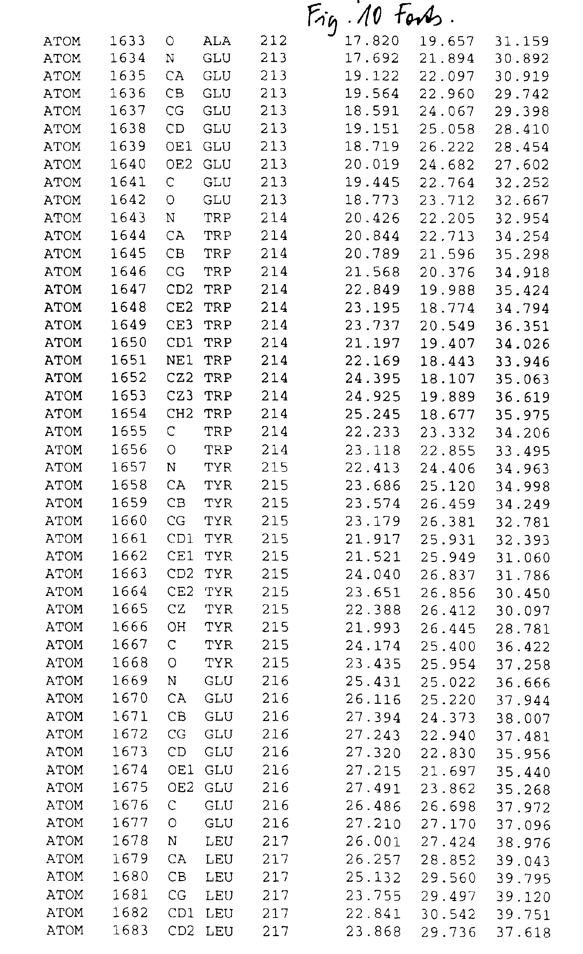

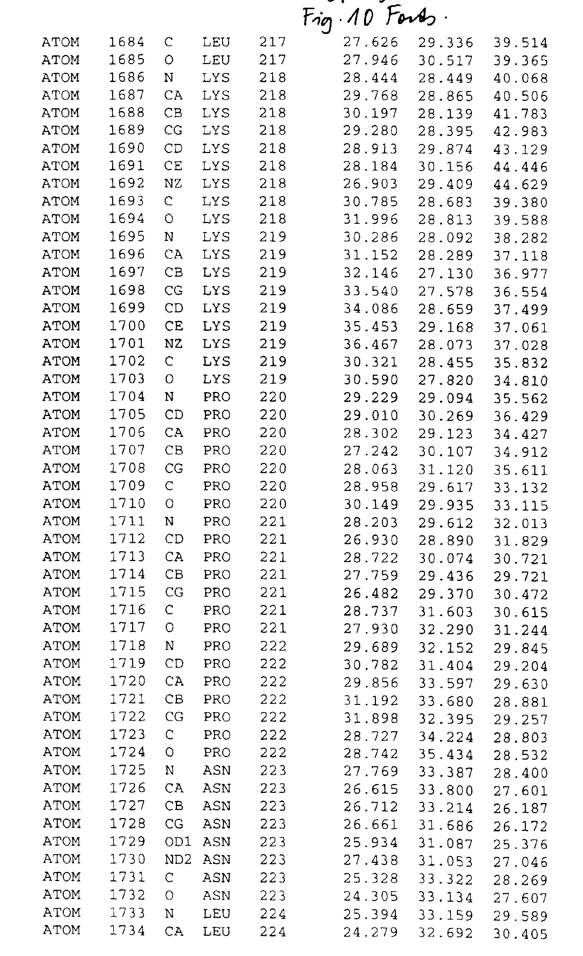

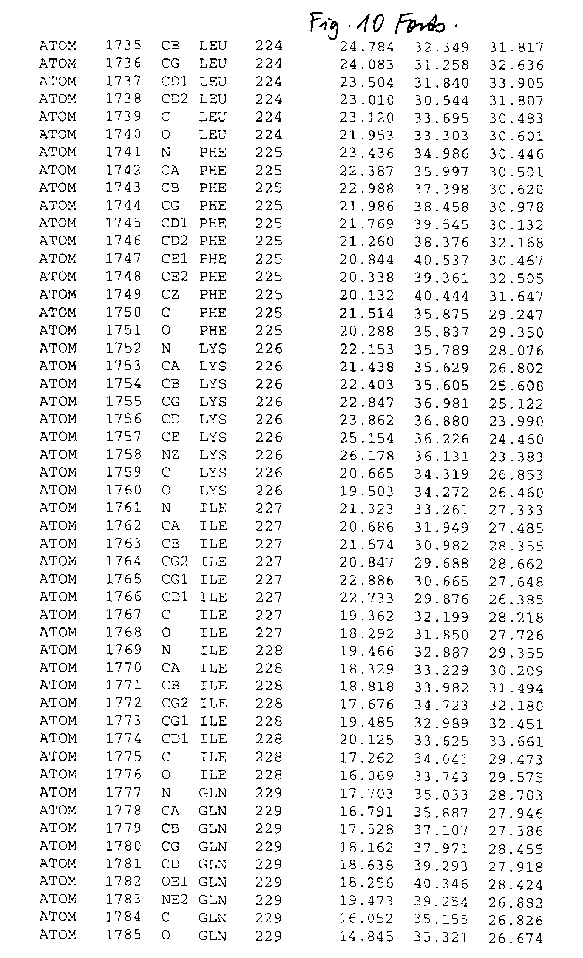

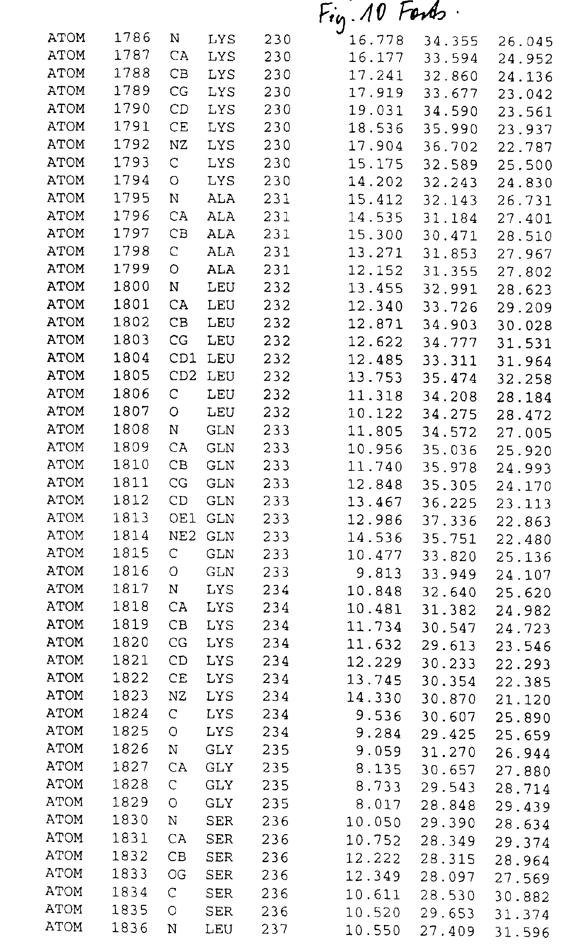

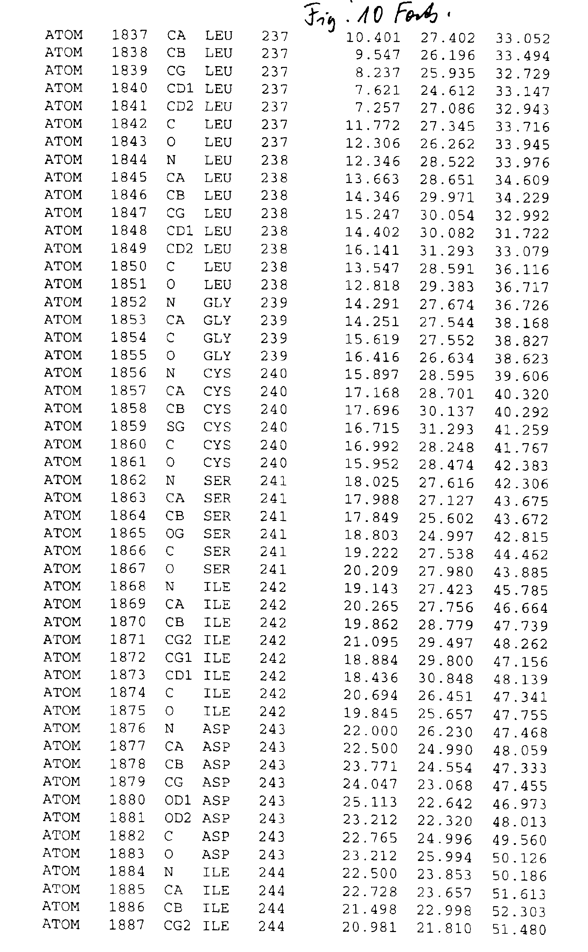

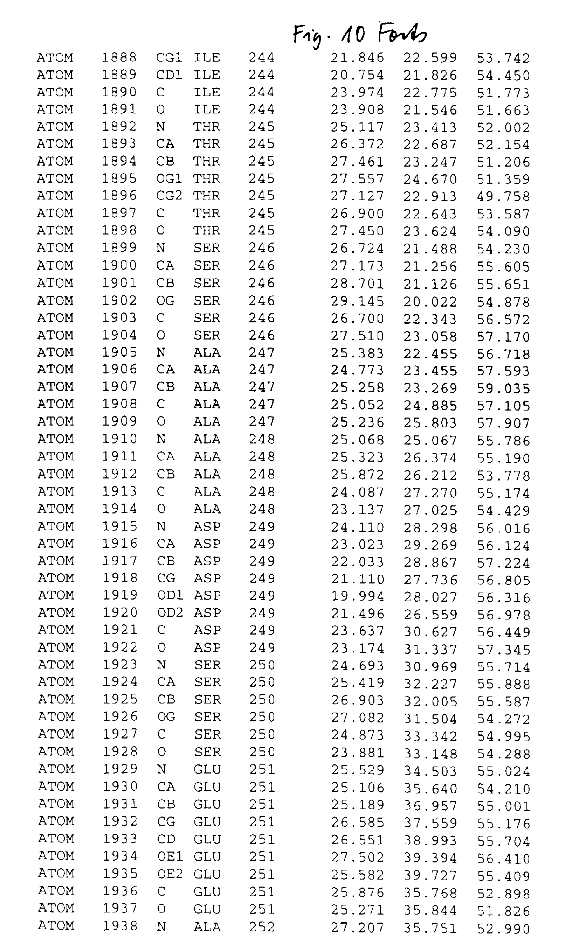

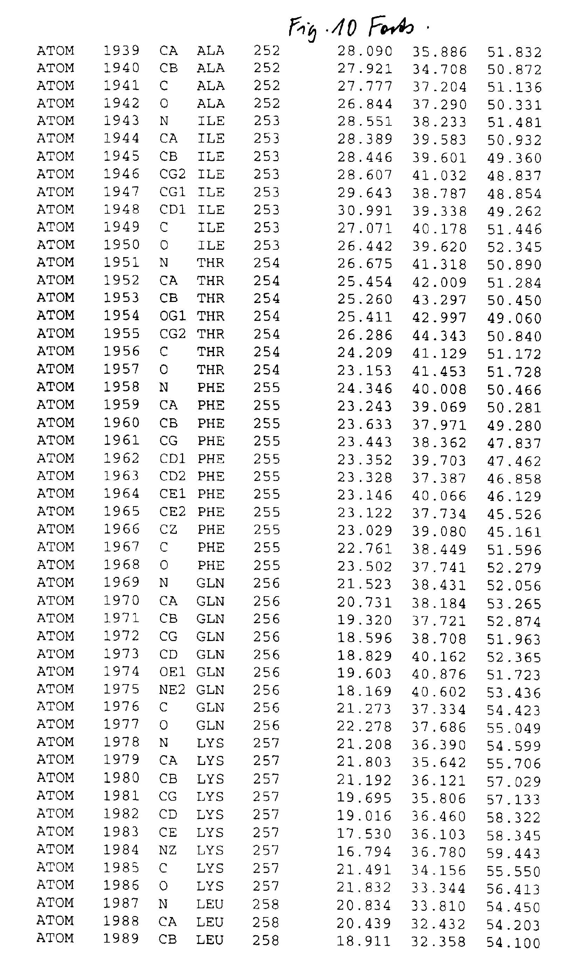

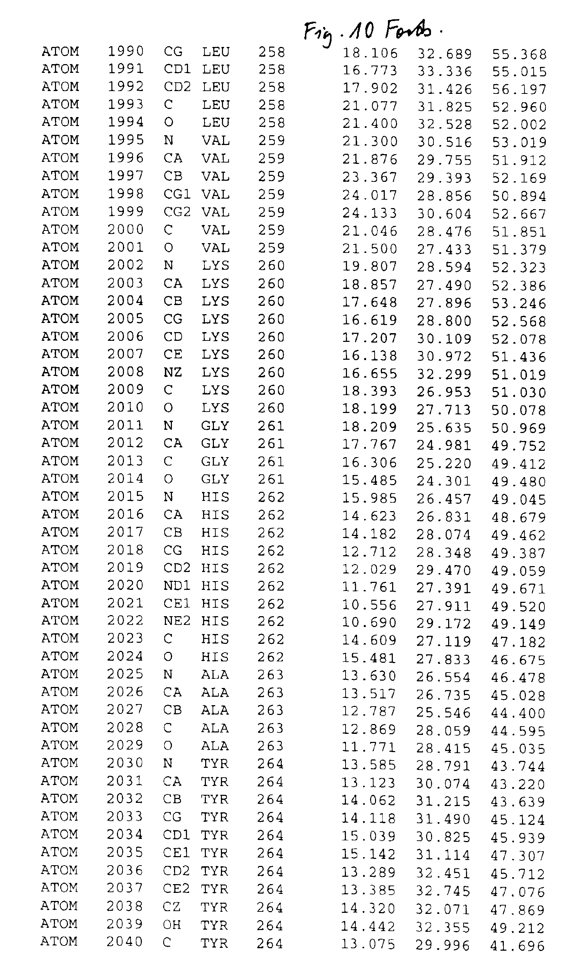

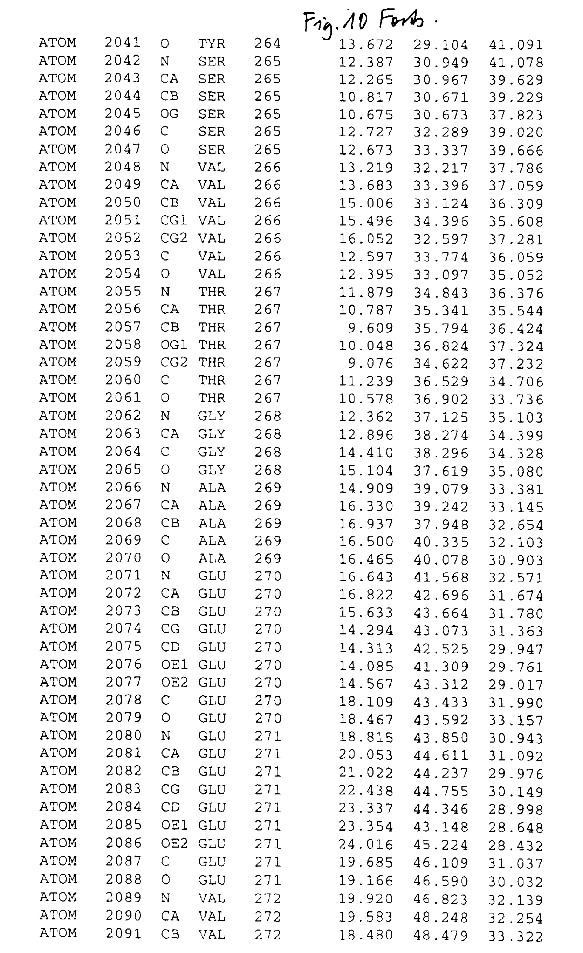

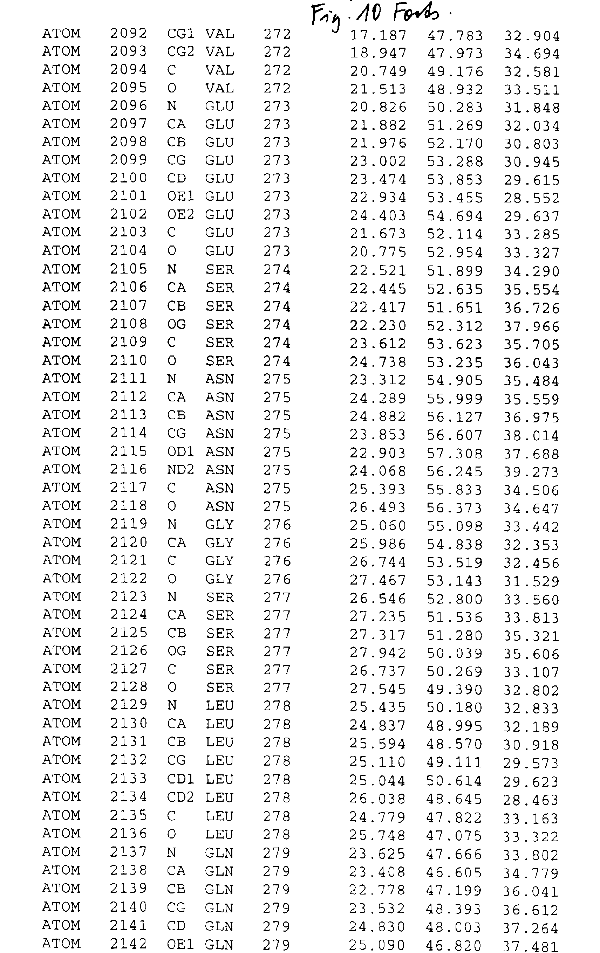

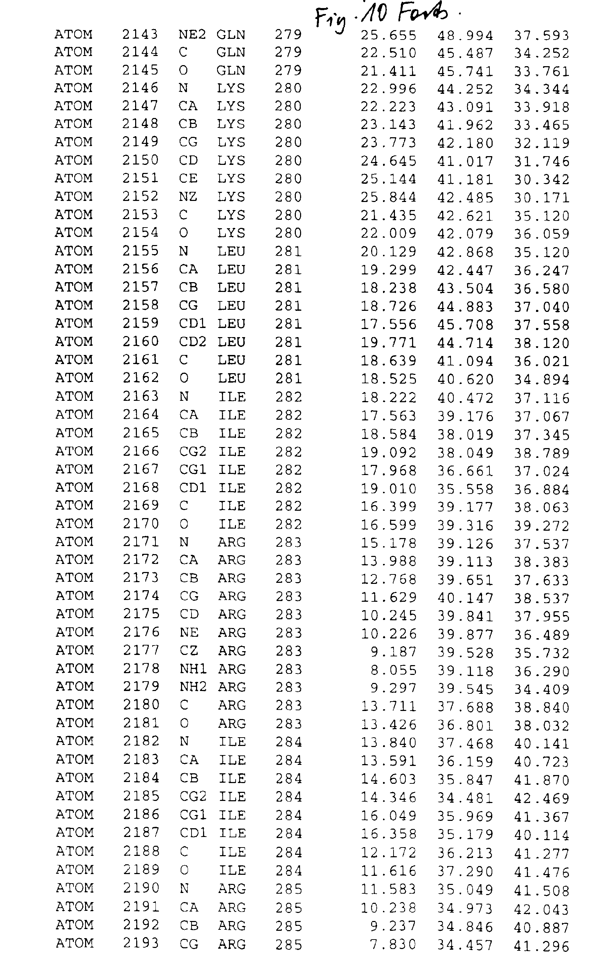

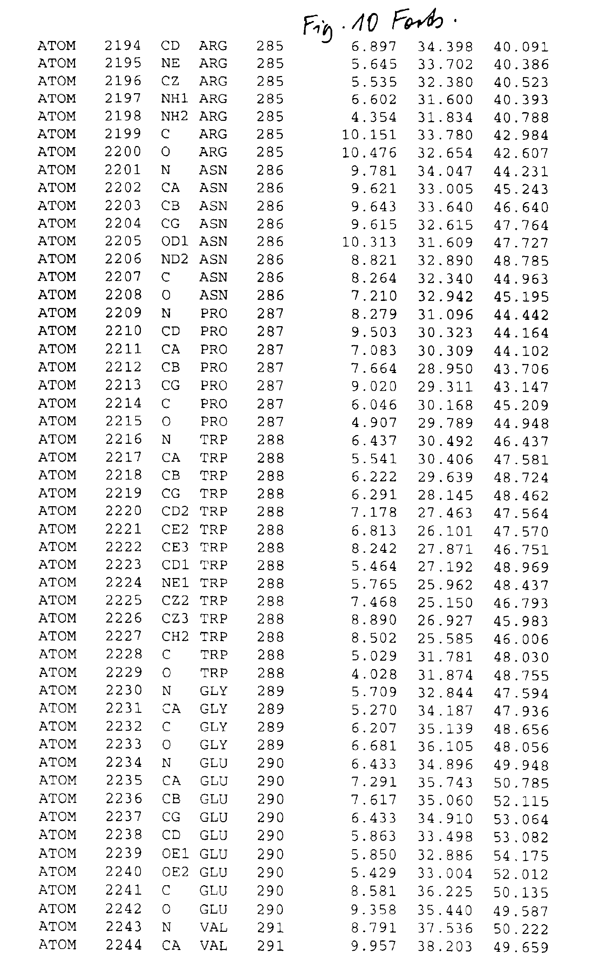

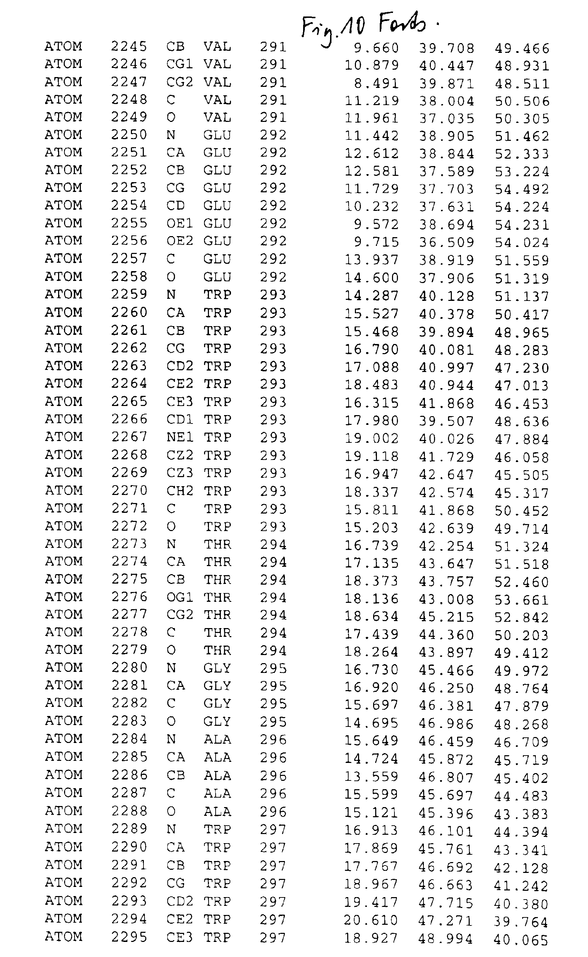

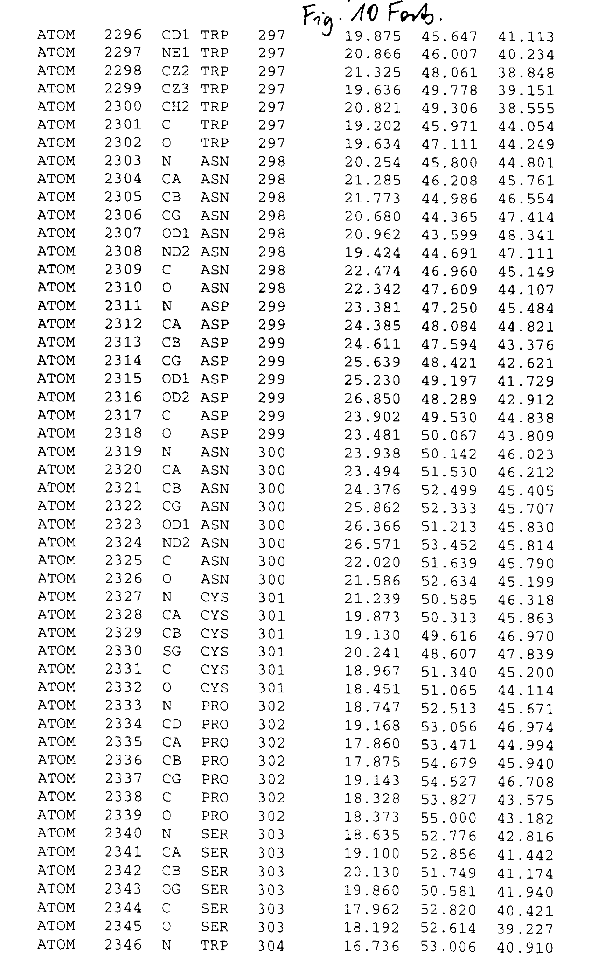

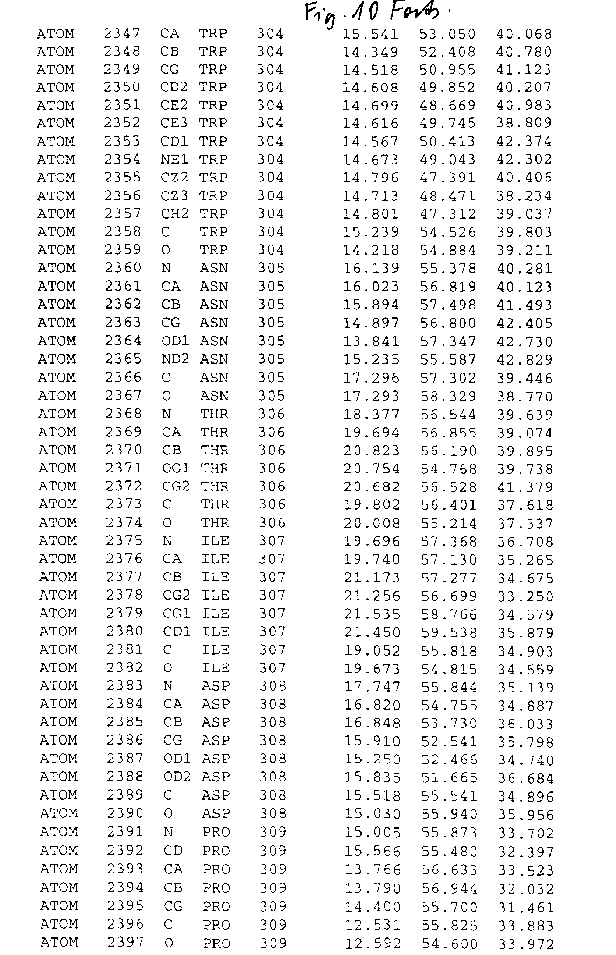

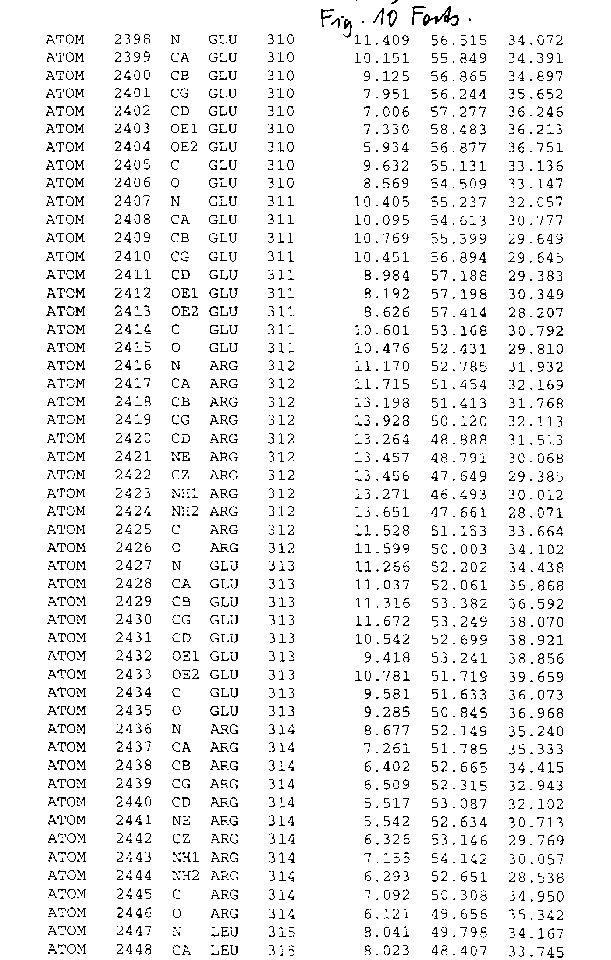

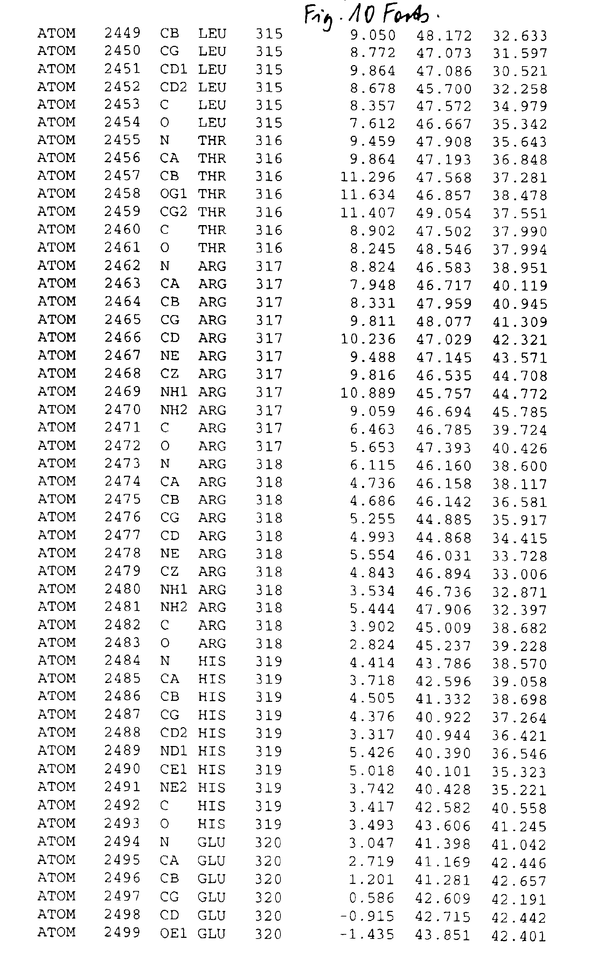

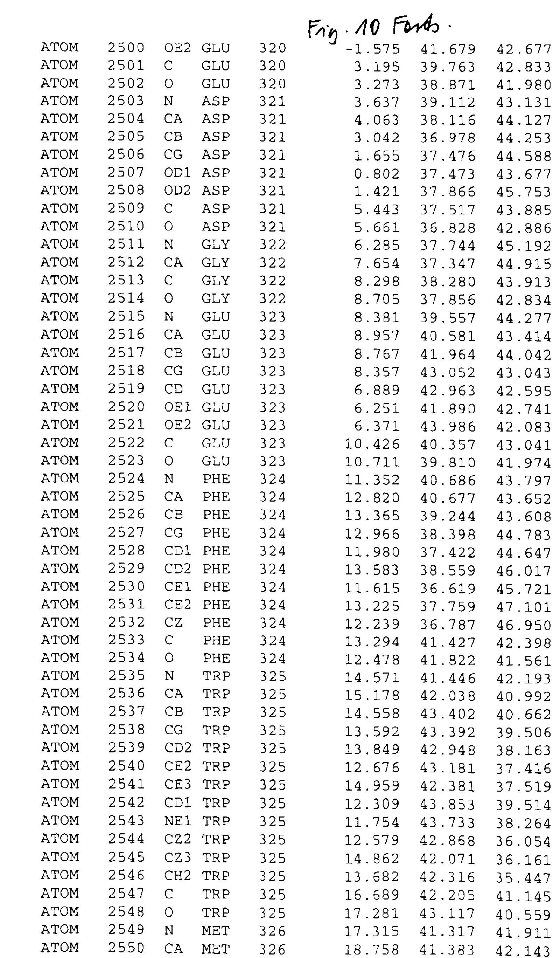

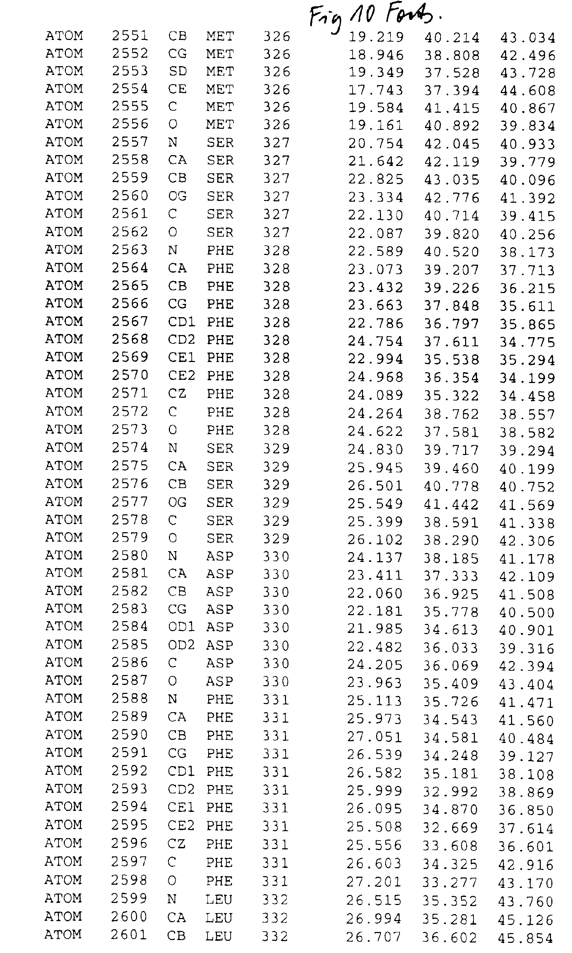

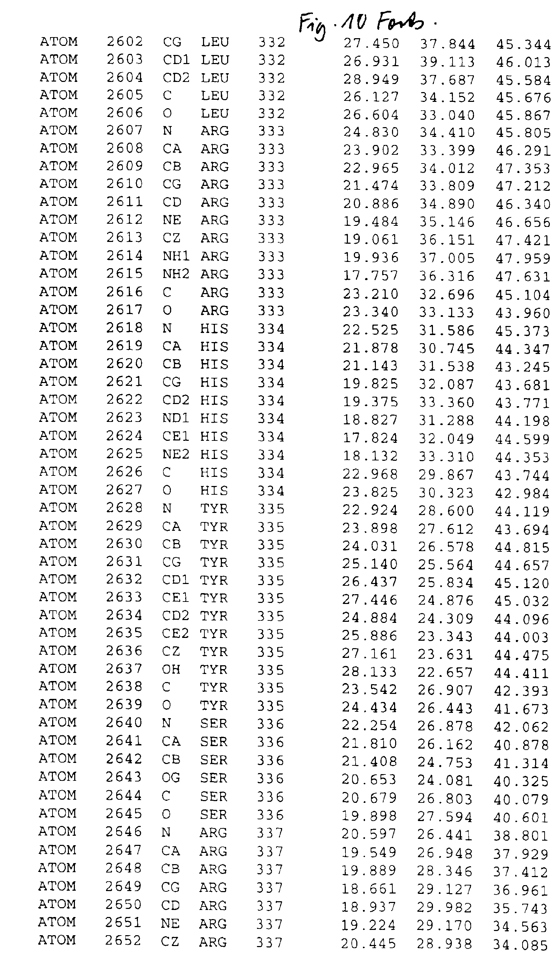

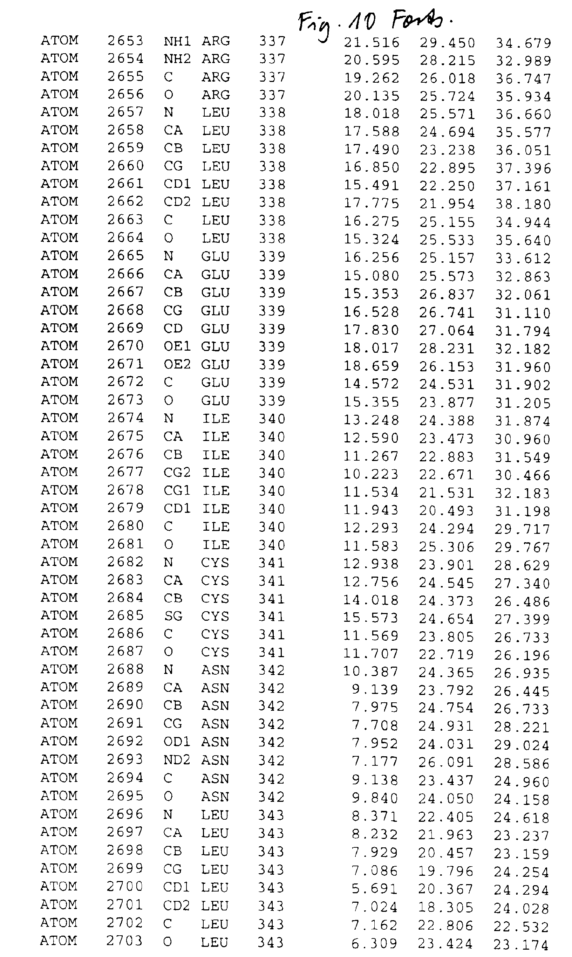

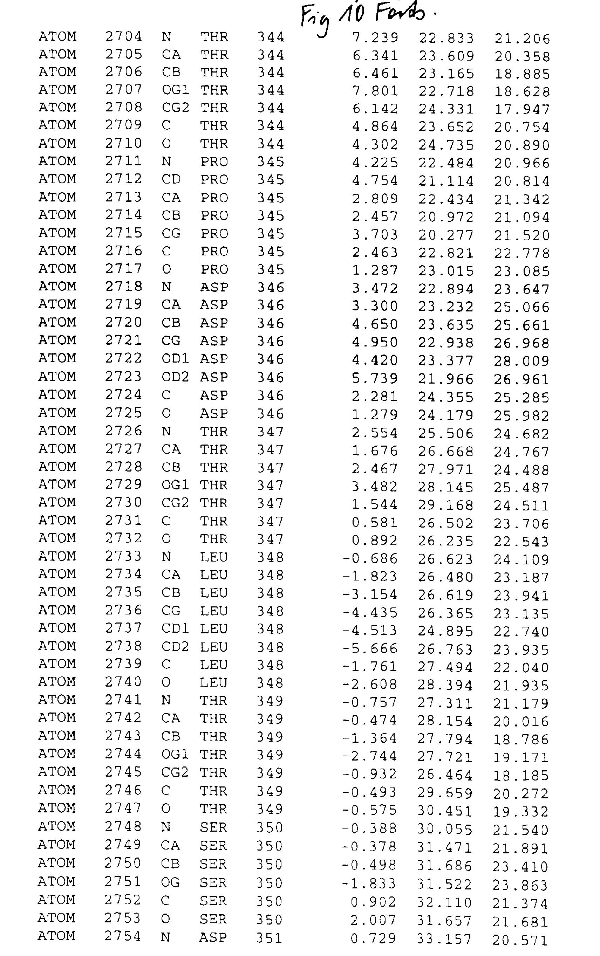

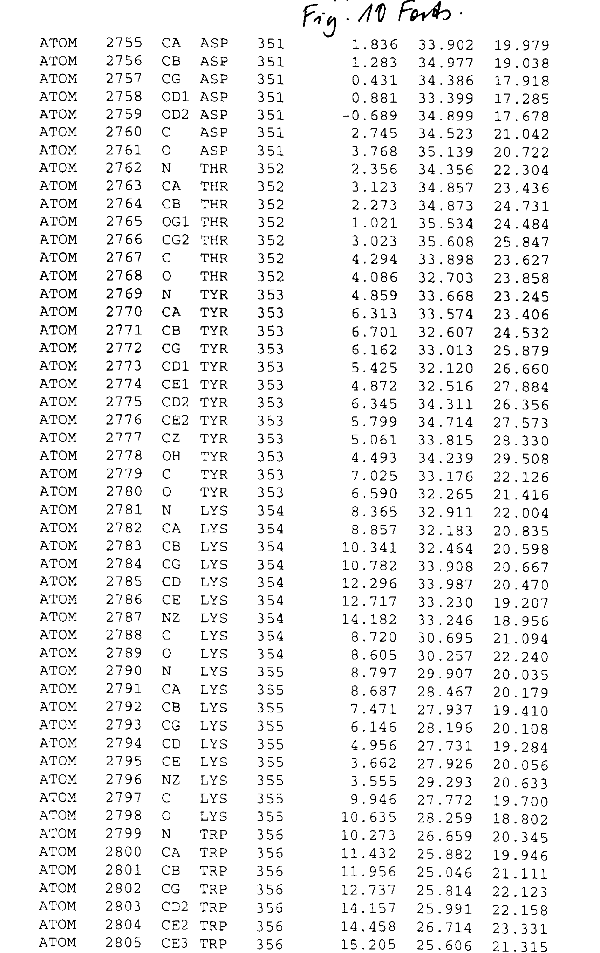

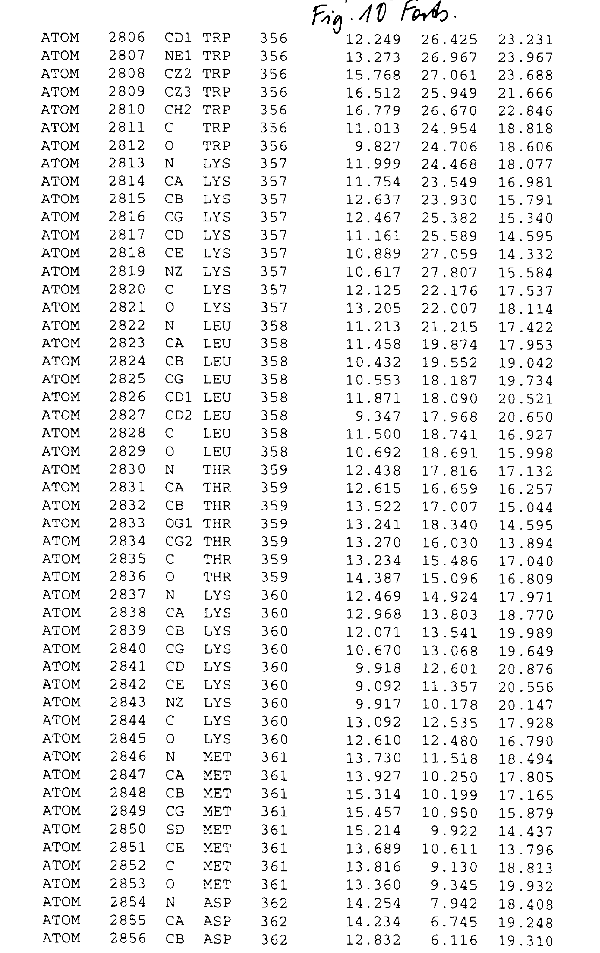

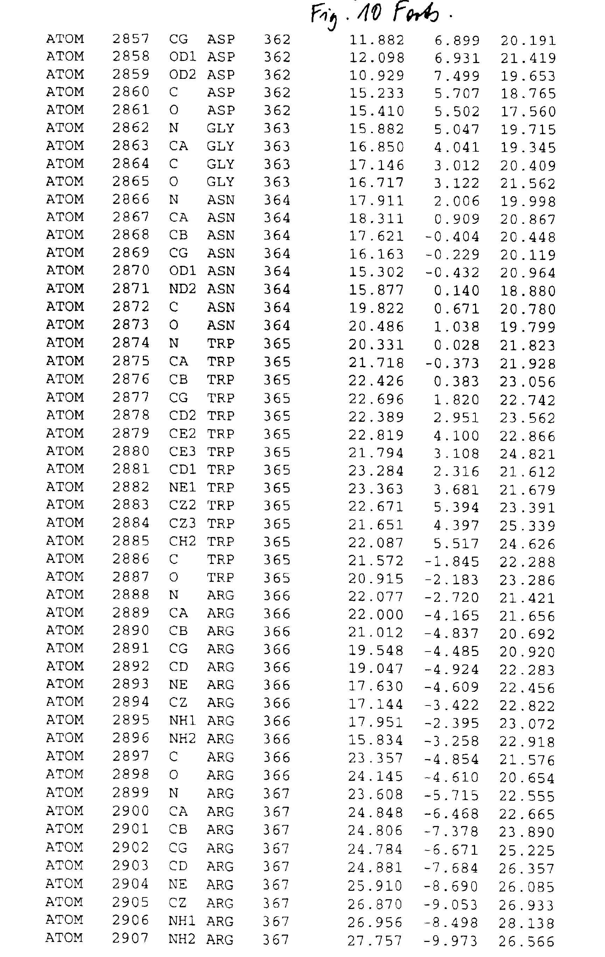

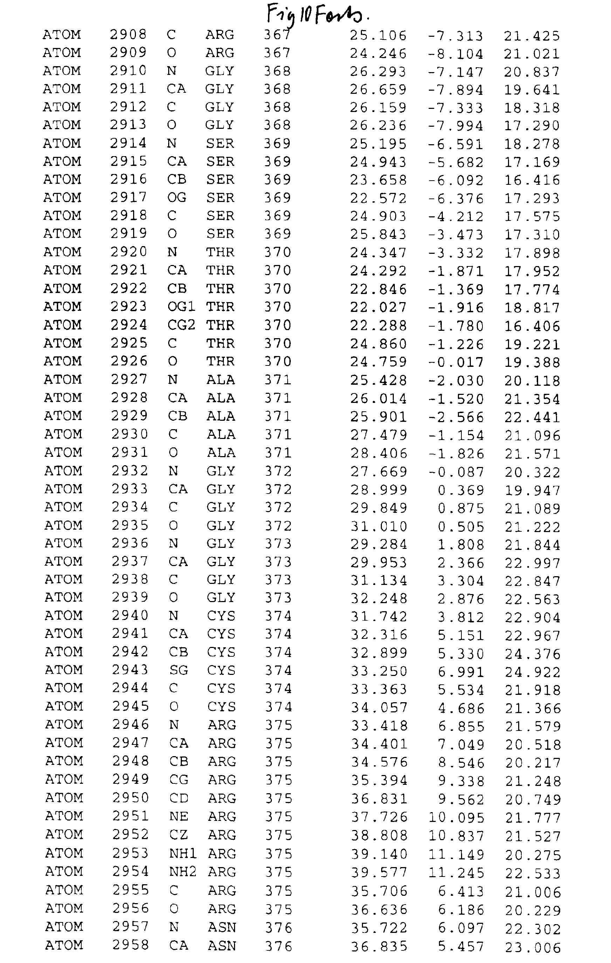

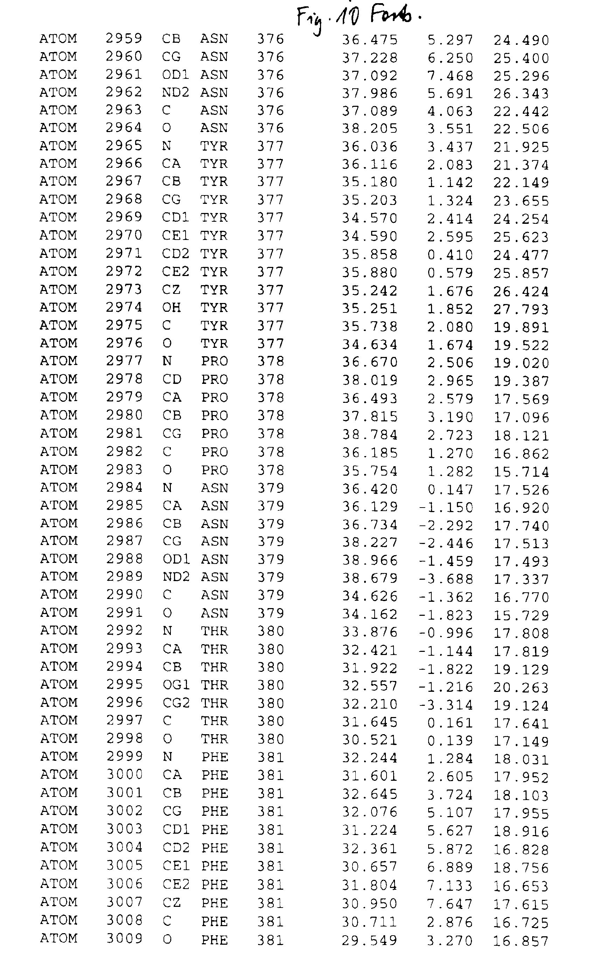

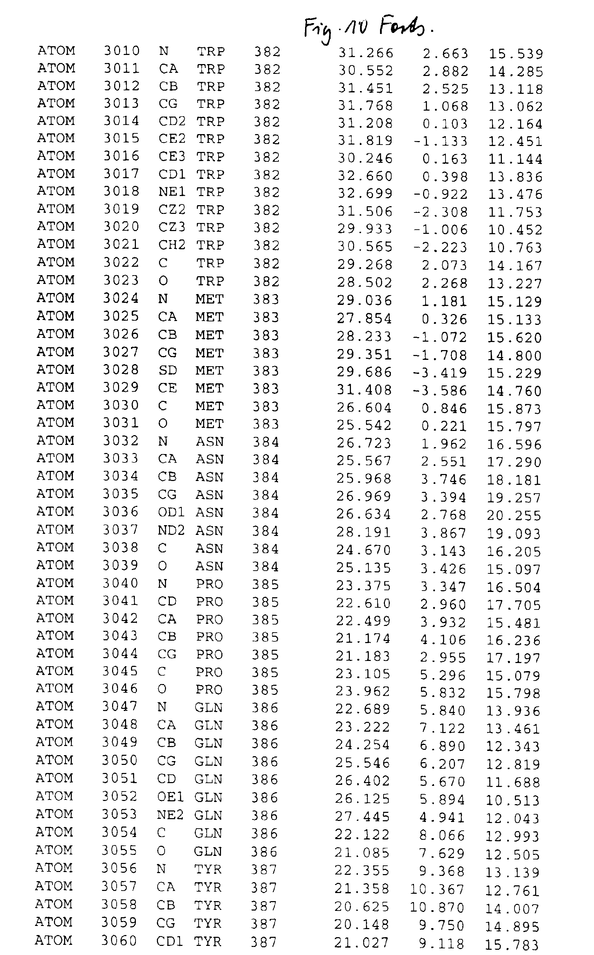

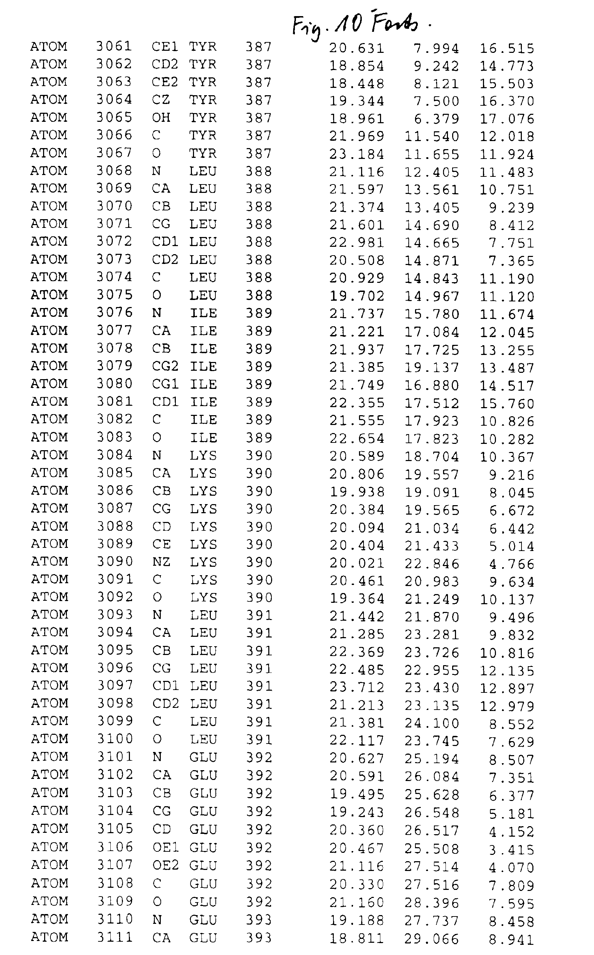

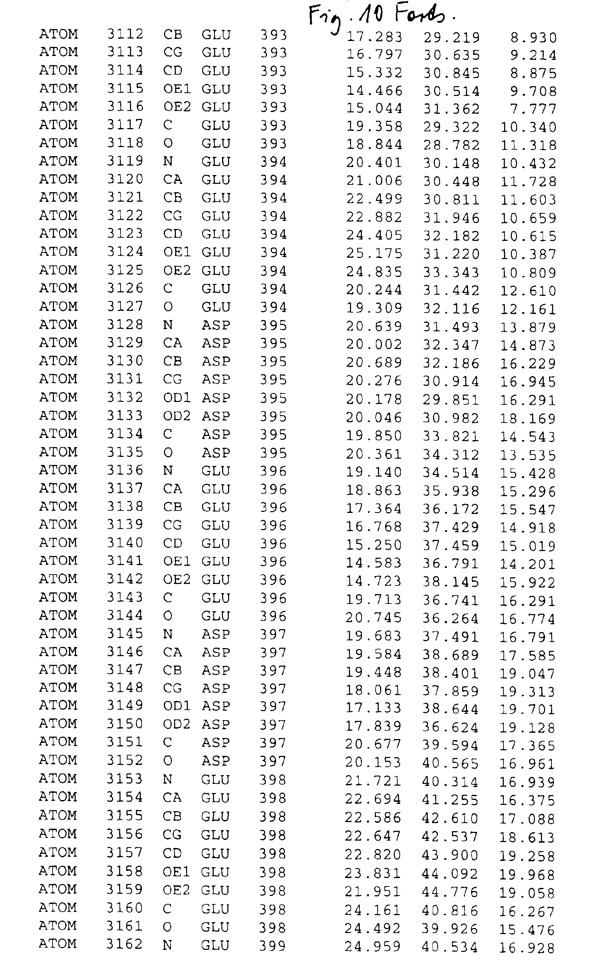

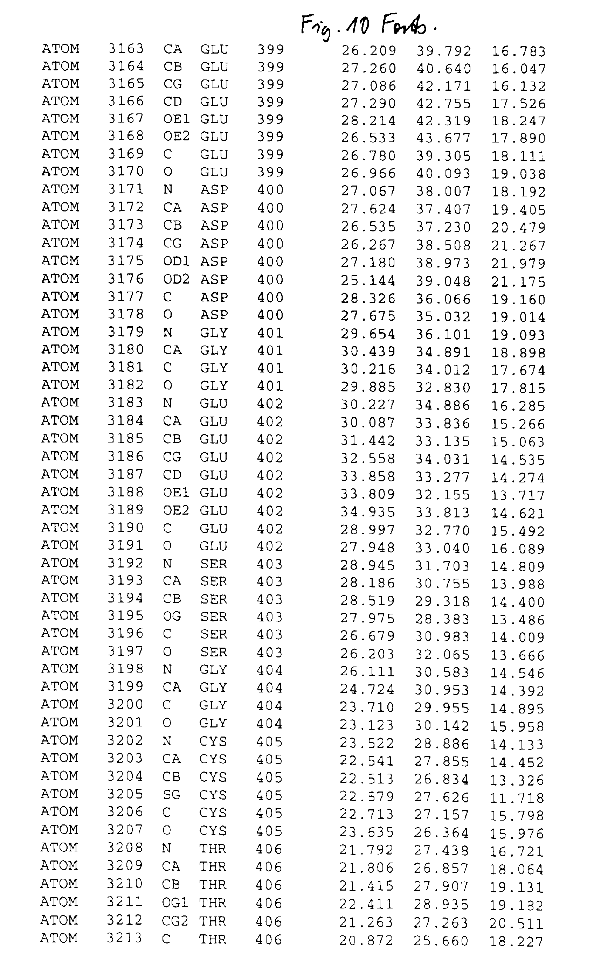

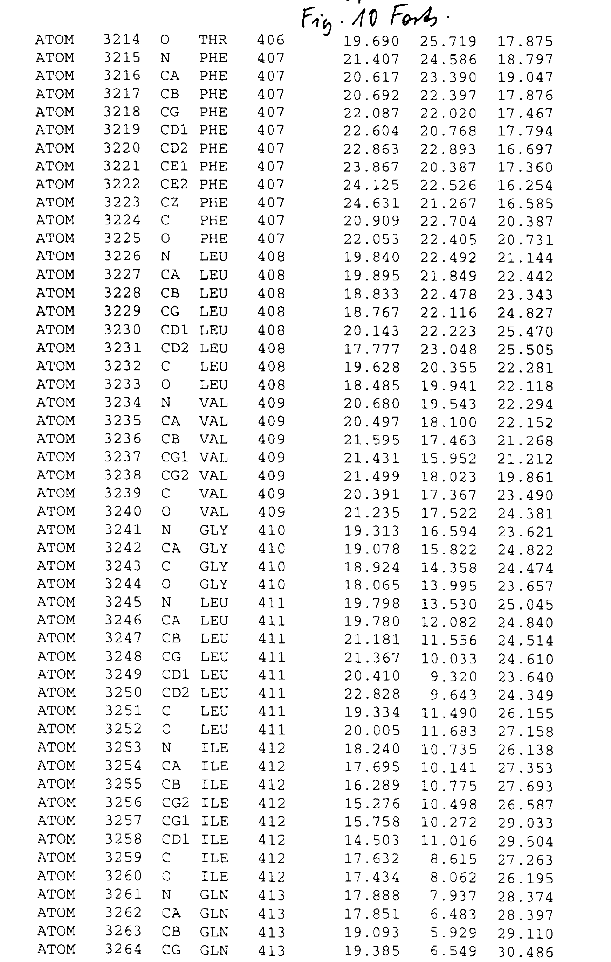

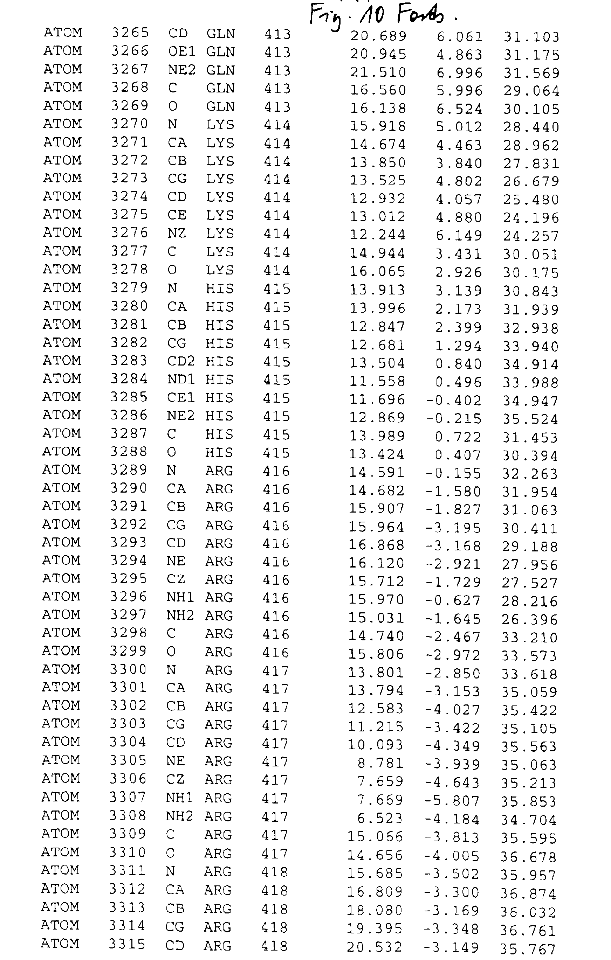

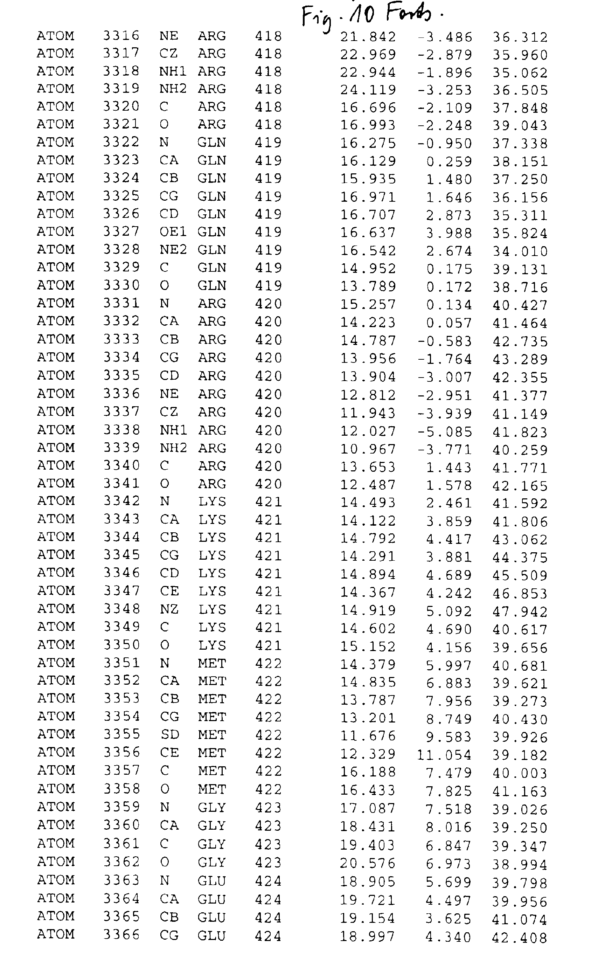

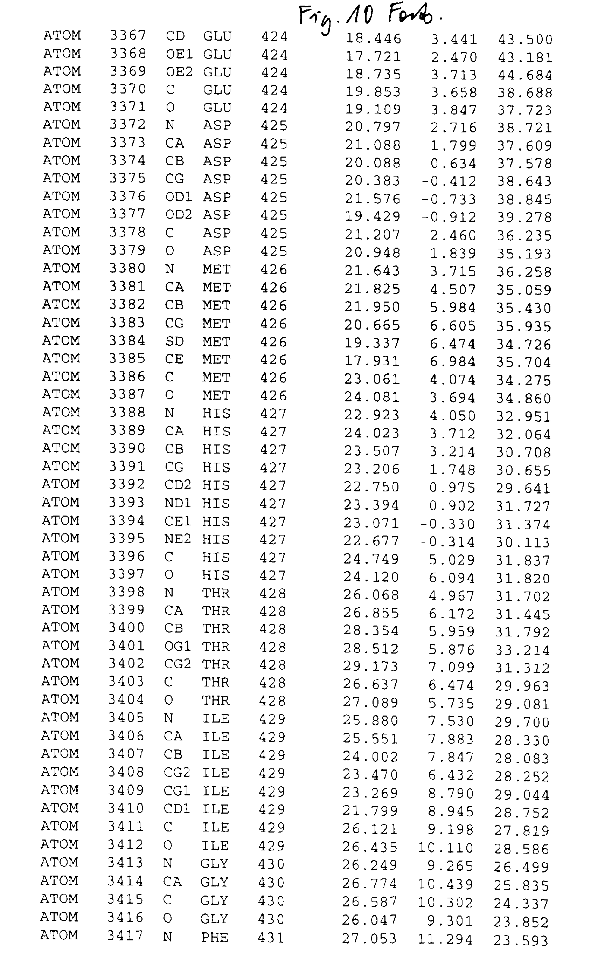

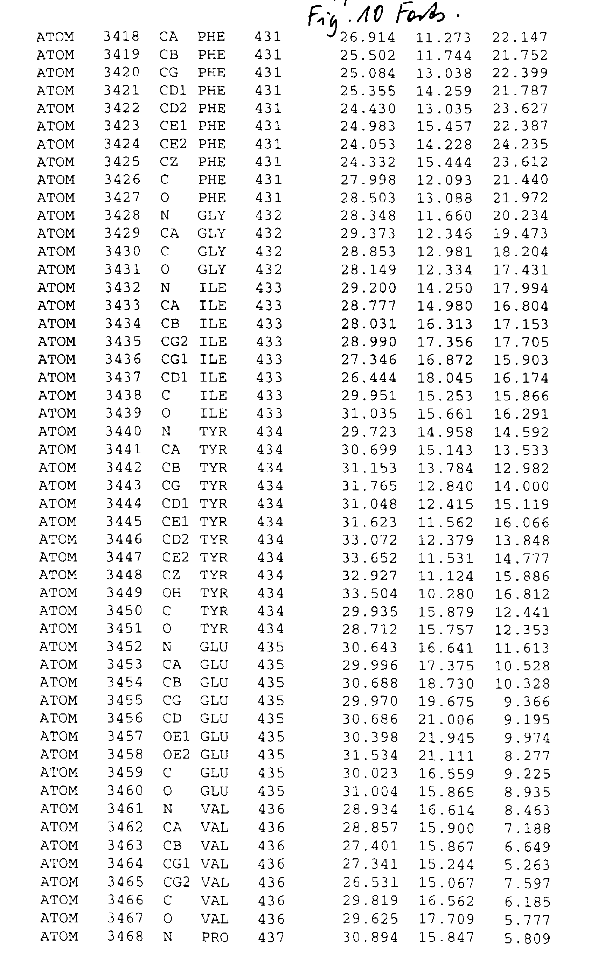

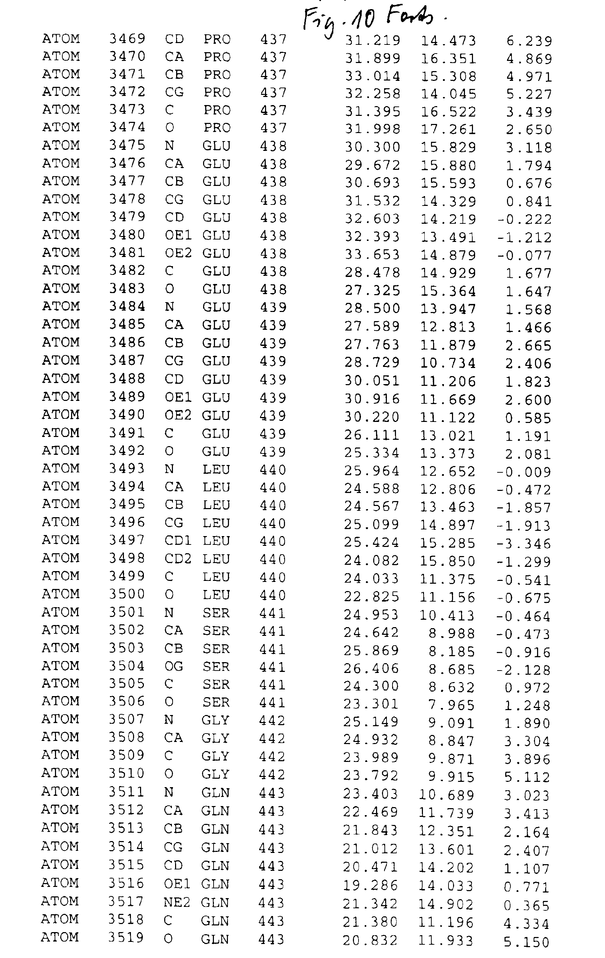

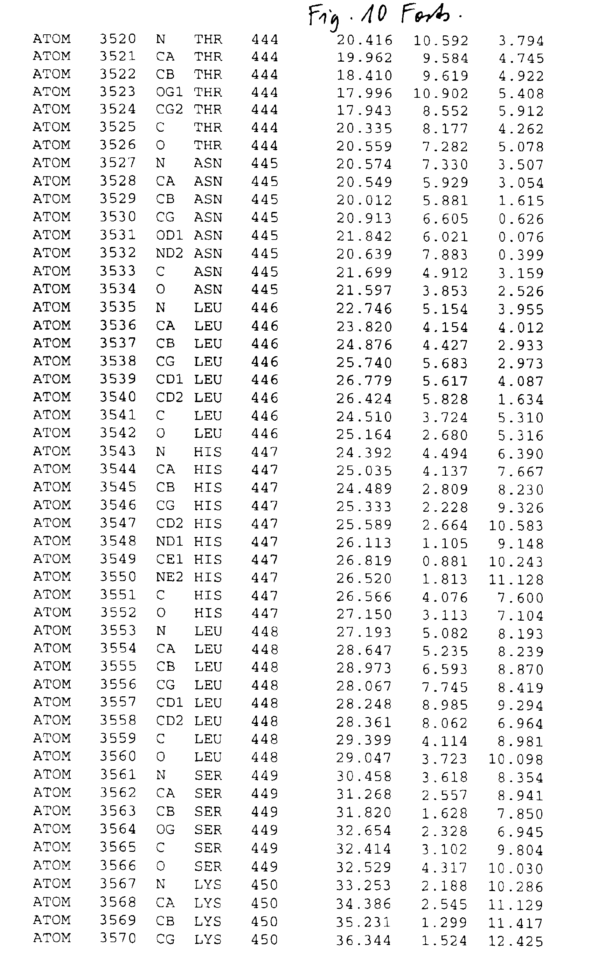

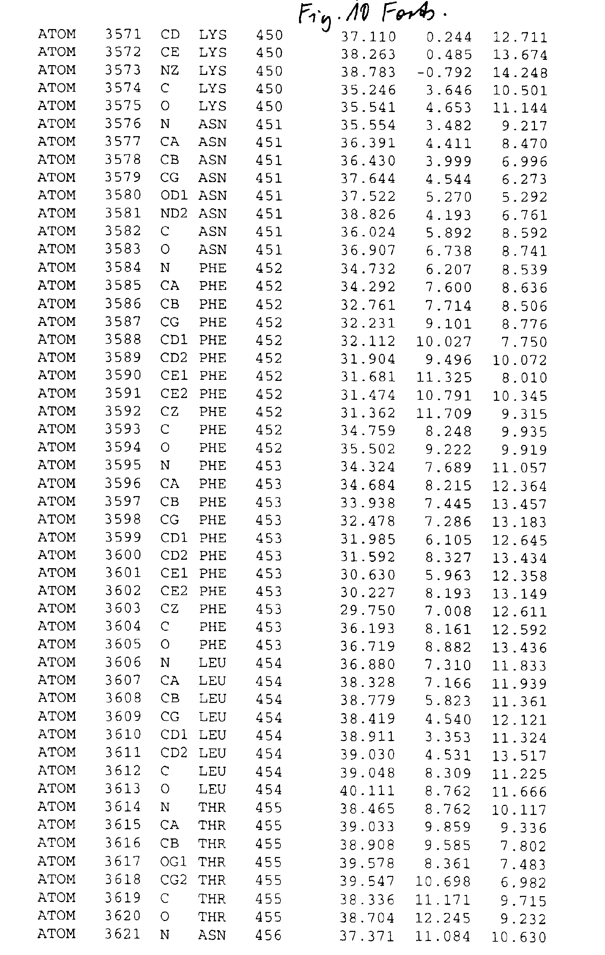

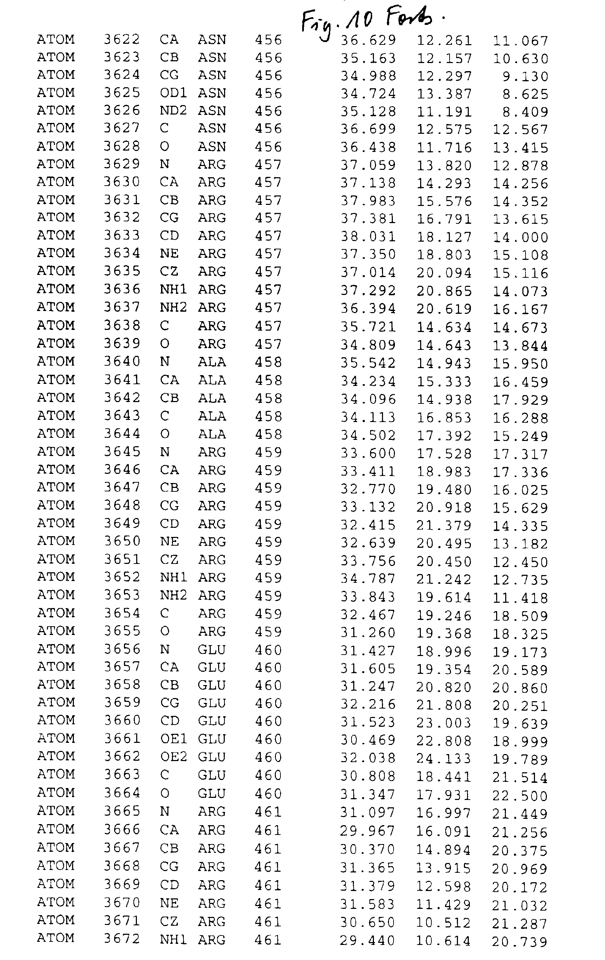

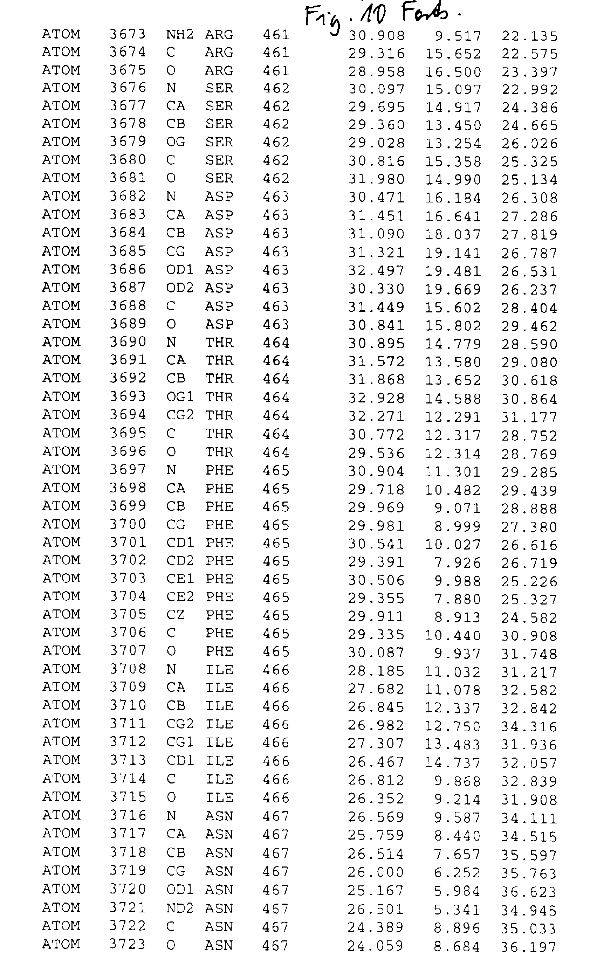

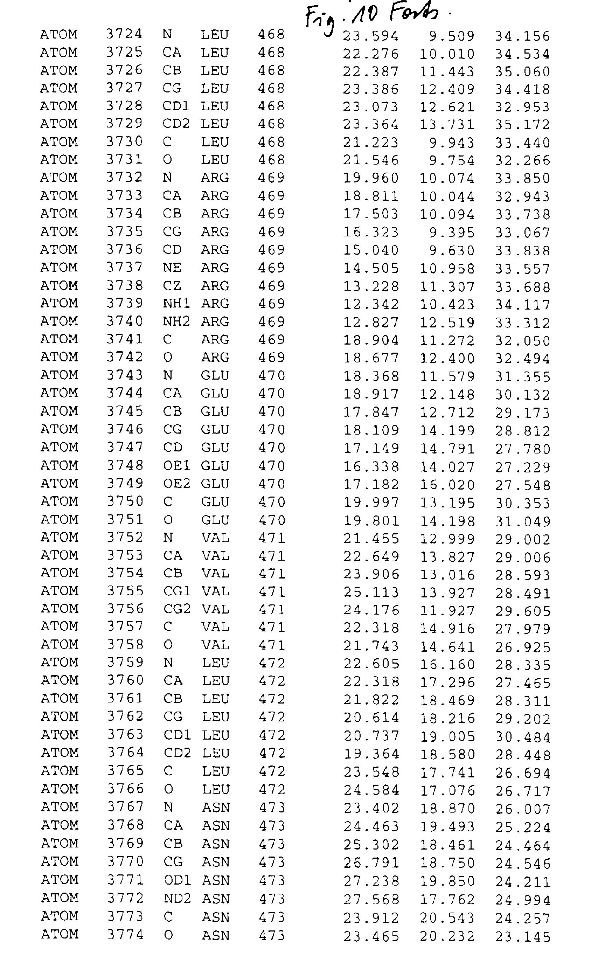

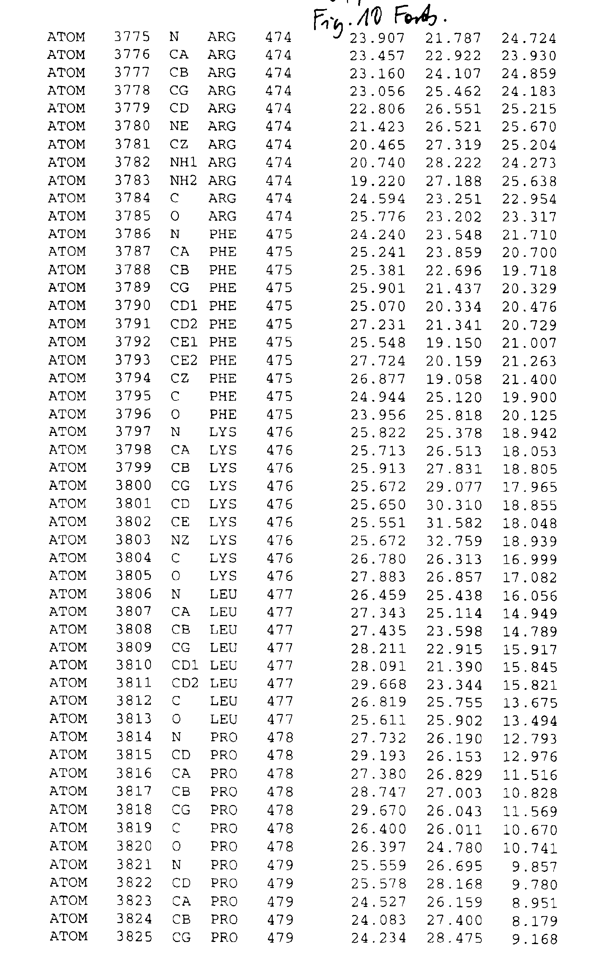

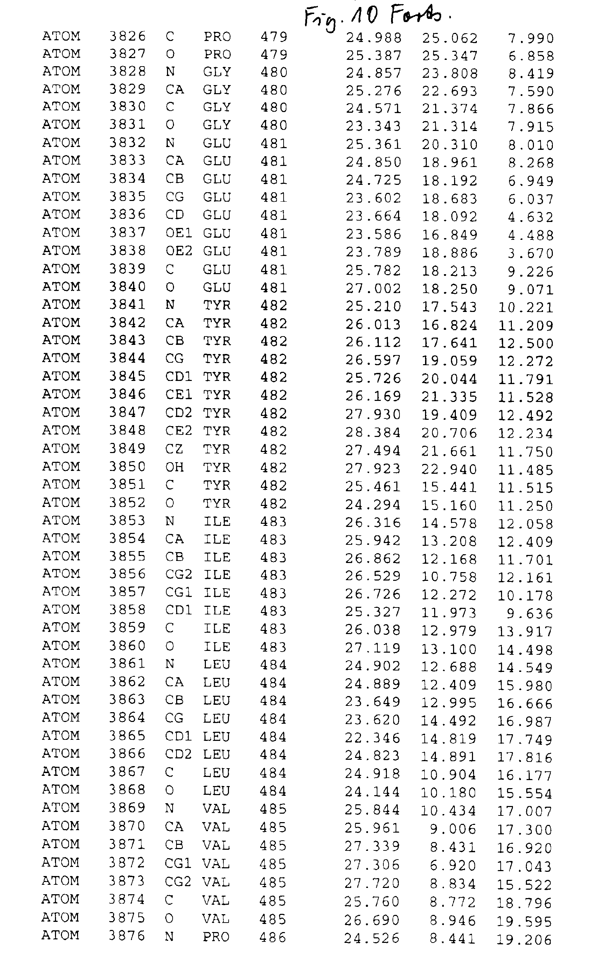

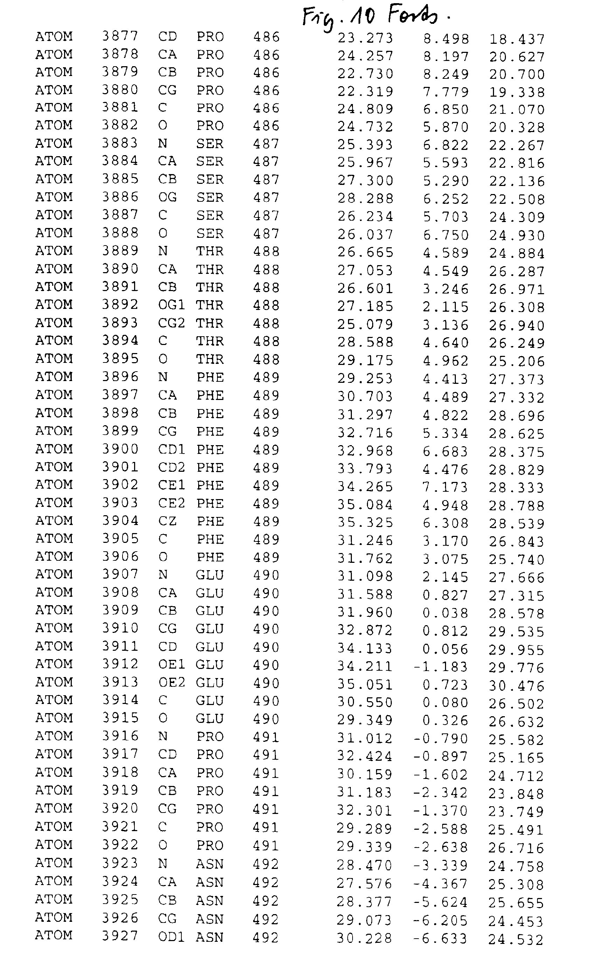

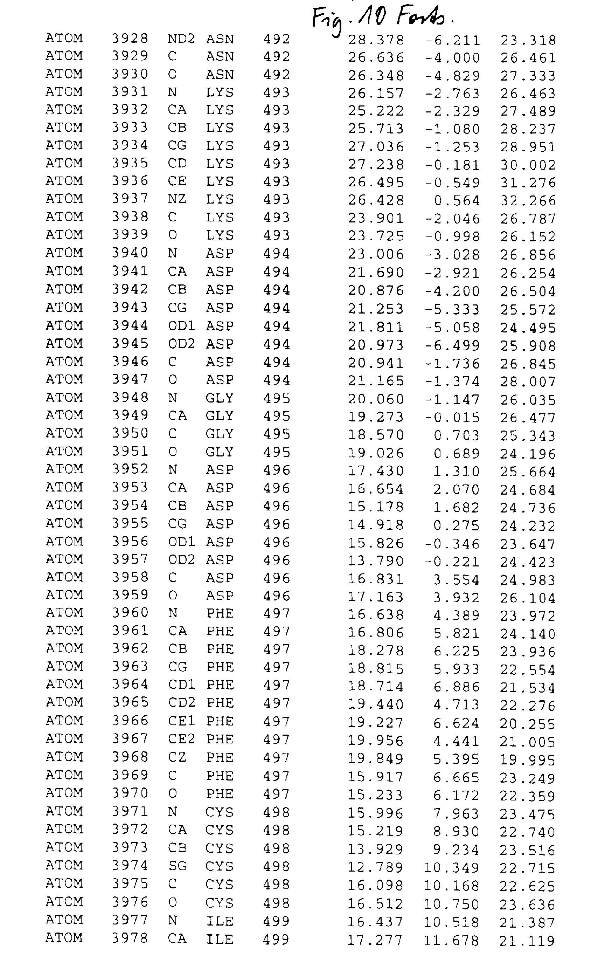

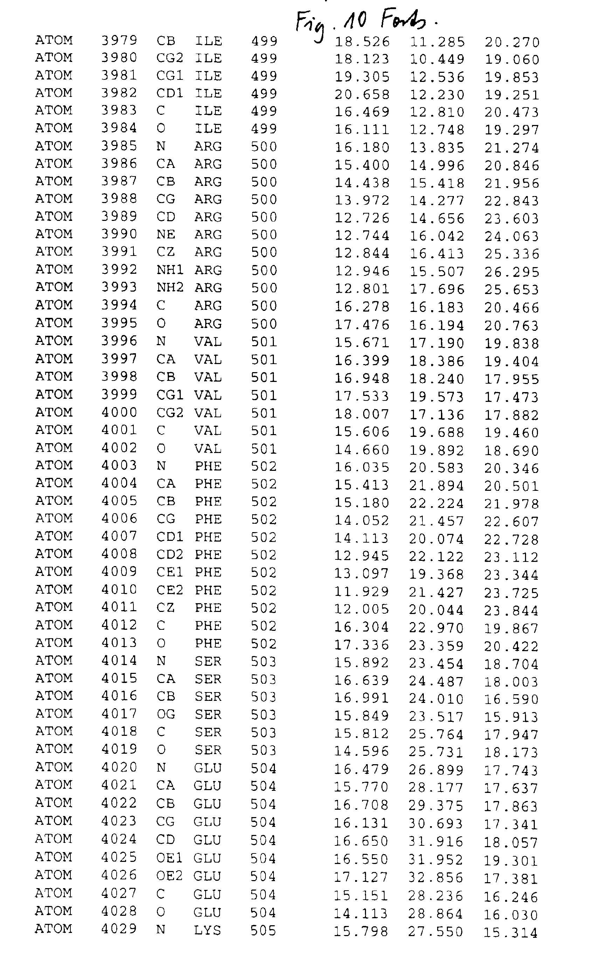

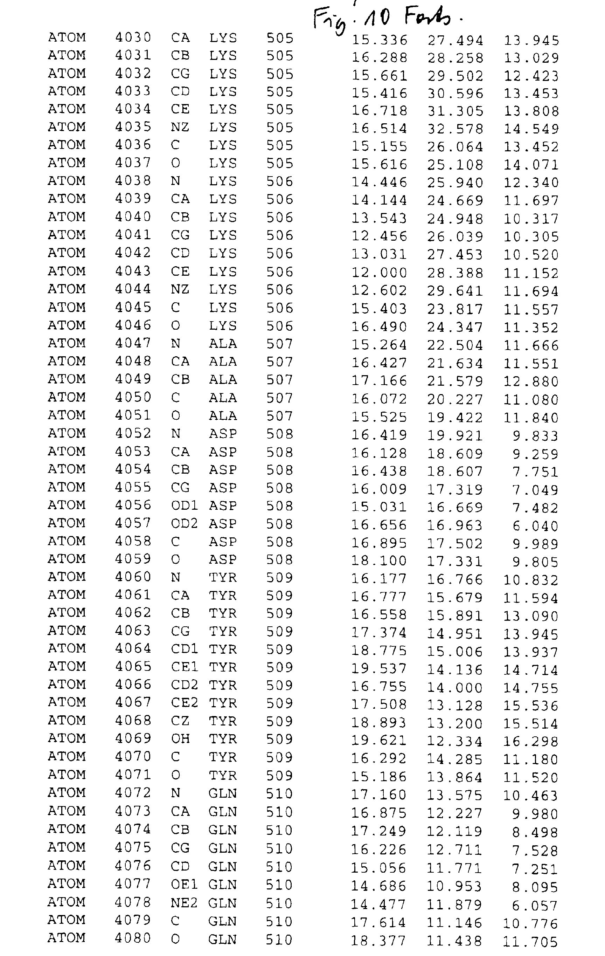

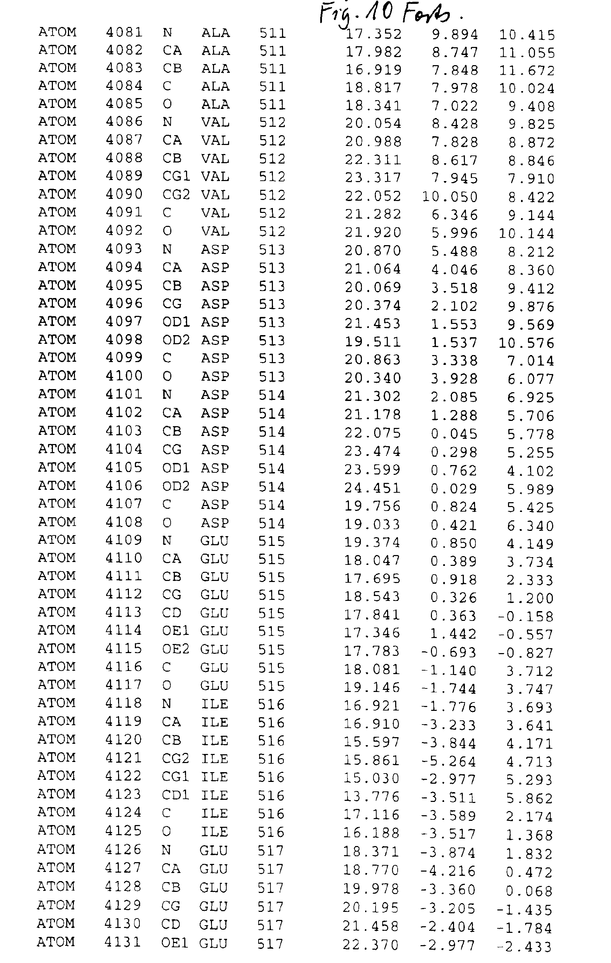

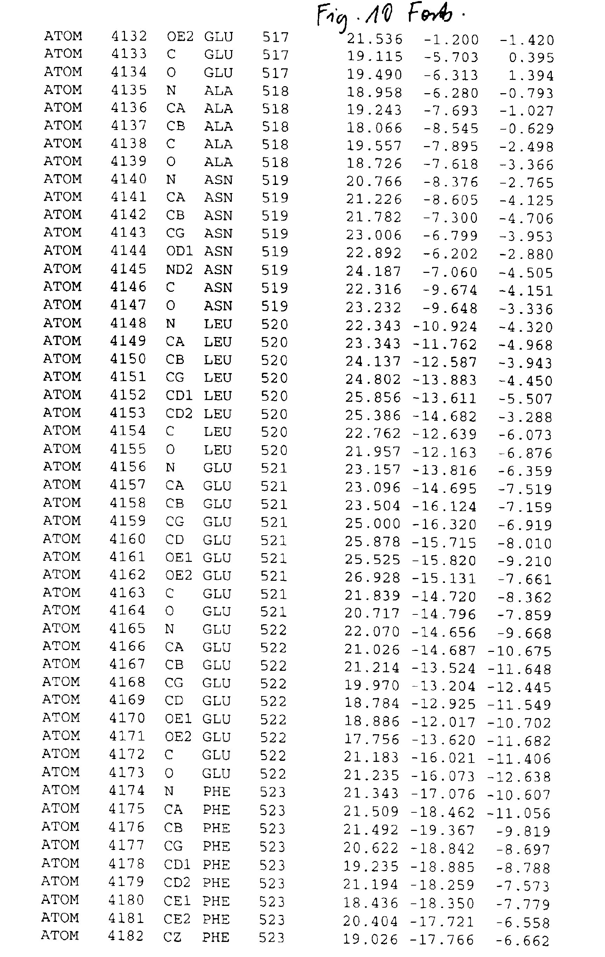

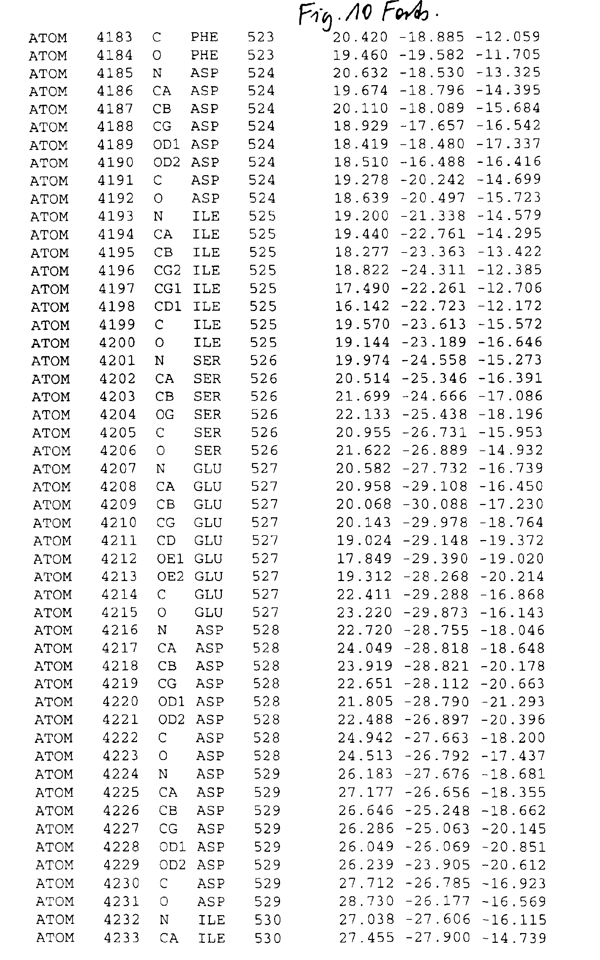

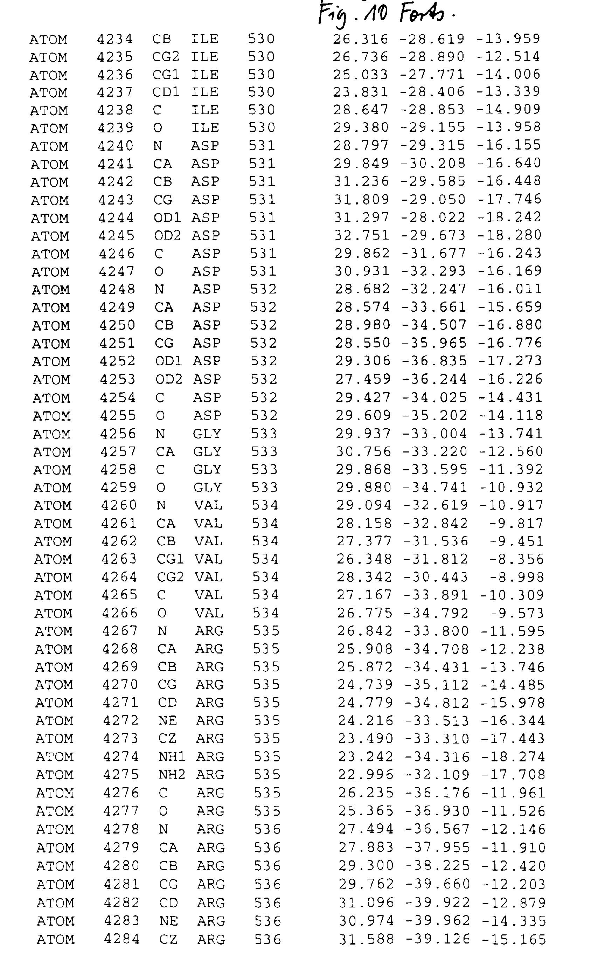

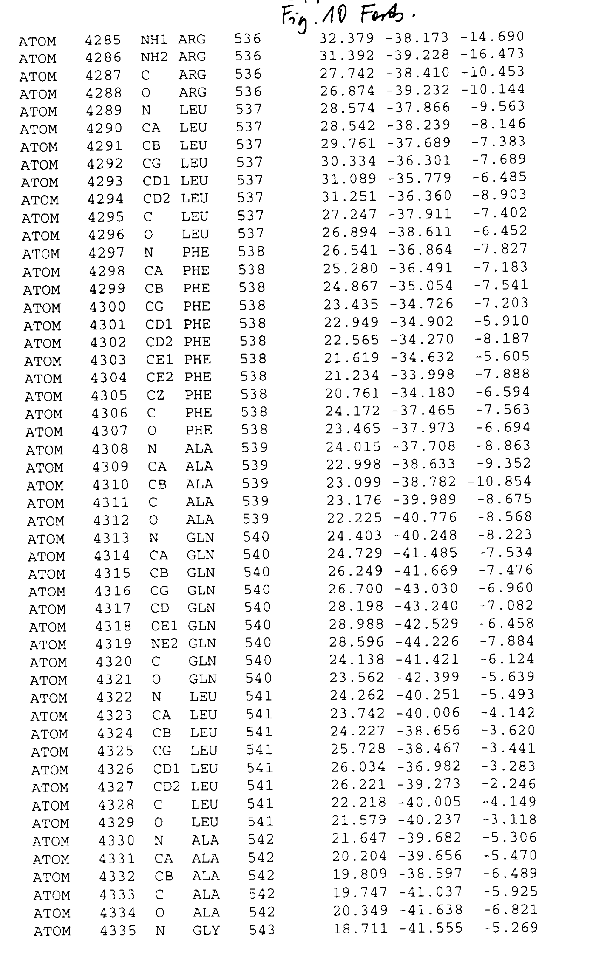

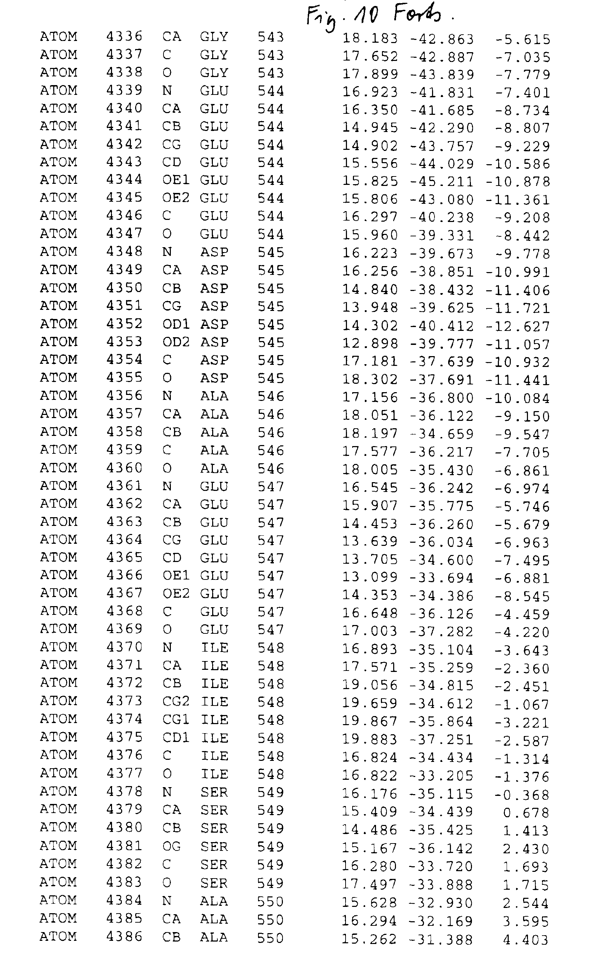

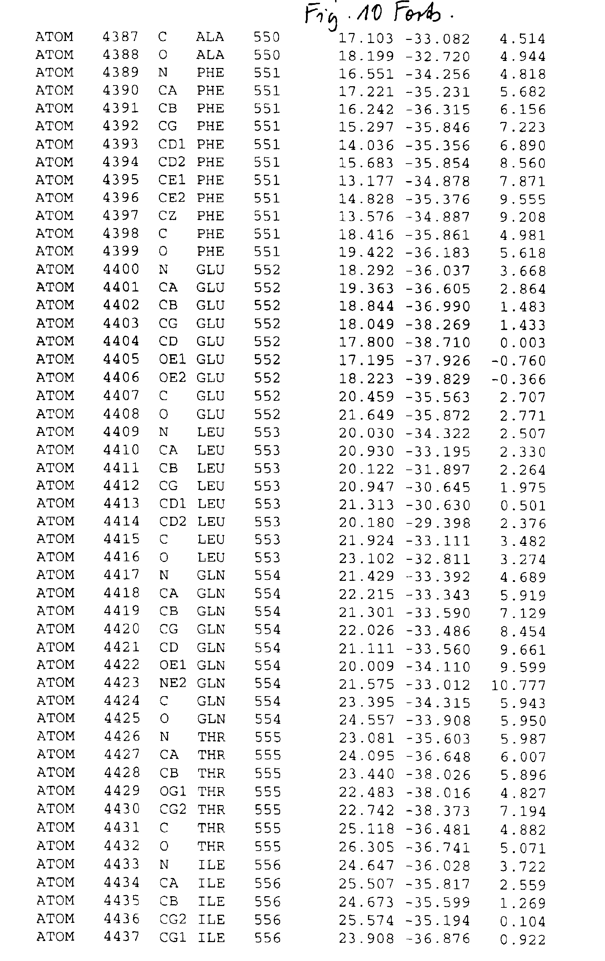

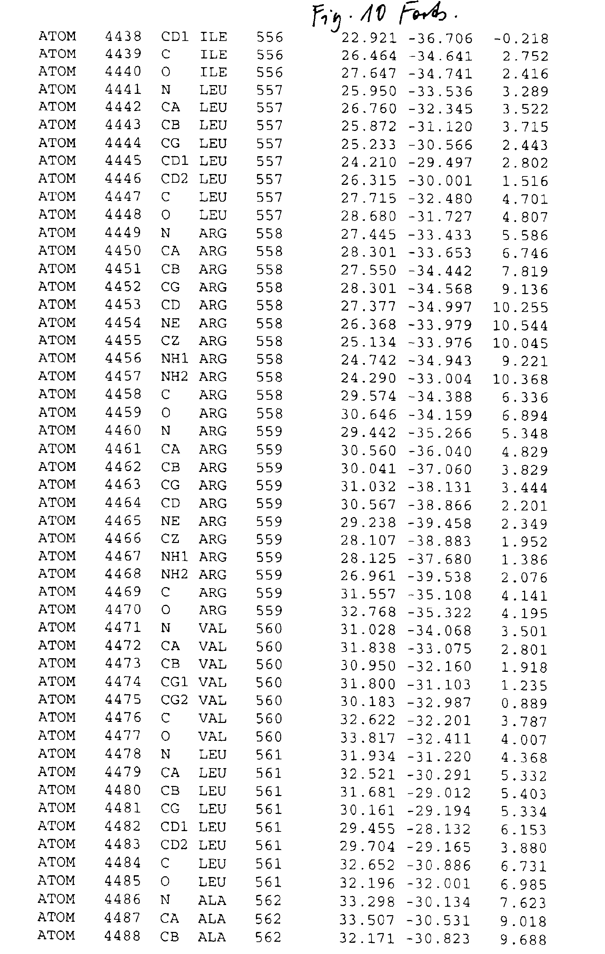

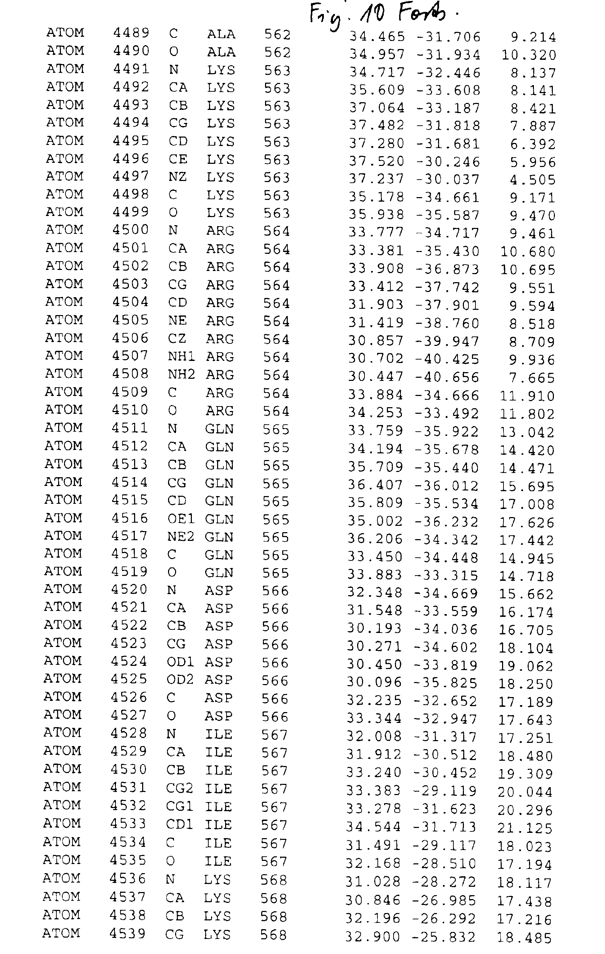

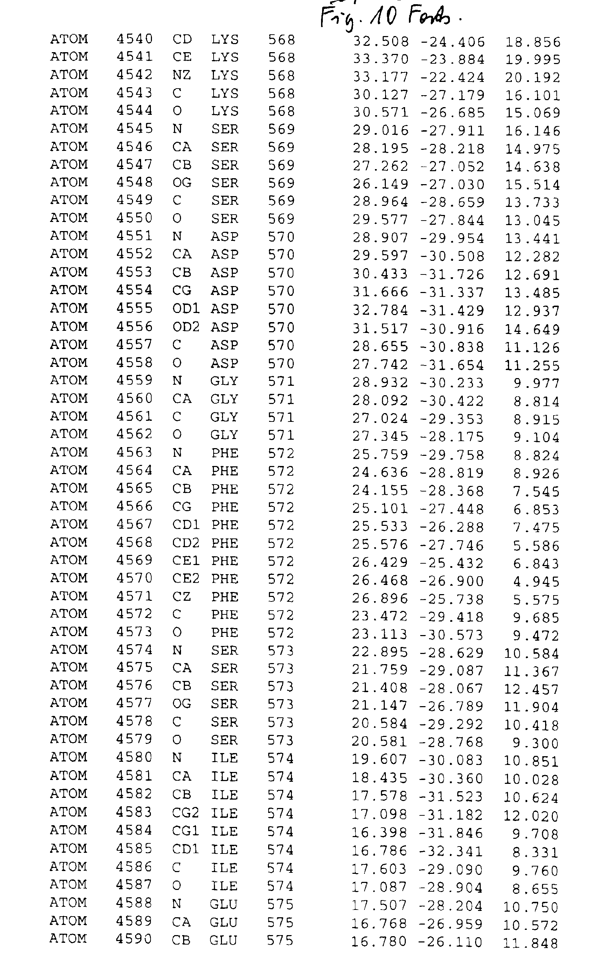

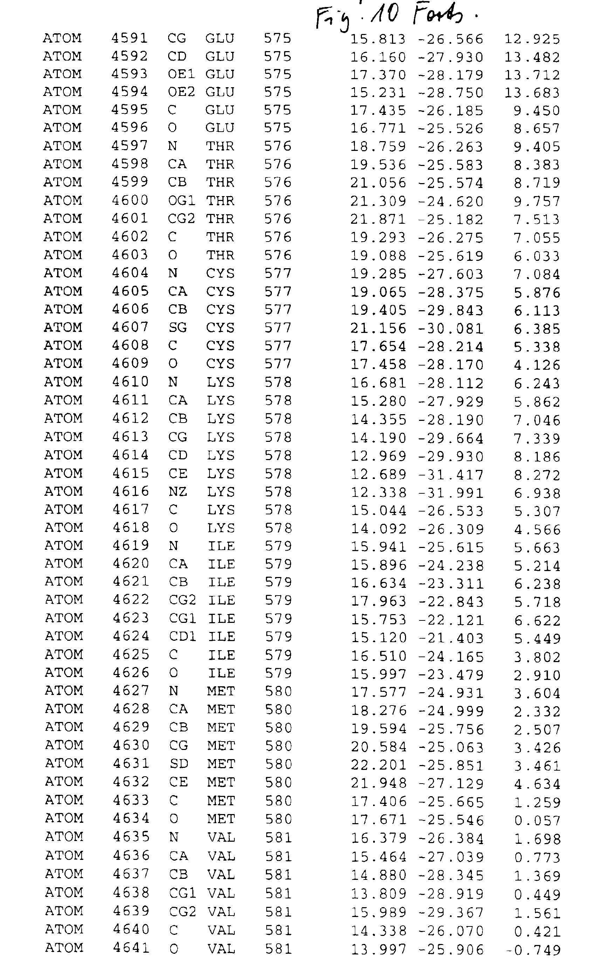

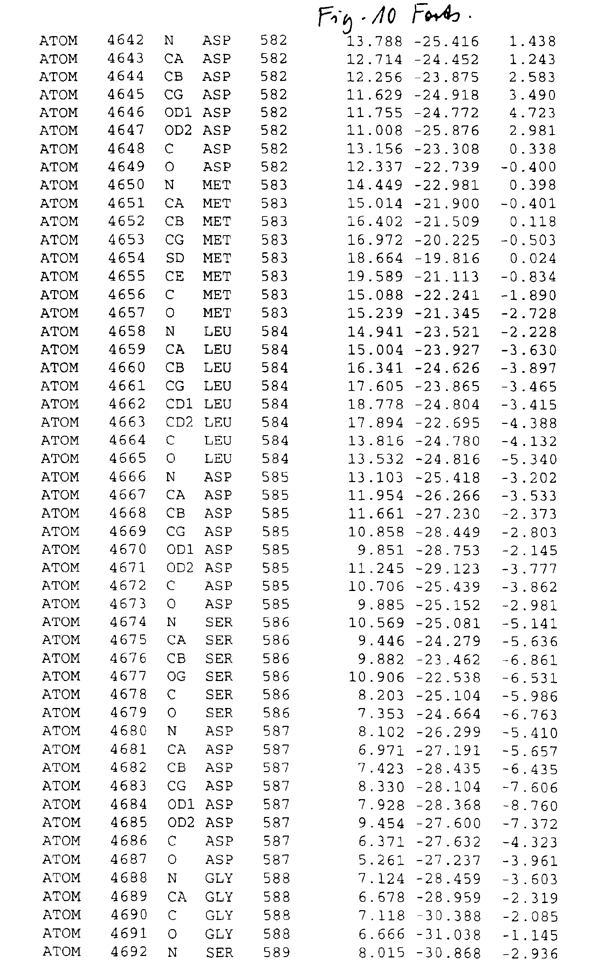

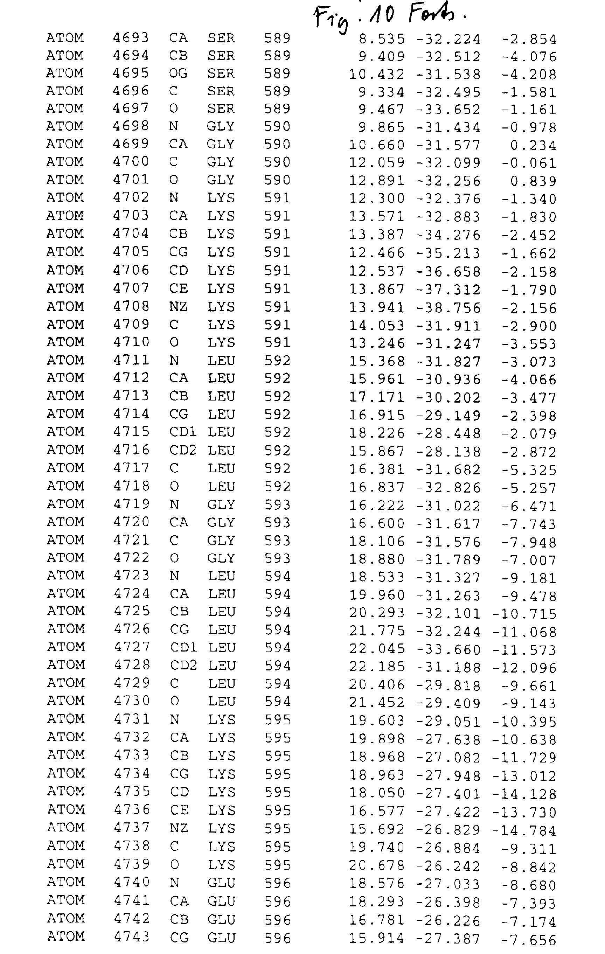

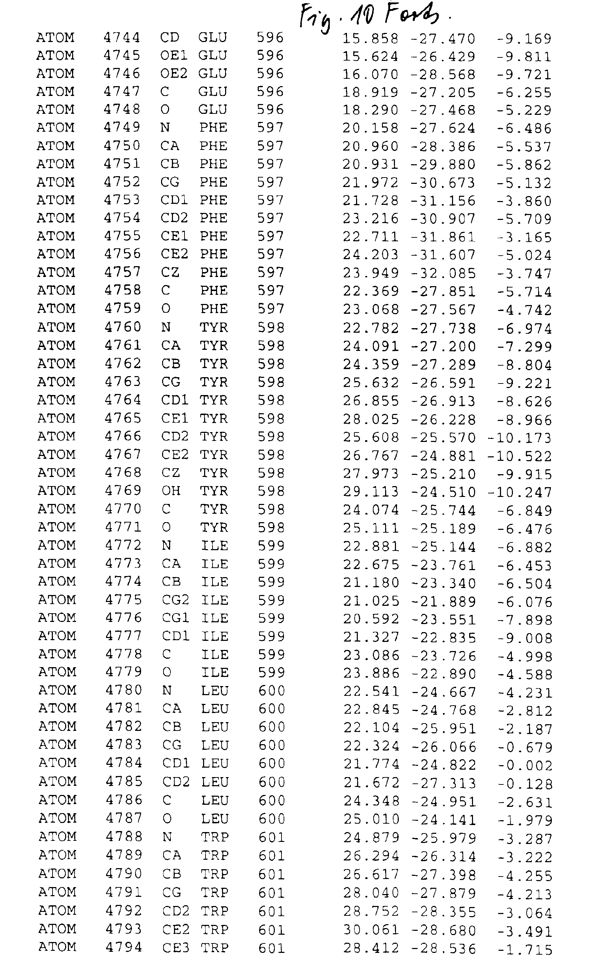

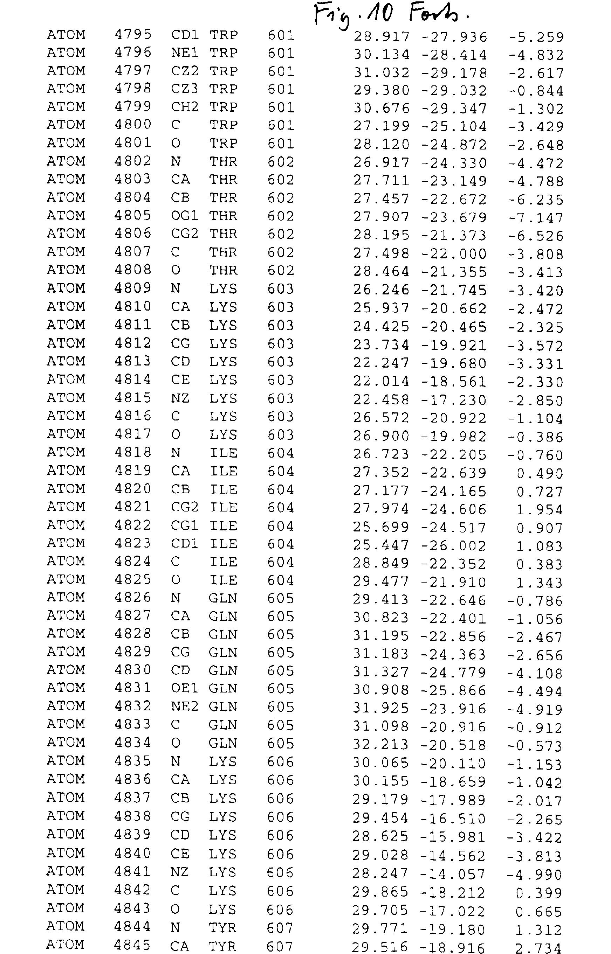

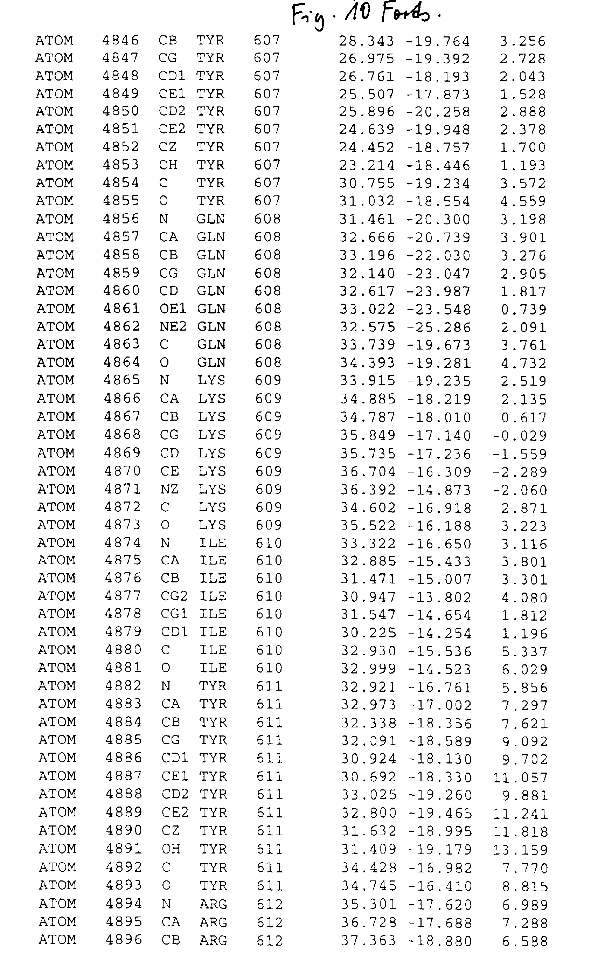

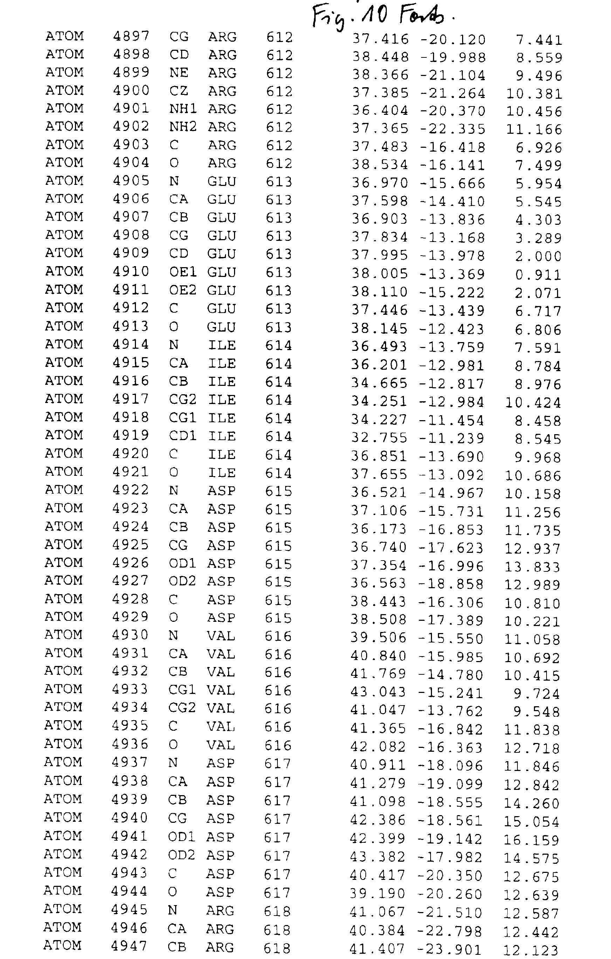

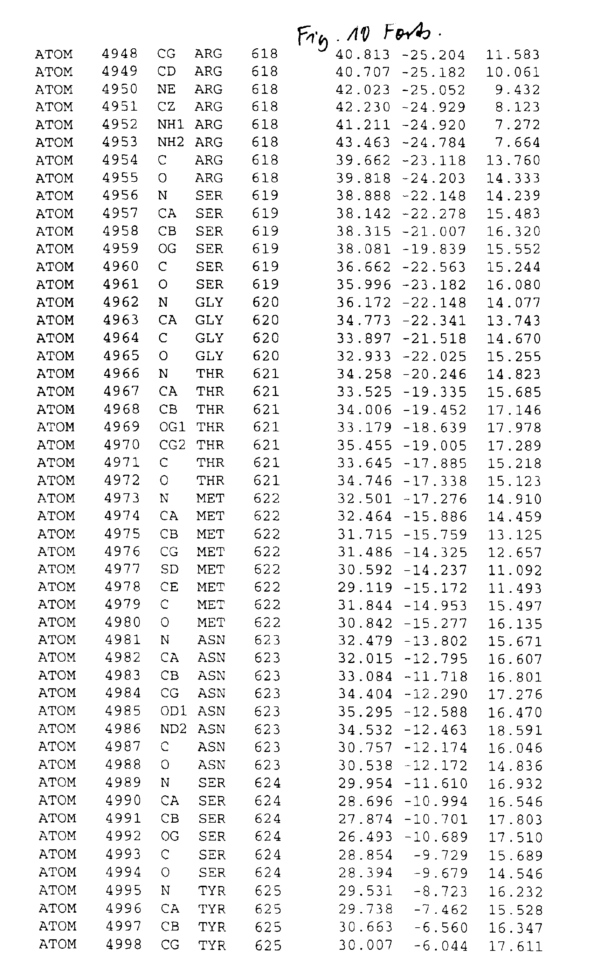

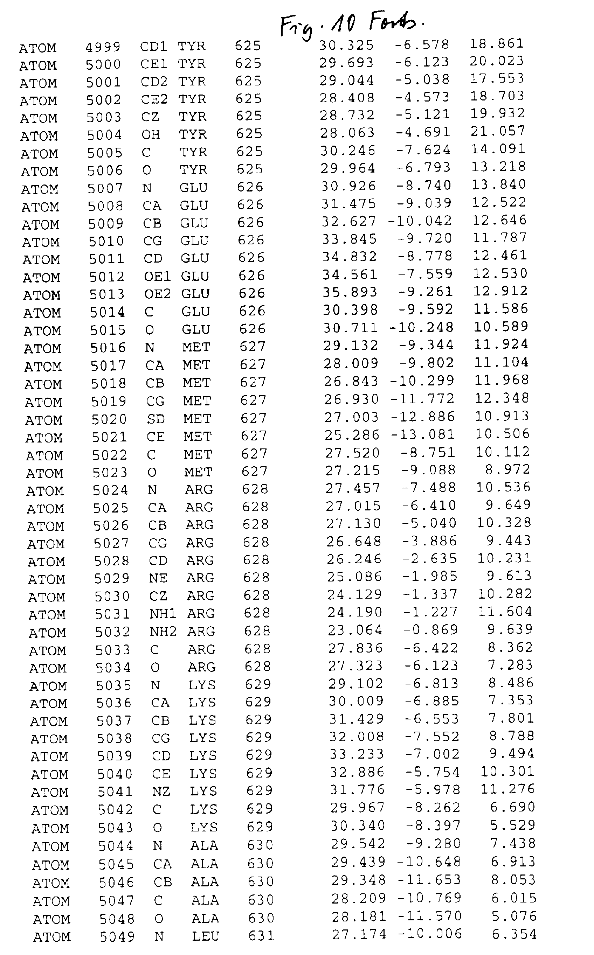

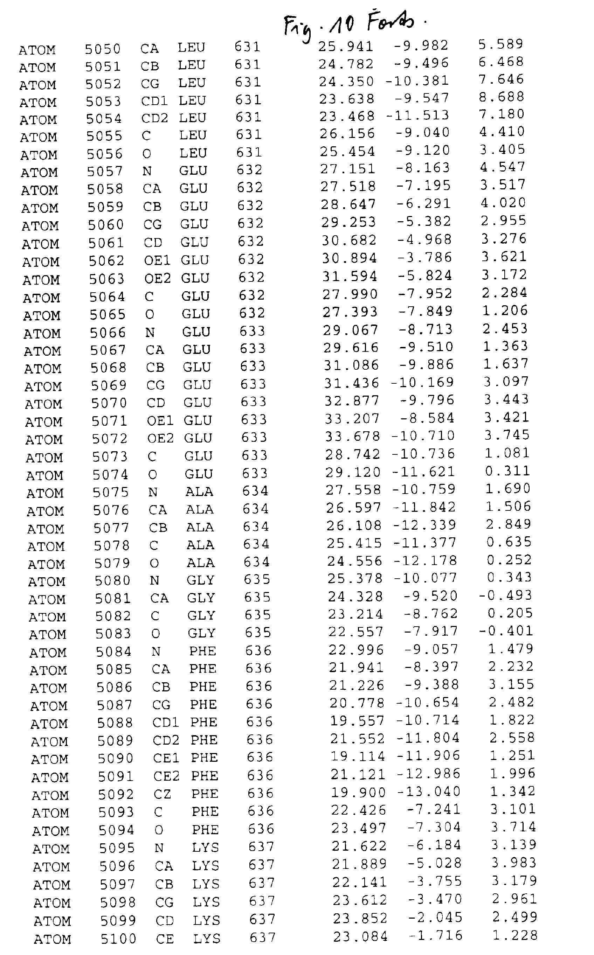

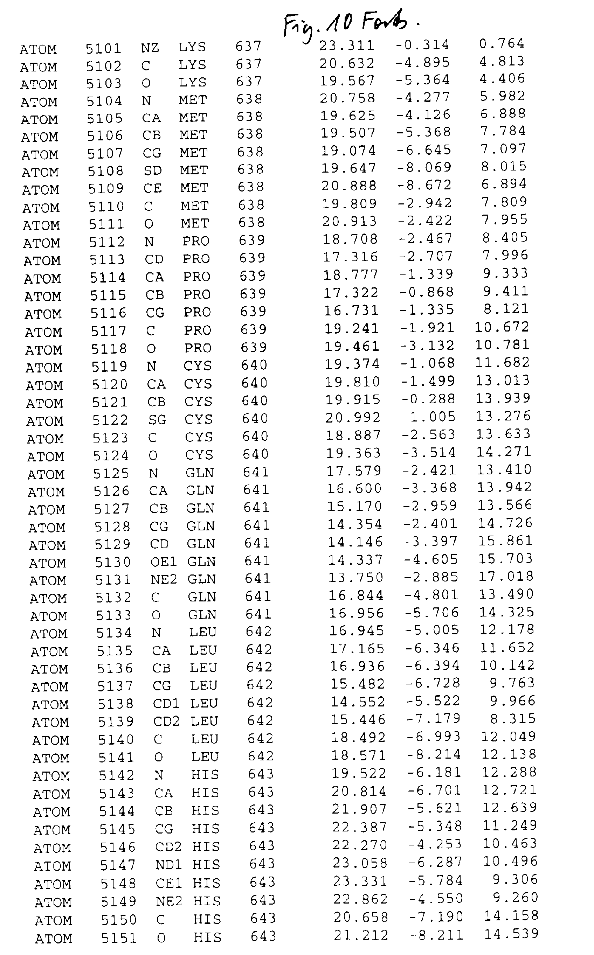

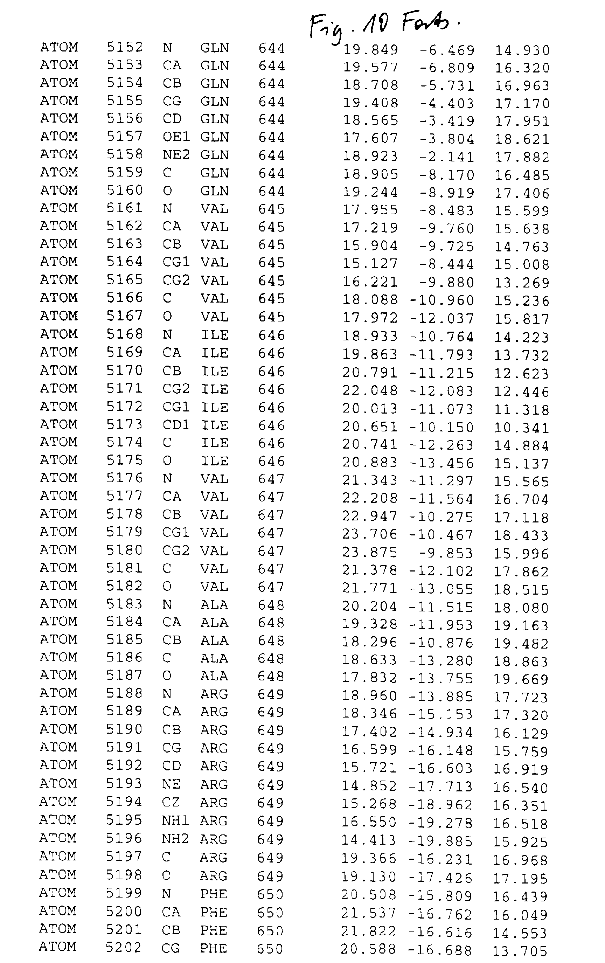

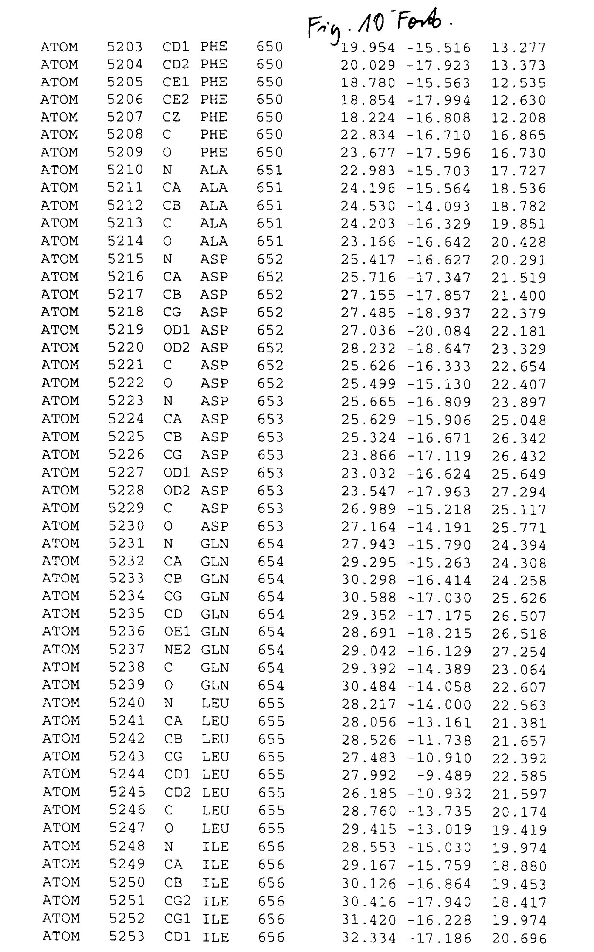

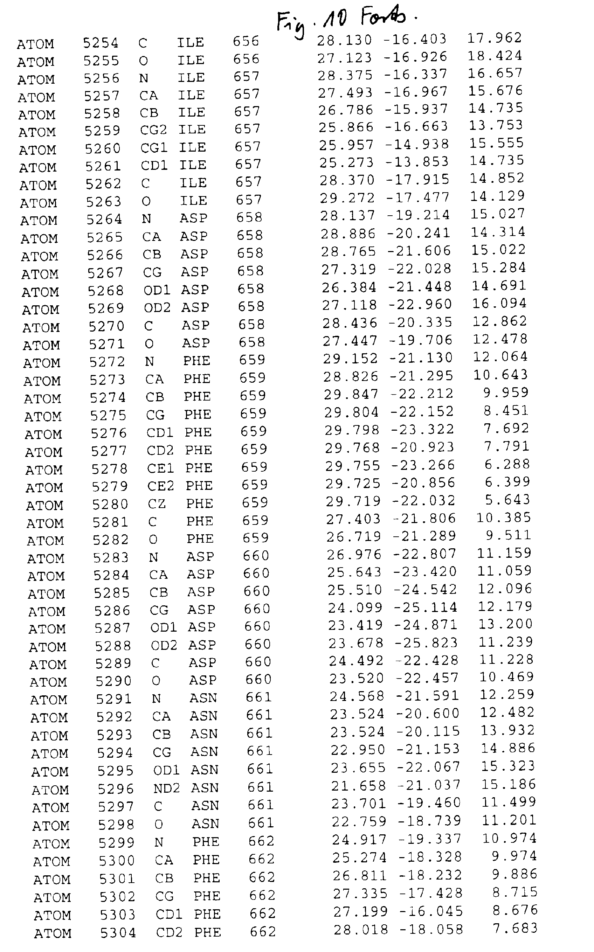

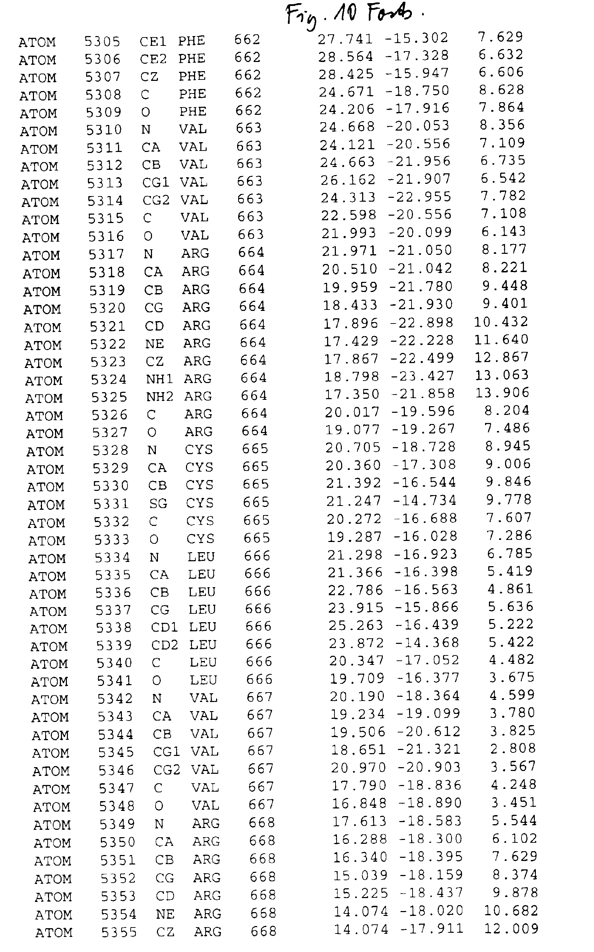

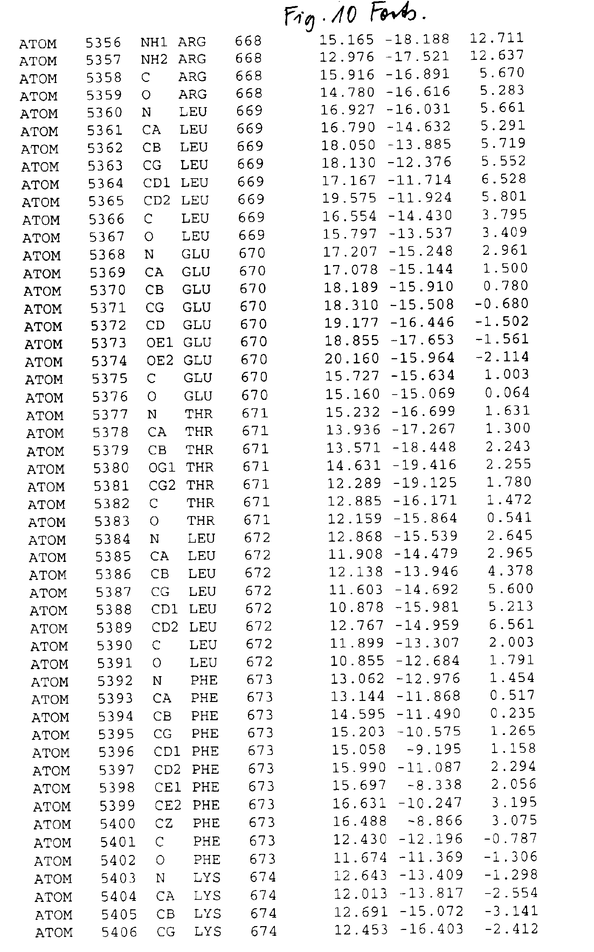

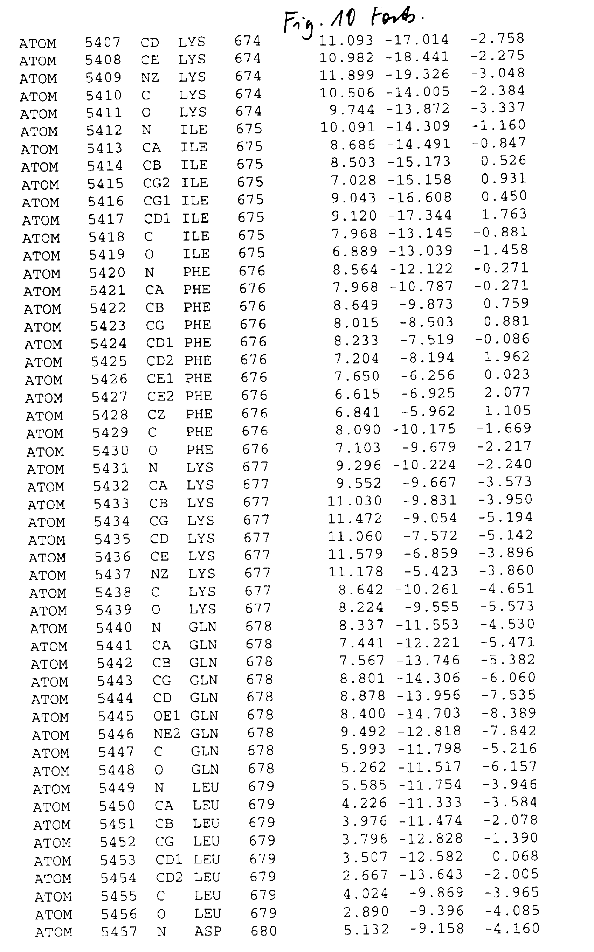

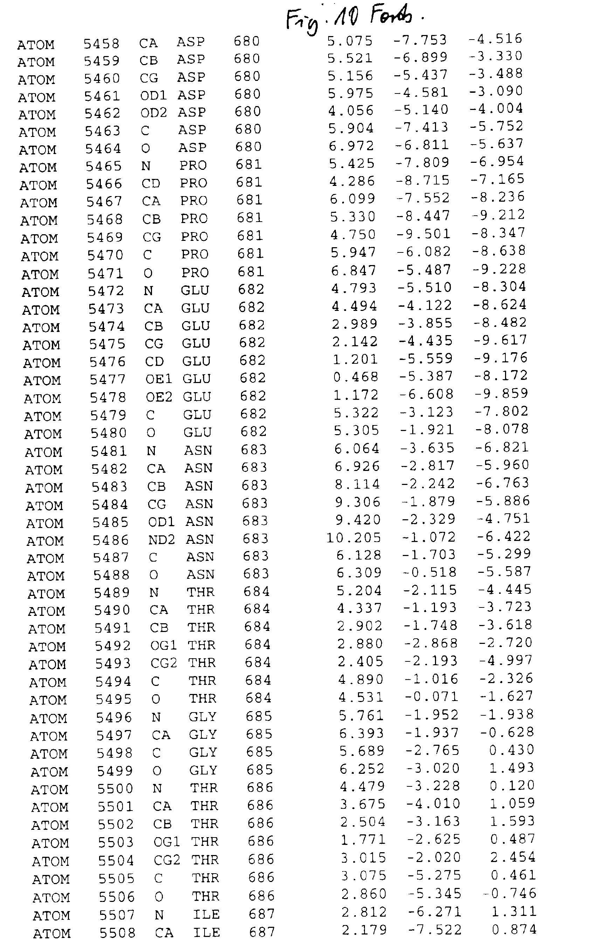

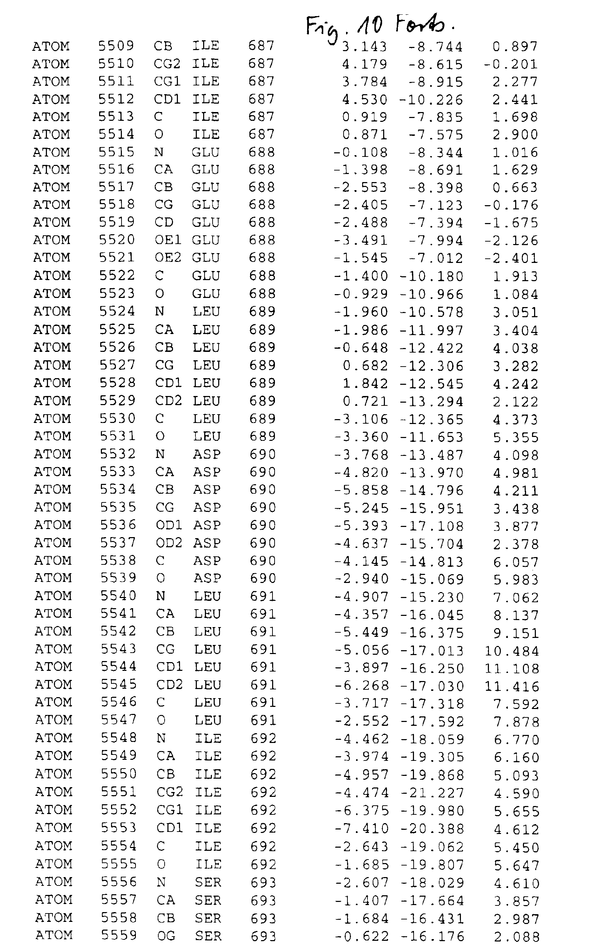

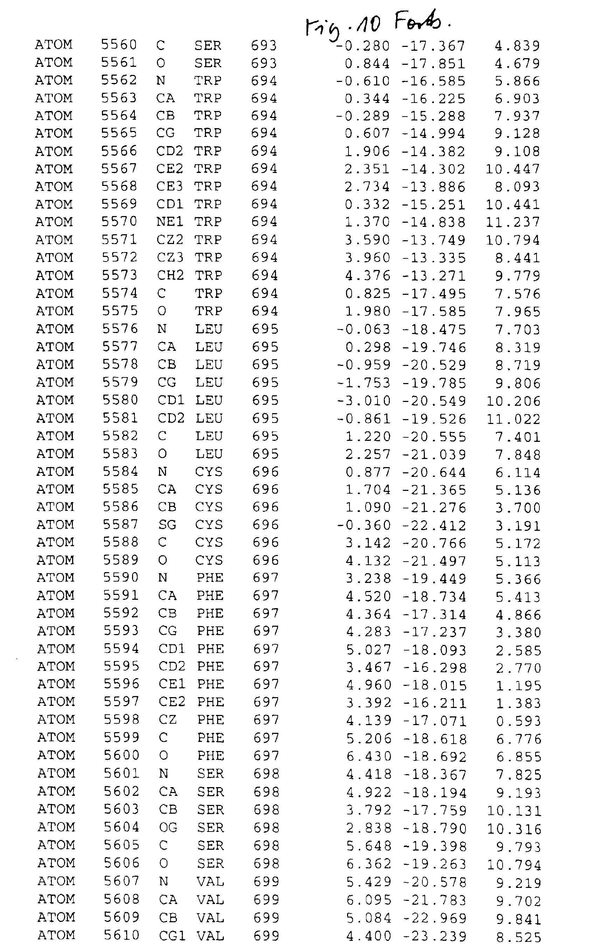

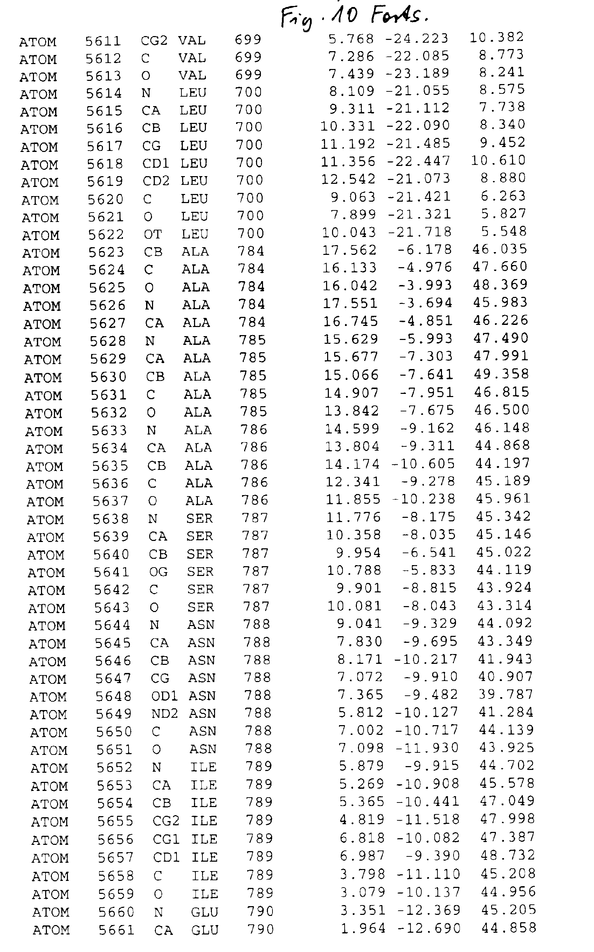

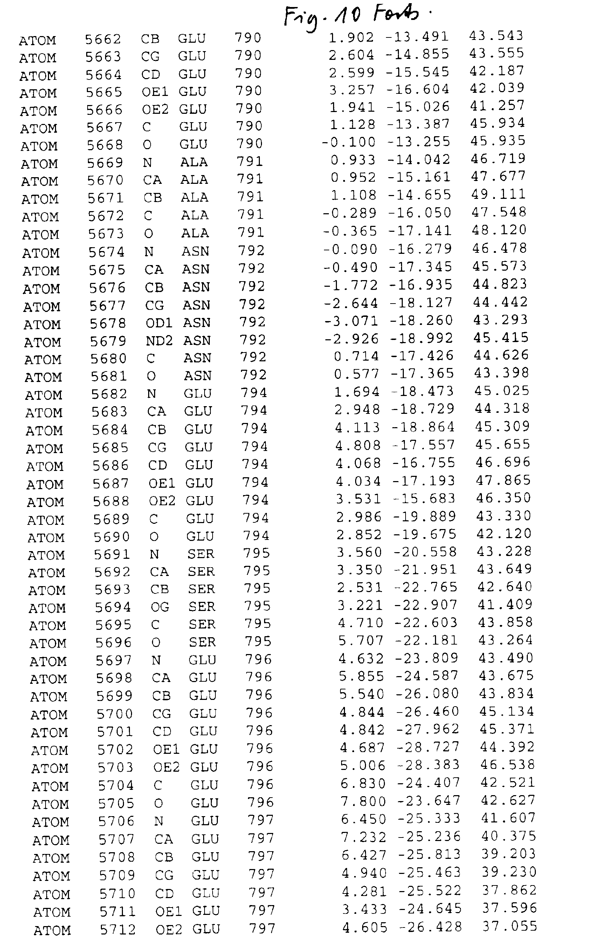

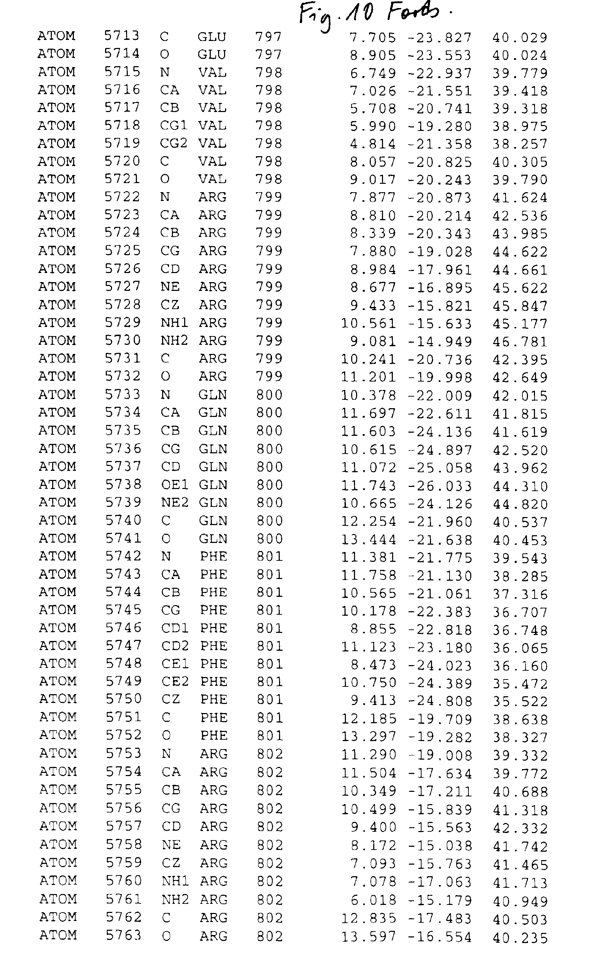

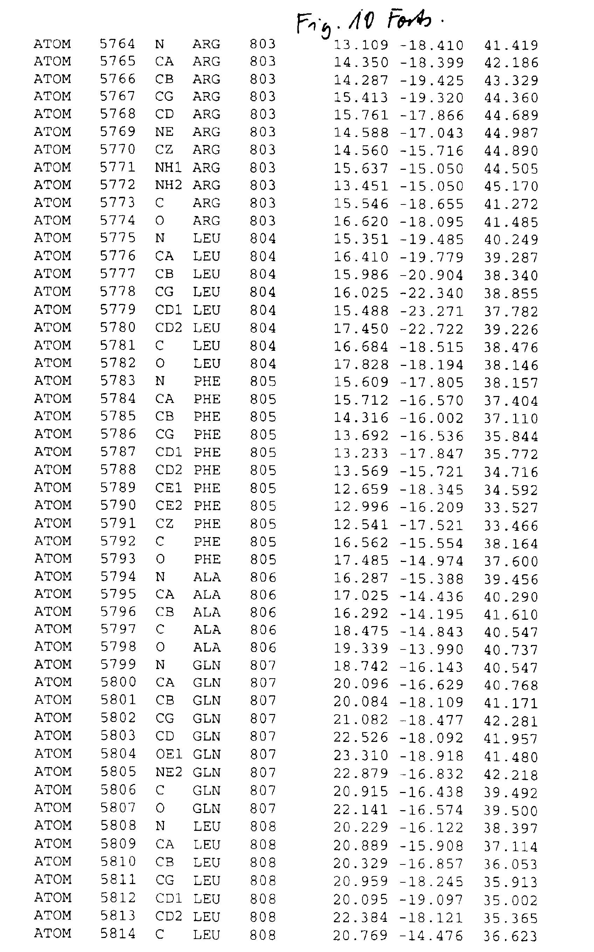

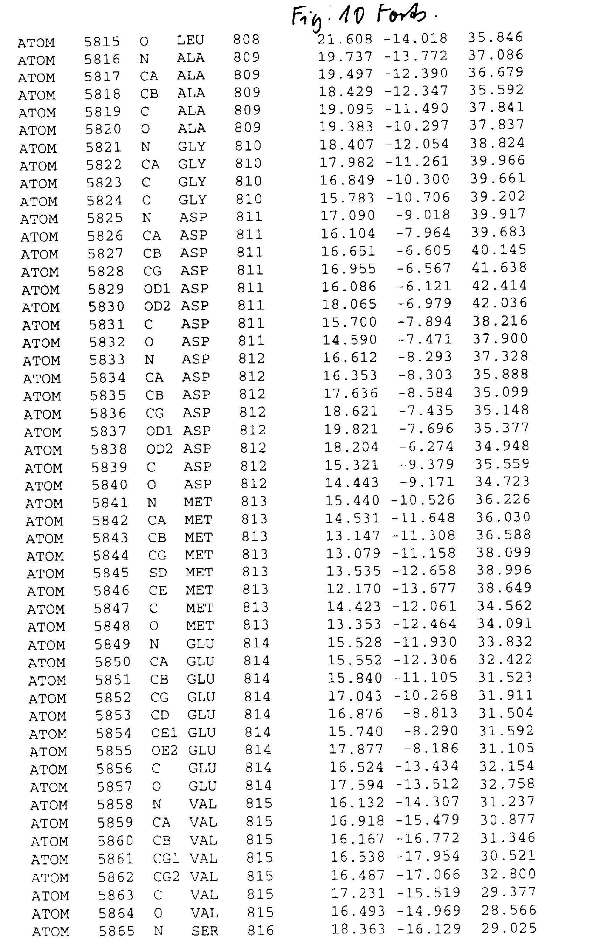

In einer weiteren bevorzugten Ausführungsform hat die an der Calpain-Reaktion beteiligte Sub-Domäne, die in der Polypeptidsequenz, deren Raumstruktur in der Kristallform vorliegt, enthalten ist, ein dreidimensionales Erscheinungsbild, wie es durch die Strukturkoordinaten gemäß Figur 10 wiedergegeben ist. Figur 10 gibt die Strukturkoordinaten für die Kristallform der Sub-Domäne IIa und IIb von humanem m-Calpain wieder. Noch weiter bevorzugt ist eine Kristallform eines Heterodimers aus großer und kleiner Calpain-Untereinheit, wobei die Polypeptide (1) und (2), die der kleinen bzw. großen Untereinheit entsprechen, durch eine Kristallform nach Maßgabe der Strukturkoordinaten der Figur 10 wiedergegeben werden können.

Kristallformen der erfindungsgemäßen Art sind insbesondere dann bevorzugt, wenn sie eine Auflösung von kleiner als 3,5 Å, vorzugsweise kleiner als 3,0 Å und ganz besonders bevorzugt kleiner als 2,5 Å besitzen.

Ein weiterer Gegenstand der vorliegenden Erfindung sind Verbindungen, die als Liganden an eine erfindungsgemäße Raum- oder bevorzugte Kristallform binden können bzw. er-findungsgemäße Kristalle, die derartige mikroskopische Raum- oder Kristallformen aufweisen. Diese Liganden werden typischerweise eine Eigenschaft als Pseudo-Substrat, Substrat, Aktivator oder Inhibitor besitzen und zeichnen sich dadurch aus, daß sie über sterische Eigenschaften und/oder funktionelle Gruppen verfügen, die in Wechselwirkung mit den Haupt- und/oder Seitenketten der katalytischen Sub-Domäne(n) oder eines für die Regulation der katalytischen Subdomäne(n) relevanten Sequenzabschnitte treten können. Durch diese Wechselwirkungen von mindestens einem Liganden mit Abschnitten der kleinen und/oder großen Untereinheit von Calpain werden gegebenenfalls Konformationswechsel hervorgerufen, die auf die proteolytische Aktivität von Calpain Einfluß nehmen kann (können), insbesondere deren katalytische Aktivität inhibieren kann (können). Der Ligand muß demnach über sterische Eigenschaften oder ein Wechselwirkungspotential verfügen, das komplementär ist zu den Haupt- und/oder Seitenketten des Calpains in seiner Raum- oder Kristallform oder dessen sterischen Gegebenheiten (z.B. für Furchen oder nicht kompakt ausgefüllte Bereiche im Proteininne-ren), wie durch die Raumform bzw. bevorzugt die Kristallform, erfindungsgemäß vorgegeben.
Besonders bevorzugt sind hierbei aber solche Liganden, die an die Domäne III von Calpain binden und vor allem mit der extrem sauren Schleife ("Loop") β2III/β3III und/oder strukturell der sauren Schleife benachbarten Aminosäuren in Wechselwirkung treten. Hierbei handelt es sich insbesondere um (a) die Region mit den Aminosäuren 392 bis 403, die insgesamt zehn negativ geladene Aminosäuren, also Aspartate oder Glutamate, umfaßt; (b) dieser Region gegenüber die Aminosäuren der amphipatischen Helix a7II; um (c) den Sequenzabschnitt, der durch die Aminosäuren K354 bis K357 gebildet wird; und/oder (d) um die die Aminosäuren K505 und K506.

Dabei wäre ein an diesen "Loop" bindender Ligand typischerweise so ausgestaltet, daß er mindestens eine positive Ladung und/oder mindestens eine positive Teilladung aufweist und mit dieser Ladungsstruktur an den negativ geladenen sauren "Loop" andockt. Auf diese Weise verhindert ein derartig strukturierter Ligand die Interaktion des sauren Loops β2III/ß3III mit der amphipatischen Helix α7II, die die basischen Aminosäuren K226, K230 und K234 der großen Untereinheit umfaßt. Ein zumindest teilweise positiv geladener Ligand könnte den an der vorgenannten Schleife befindlichen negativen Ladungsüberschuß kompensieren und im Ergebnis als Aktivator der katalytischen Aktivität von Calpain wirken. Auf diesem Wege wird die positiv geladene Helix der katalytischen Sub-Domäne IIb nicht von dem negativ geladenen sauren "Loop" der Domäne III durch die Ausbildung von Salzbrücken aus ihrer kompakten Faltung gelöst, weswegen die katalytische Aktivität aufrechterhalten bleibt.

Eine erfindungsgemäße Verbindung, die in diesem Strukturbereich von Calpain als Ligand bindet, ist mit ihren chemischen, geometrischen und/oder physikalischen entsprechend den strukturellen Vorgaben einer erfindungsgemäßen Kristallform auszugestalten. Sie kann im übrigen kovalent oder nicht-kovalent in einer erfindungsgemäßen Kristallform mit dem mindestens einen Polypeptid vorliegen.

Insbesondere sind eine oder mehr Randbedingungen, die nachfolgend für gewisse funktionelle Gruppen von Ami-nosäureseiten- oder -hauptketten, auch unter Berücksichtigung spezifischer Abstände zu oder zwischen diesen funktionellen Gruppen, für die Konzeption von erfindungs-gemäßen Verbindungen genannt sind, zu beachten. Zwischen der funktionellen Gruppe von K226, nämlich dem Atom NZ, und der funktionellen Gruppe von D400, also den beiden Sauerstoffatomen liegt in einer erfindungsgemäßen Kristallform eine Distanz von 3,32 bzw. 3,8 Å. Weiterhin besteht zwischen dem Lysin K230 der amphipatischen Helix und **D397** eine Interaktion, nämlich zwischen dem Atom NZ von K230 und den beiden Sauerstoffatomen des Aspartats D397 in einem Abstand von 3,73 bzw. 3,66 Å. Darüber hinaus interagiert die Seitenkette von K234 (NZ) mit einem Sauerstoffatom von E504 (OE1). Ein weiterer Kontakt besteht zwischen K354 (NZ) mit den Atomen OE1 bzw. OE2 von E504 (2,7 Å bzw. 3,36 Å). Auch die Aminosäure K505 (NZ) steht mit dem Atom OE1 von E396 über eine Salzbrücke (4,65 Å) in Verbindung. Darüber hinaus ergeben sich Interaktion zwischen K506 (NZ) mit E393 (OE1) (Distanz von 2,86 Å) und E393 (OE2) (Distanz gleich 4,93 Å). Weiterhin sind Wechselwirkungen zwischen der Aminosäure K357 (NZ) (aus dem Strukturbereich (c)) mit dem Amidsauerstoff aus der Hauptkette von E504 zu beobachten (3,68 Å).

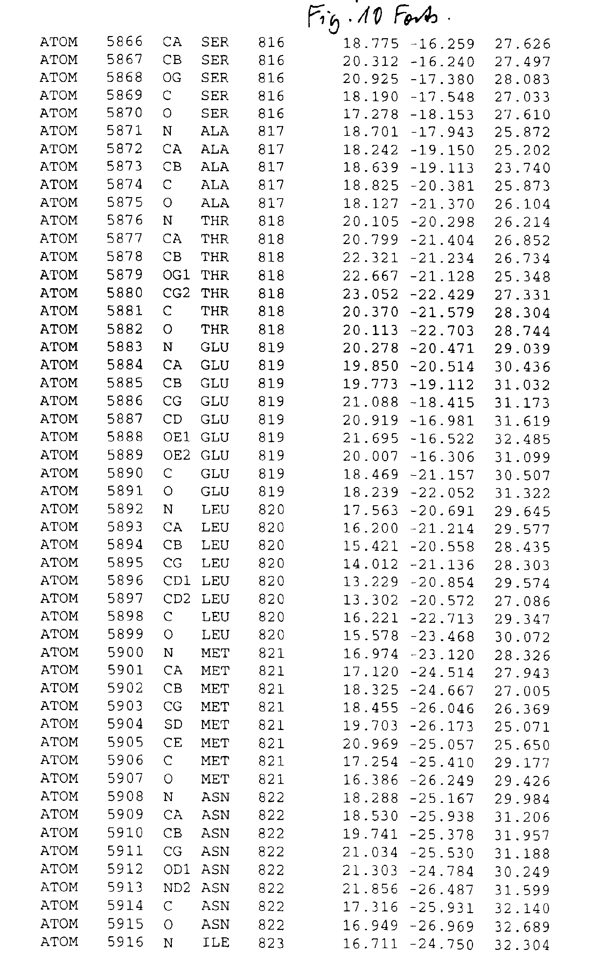

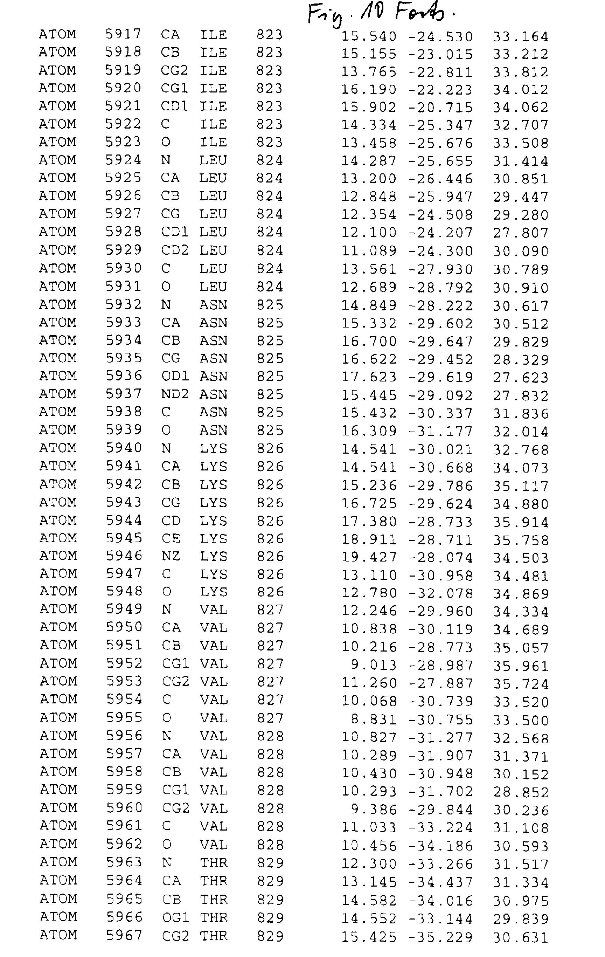

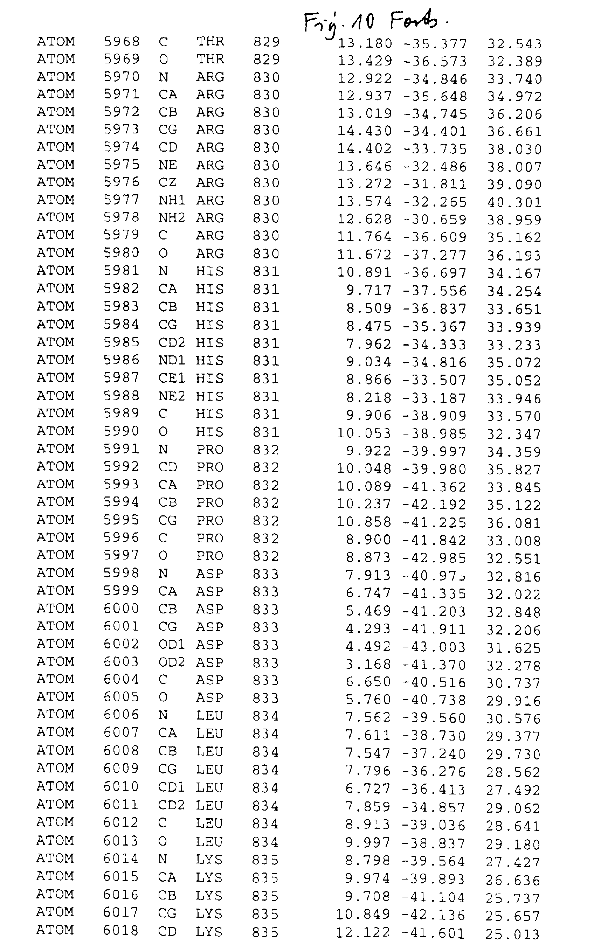

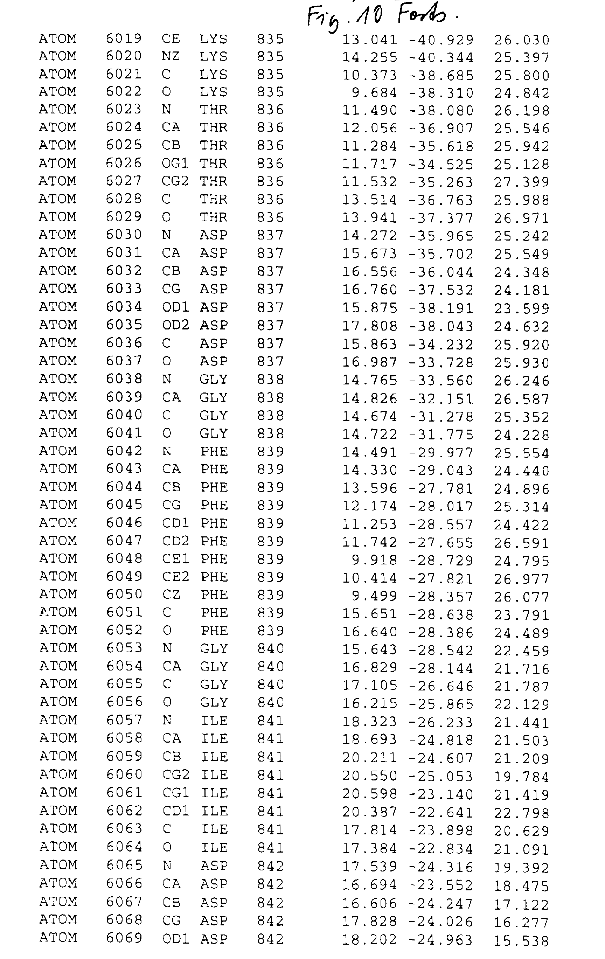

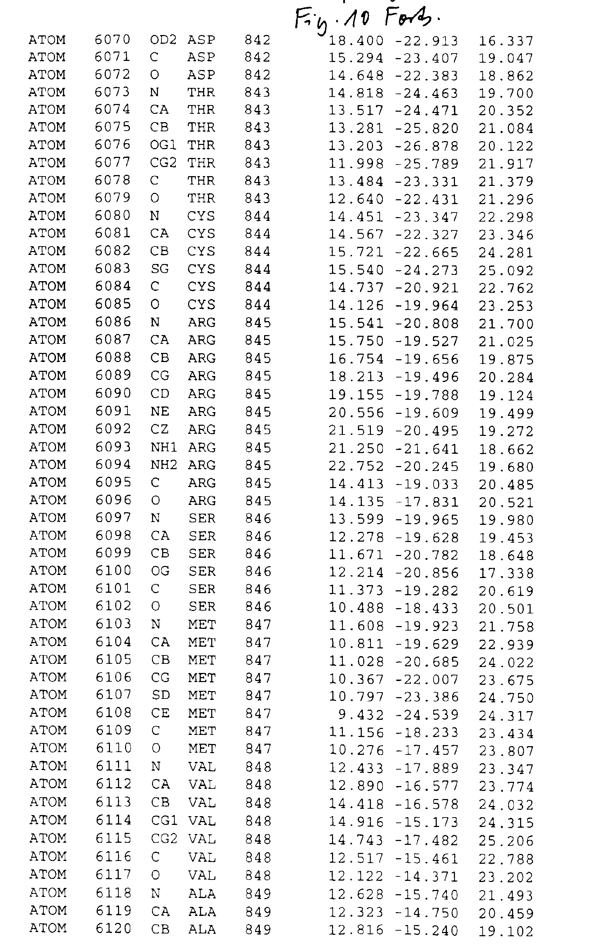

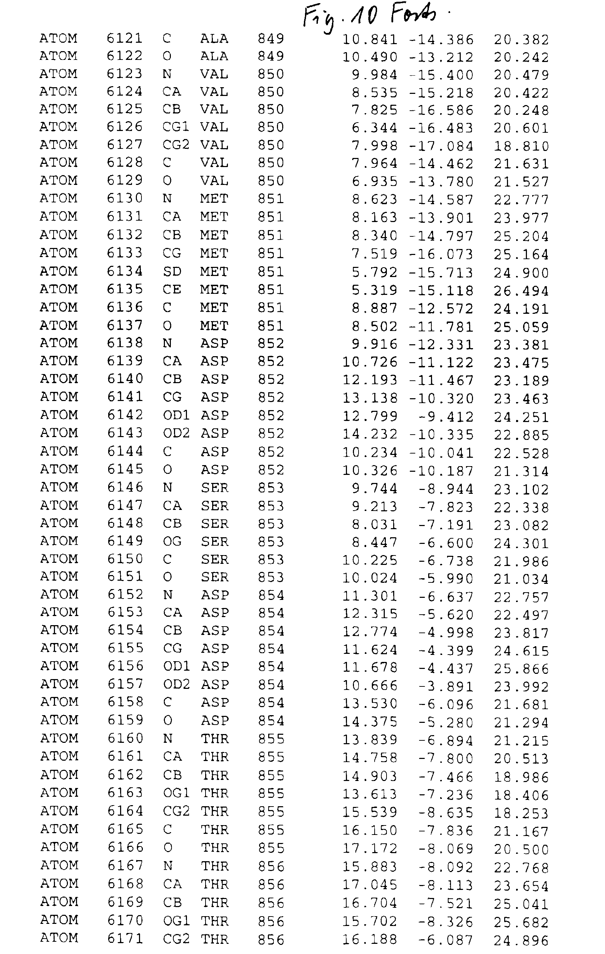

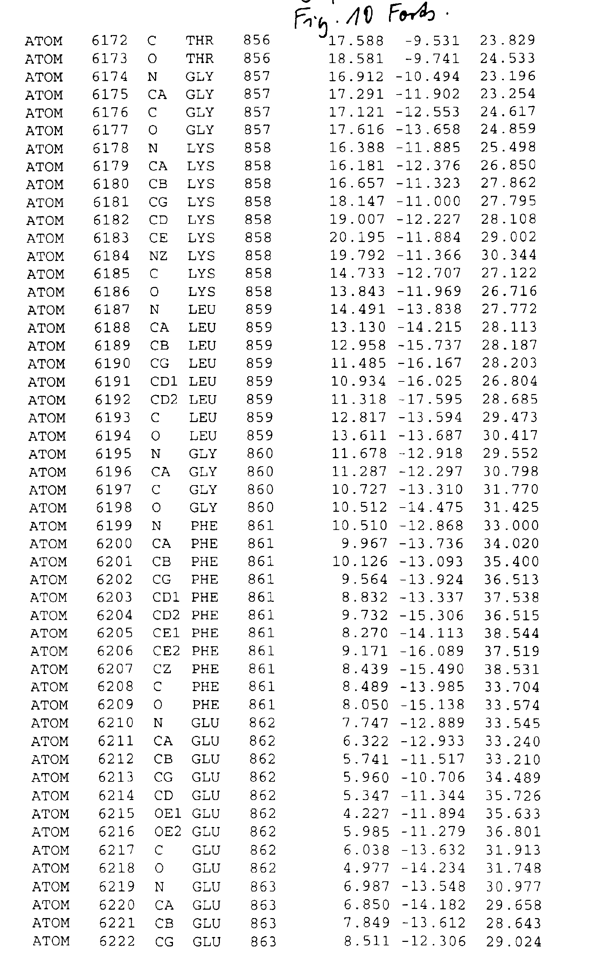

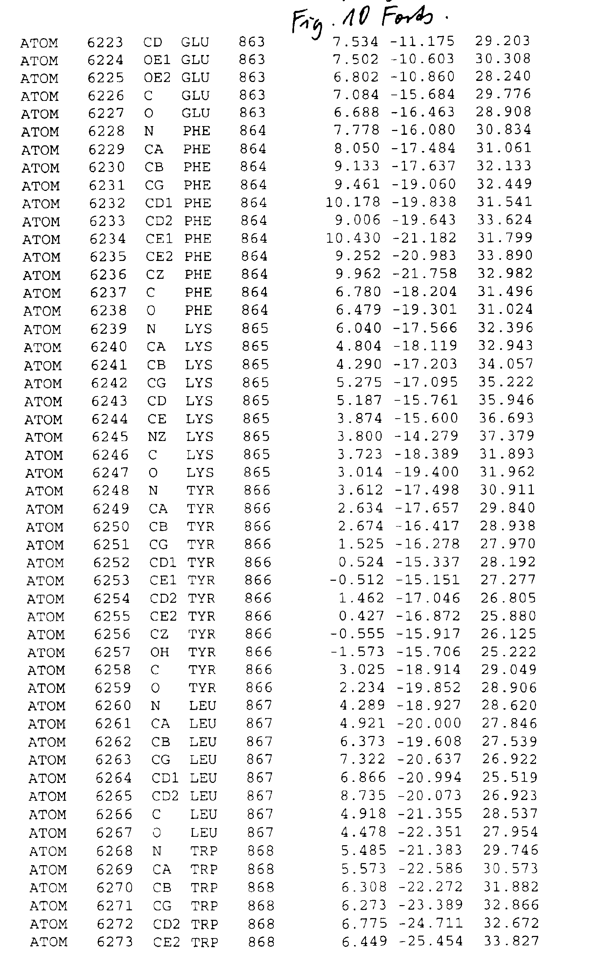

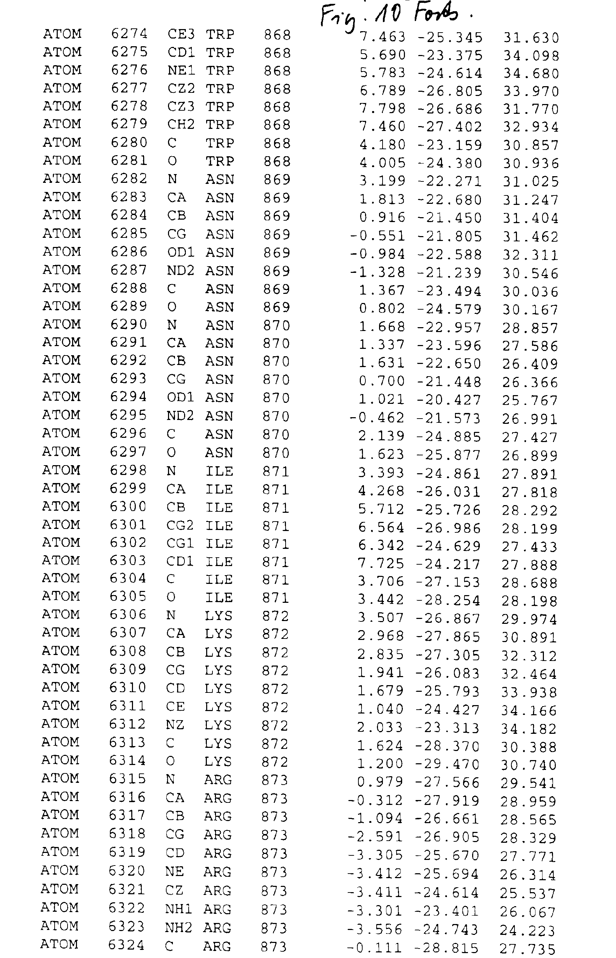

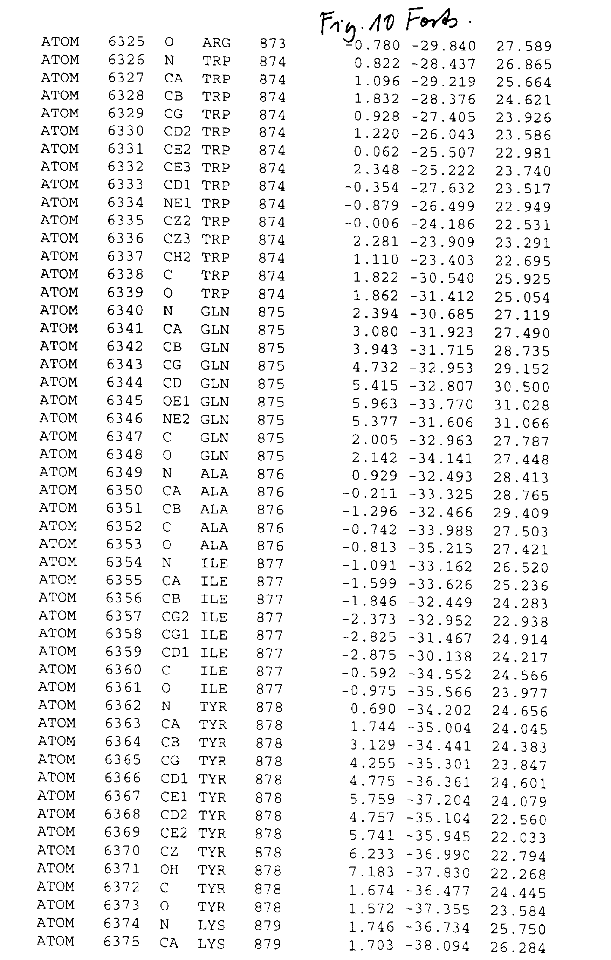

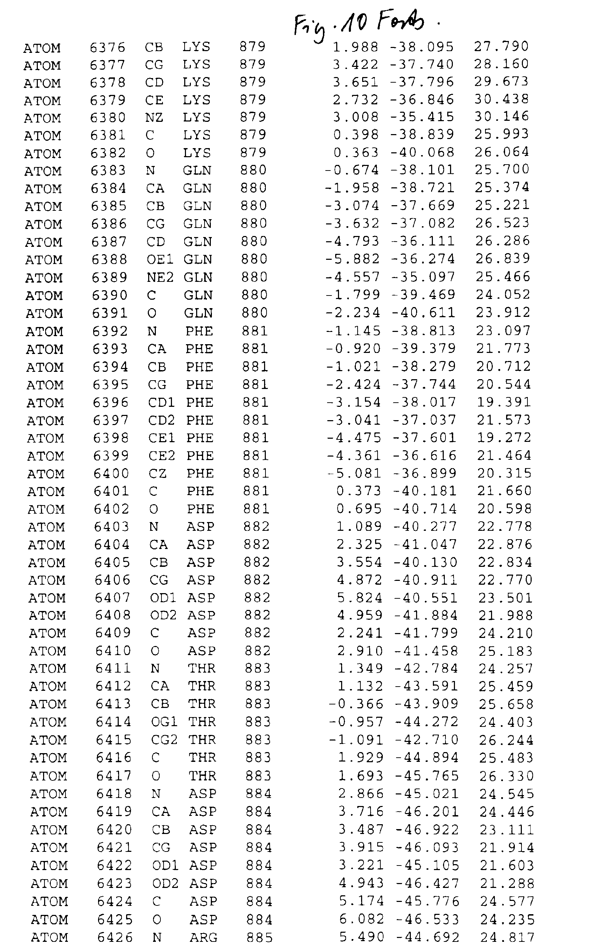

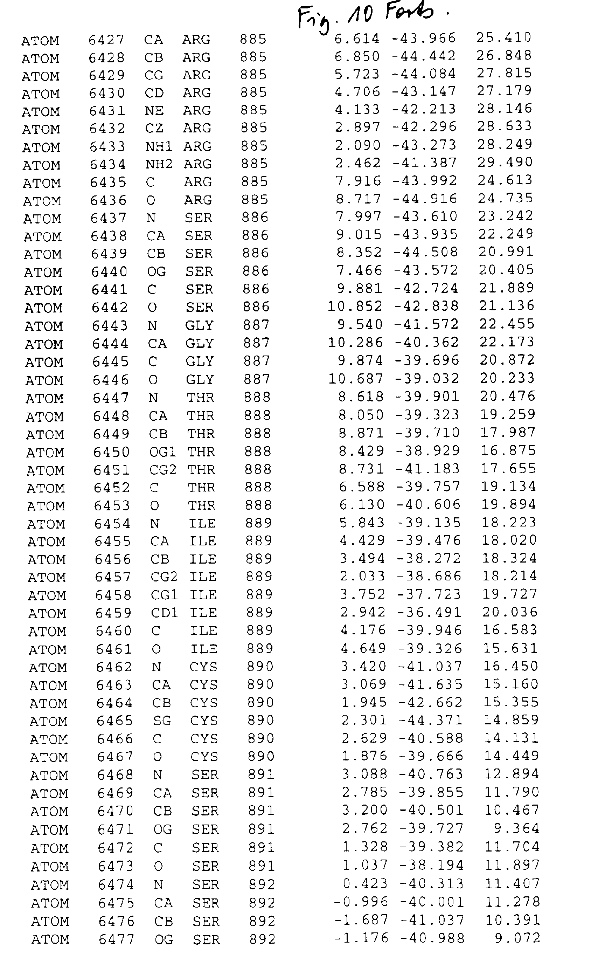

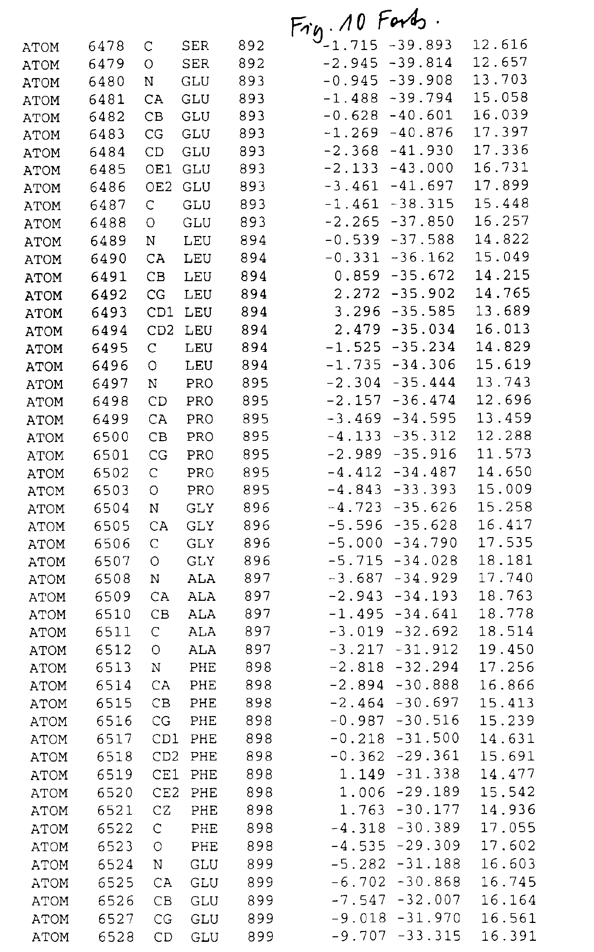

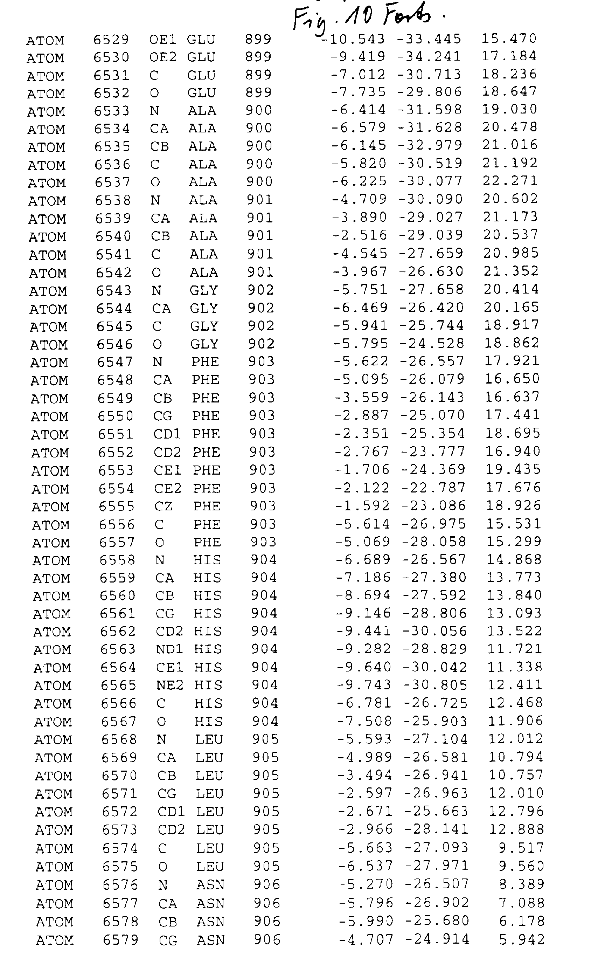

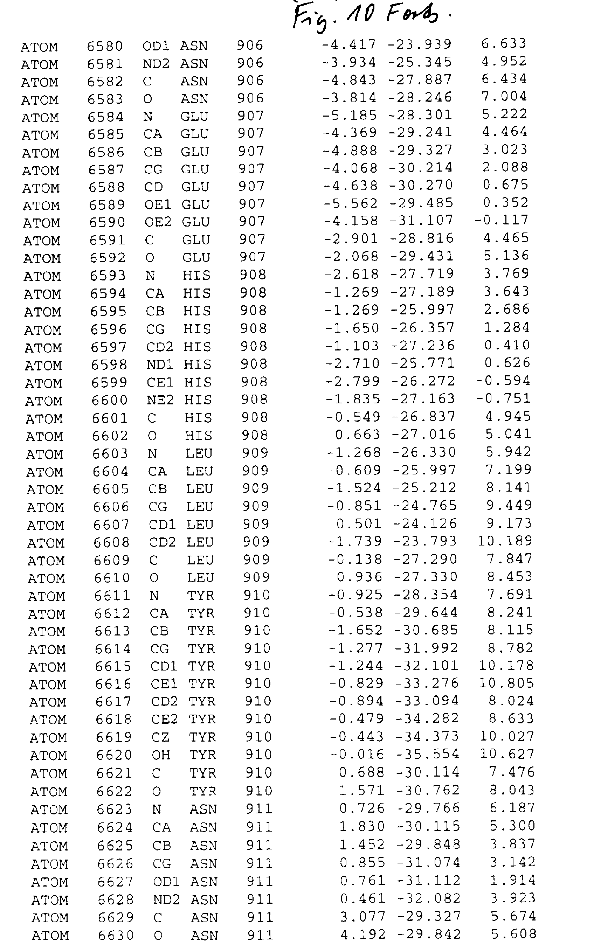

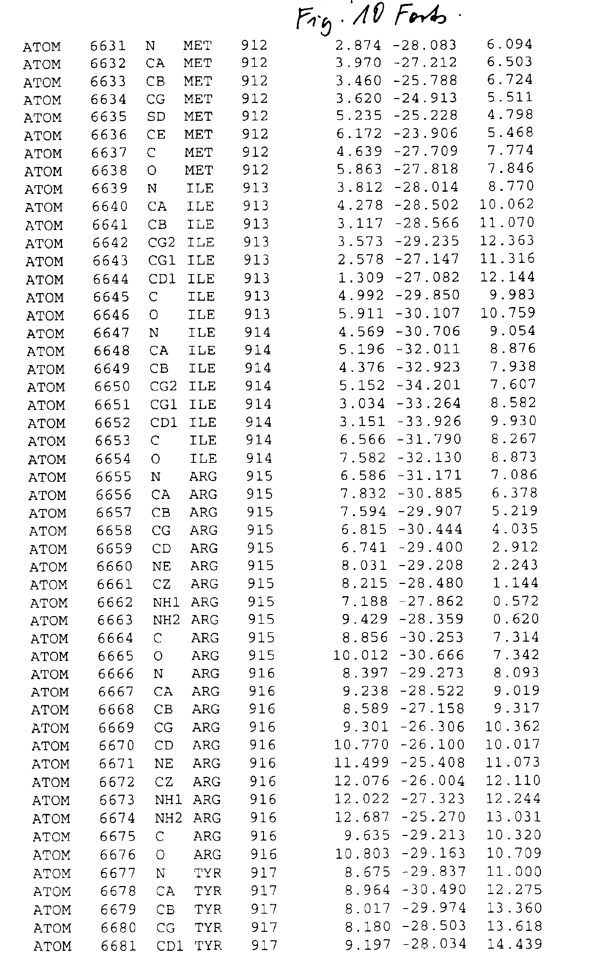

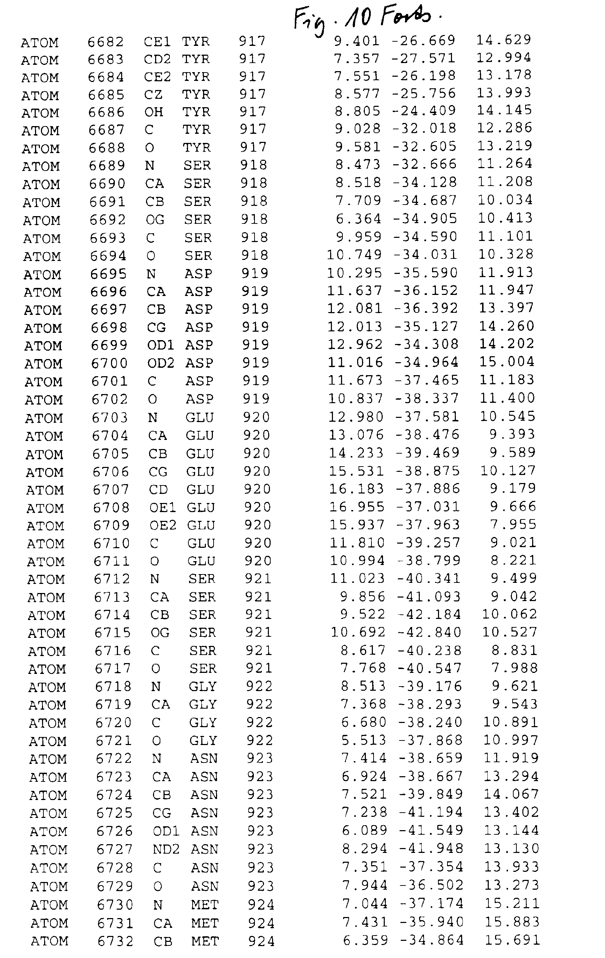

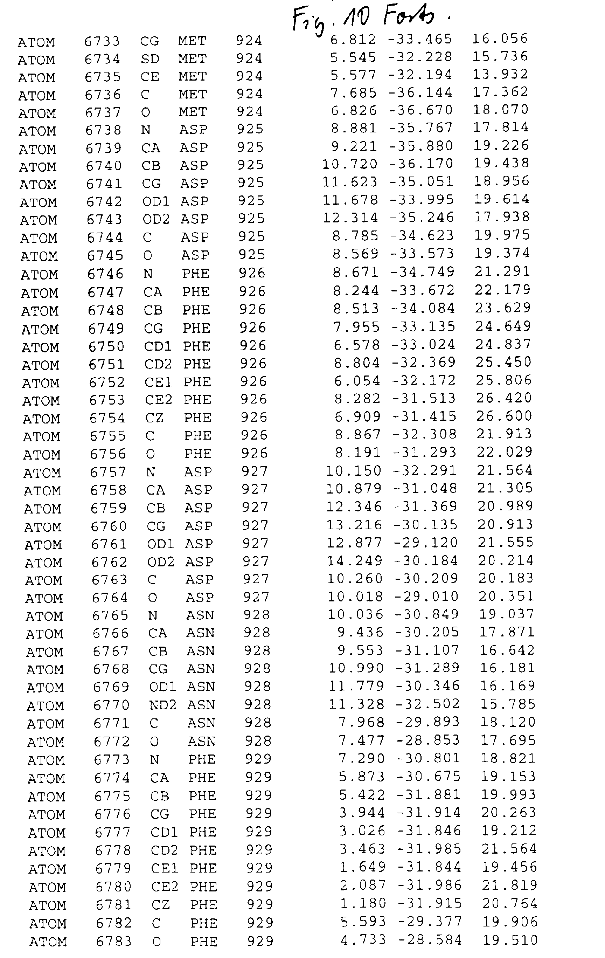

Eine erfindungsgemäße Verbindung, die an die Domäne III von Calpain gemäß erfindungsgemäßer Kristallform bzw. an einen erfindungsgemäßen Kristall, der derartige erfin-dungsgemäße Kristallformen aufweist, bindet, hat vorzugsweise den Charakter eines Ca²⁺-Analogons. Im Ergebnis führt die Wirkung des positiv geladenen Calpain-Aktivators zu einer dauerhaften konformationellen Nähe der katalytischen Subdomänen IIa und IIb, ohne Ausbildung einer Furche, wie sie in Figur 11 erkennbar ist, die Voraussetzung für die katalytische Aktivität von Calpain.

Verbindungen, die an das aktive Zentrum von Calpain binden, wie es nach Assoziierung der beiden Sub-Domänen IIa und IIb vorliegt, und dort ggf. als Inhibitoren der katalytischen Reaktion wirken, treten typischerweise mit mindestens einer der folgenden Aminosäuren Gln99, Cys105, Ser241, Asp243, Asn286, Gly261, His262 und/oder Trp288 in Wechselwirkung.

Um eine erfindungsgemäße inhibitorische Verbindung, die die inaktive Konformation der beiden Subdomänen IIa und IIb konserviert und damit die zwischen diesen beiden Sub-Domänen vorhandene Furche ganz oder teilweise ausfüllt, zur Verfügung zu stellen, wird deren chemisch-physikalischer und/oder geometrischer Charakter vorzugsweise eine oder mehr der folgenden durch die dreidimesio-nale Struktur eines Calpains vorgegebene Randbedingungen erfüllen. Besonders bevorzugt sind dabei Kontakte mit einer oder mehr der folgenden Aminosäuren (Seiten- oder Hauptkette): Gln99, Cys105, Ser241, Asp243, Gly261, His262, Trp288, Arg94, Asp96, Cys98, Trp106, Ala109, Thr200, Ile244, Lys260, Asn286, Glu290, Leu108, Thr201, Phe204, Trp214, Asp249, Lys257, Ala263, Tyr264, Glu292, Ser336 und/oder L338 (nach der Zählweise für humanes m-Calpain) bzw. entsprechende Aminosäuren für andere Cal-pain-Formen.

Ganz besonders bevorzugt sind inhibitorische Verbindungen der inaktiven Calpain-Form mit Furche dann, wenn sie bspw. an Wechselwirkungen mit Q99 mit ihren beiden funktionellen Gruppen am Atom NE2 bzw am Atom OE1. Beide Atome können in Wasserstoffbrücken mit dem Inhibitor invol-viert sein, die dann typischerweise die Länge der Wasserstoffbrückenbindung größer als 2,0 Å, vorzugsweise größer als 2,2 Å ist, wobei die geometrischen Gegebenheiten bei Wasserstoffbrückenbindungen (z.B. Direktionalität) zu berücksichtigen sind. Weiterhin kann ein derartiger Inhibitor vorzugsweise bspw. mit der Aminosäure C105 in Wechselwirkung treten. Hierbei sind insbesondere die Carbonylfunktion im Rückgrat und der Wasserstoff bzw. die freien Elektronenpaare am Schwefel SG von Cys105 zu nennen. Die korrespondierenden funktionellen Gruppen des Inhibitors sind von den vorgenannten Bereichen des Cys105 in einem Abstand von größer als 2 Å und typischerweise kleiner als 3,5 Å angeordnet.

Darüber hinaus kann der in der Furche bindende, vorzugsweise inhibitorische Ligand mit der Seitenkette der Aminosäure S241, vorzugsweise mit der Hydroxylgruppe an diesem Serinrest in Wechselwirkung stehen. Auch diese Hydroxylgruppe kann bspw. mindestens eine Wasserstoffbrückenbindung zum Inhibitor in einer für Wasserstoffbrückenbindungen typischen, direktionalen Weise mit einem Abstand zwischen 2,0 und 3 Å ausbilden. Weiter bevorzugt sind bspw. auch Interaktionen des Inhibitors über Salzbrücken mit der Carboxylgruppe an der Seitenkette der Aminosäure D243. Typischerweise wird der Inhibitor in einem Abstand von größer 2 Å mindestens eine positive Ladung und/oder Teilladung zu dieser Carboxylgruppe aufweisen.

Auch zur Carbonylgruppe von Aminosäure G261 kann bspw. bevorzugt ein Kontakt durch eine korrespondierende chemische Gruppe des Inhibitors bestehen, der typischerweise als Wasserstoffbrückenbindung mit einem Abstand von mindestens 2,0 Å ausgestaltet sein sollte. Typischerweise wird an einer Interaktion mit einem inhibitorischen Liganden auch die Seitekette der Aminosäure W288 beteiligt sein. Hierbei kann sowohl der am Indol-Stickstoff sitzende Wasserstoff eine Wasserstoffbrückenbindung im Abstand von typischerweise mehr als 2,0 Å mit dem Inhibitor eingehen als auch der Inhibitor mit einer diesbezüglich typischerweise ringförmigen, vorzugsweise aromatischen, Struktur oder einem derartigen Strukturelement mit der aromatischen Indolgruppe bspw. über ein sog. "Stacking" in Wechselwirkung treten. Hierbei handelt es sich um eine stapelweise parallele Anordnung von bspw. aromatischen Ringsystemen.

Vorzugsweise wird der Inhibitor bspw. Wasserstoffbrücken zu dem Wasserstoff an einem der Stickstoffatome des Hi-stidinrings von H262 eingehen. Auch mit dem Histidinring ist eine hydrophobe Wechselwirkung über das bereits oben erwähnte "Stacking" mit ringförmigen Fragmenten des Inhibitors möglich. In diesem Fall stehen die ringförmigen Systeme typischerweise parallel zueinander.

Gegebenenfalls kann auch die Aminosäure R94 mit der positiven Ladung an den Stickstoffen des Arginins an Interaktion mit dem Inhibitor beteiligt sein. Hierbei kann eine Wasserstoff- und/oder Salzbrücke mit der korrespondierenden funktionellen Gruppe des Inhibitors in einem typischen Abstand zwischen 2,0 und 3,8 Å bevorzugt sein. Ebenso kann die Carbonylfunktion von T95 mit einer direktionalen Wasserstoffbrücke mit dem Inhibitor interagieren oder auch die Carboxylfunktion der Seitenkette von D96 vorzugsweise mindestens eine Salzbrücke mit dem Inhibitor eingehen.

C98 ist bevorzugt ebenfalls an Interaktionen mit dem funktionellen Gruppen des Inhibitors beteiligt. Sowohl der Wasserstoff am Schwefelatom als auch die freien Elektronenpaare am Schwefel können für derartige Interaktionen in Betracht kommen. Weiter bevorzugt bildet bspw. W106 Wechselwirkungen mit dem Inhibitor aus. Mögliche Formen der Wechselwirkung entsprechen denen, die zuvor für W288 beschrieben worden sind, insbesondere besteht auch hier die Möglichkeit einer Wechselwirkung zwischen Aromaten, insbesondere im Wege des "Stackings".

Zwei weitere Wechselwirkungen, auf deren Basis der Inhibitor an die Furche zwischen den beiden Sub-Domänen binden kann, können bspw. auf Kontakten mit den Carbonylgruppen der beiden Aminosäuren L108 und A109, bspw. gleichfalls in Form von direktionalen Wasserstoffbrücken, beruhen.

Hydrophobe Wechselwirkung des Inhibitors mit Aminosäuren im Bereich der Furche einer erfindungsgemäßen Kristallform sind für die Seitenketten von A109 und A263 zu spezifizieren. Ferner wird in der Furche zwischen den beiden Subdomänen IIa und IIb ein hydrophobes Cluster durch die Seitenketten der Aminosäuren F204, L338 und den hydrophoben Teil der Seitenkette von T201 gebildet. Einen Inhibitor wird bevorzugt derart ausgestaltet sein, daß er über einen hydrophoben Abschnitt der mit den vorgenannten hydrophoben Gruppen in Wechselwirkung treten kann, verfügt.

Gegenüber der Helix α2II befindet sich in einer erfin-dungsgemäßen Kristallform die Seitenkette der Aminosäure I244, die ebenfalls vorzugsweise eine hydrophobe Wechselwirkung mit dem Inhibitor eingehen kann. Aus der Subdomäne IIb kann die Seitenkette der Aminosäure K260 in den zwischen den beiden Subdomänen gebildeten Spalt hineinragen, so daß vorteilhafterweise bspw. eine Salzbrücke des Inhibitors mit der positiven Ladung am NZ-Atom von K260 vorhanden sein wird. Hier würde der Inhibitor also an der zum NZ-Atom komplementären Position eine negative Ladung und/oder Teilladung vorzugsweise aufweisen.

Ein besonders bevorzugtes Angriffspunkt des Inhibitors für Interaktionen wäre eine Wechselwirkung mit der Seitenkette des an der katalytischen Reaktion beteiligten Aminosäurerests N286. Hierbei ergäbe sich bspw. für die Realisierung eines Inhibitors die Möglichkeit, direktio-nale Wasserstoffbrückenbindungen in Abständen zwischen 2,0 und 3,0 Å sowohl mit der Carbonyl- und/oder der NH₂-Gruppe von N286 einzugehen. In die Spalte des inaktiven Calpains zwischen den beiden Sub-Domänen ragt die Seitenkette der Aminosäure E290 hinein, mit negativen Ladungen, die bevorzugt durch entsprechende in räumlicher Nachbarschaft liegende, positive Ladungen eines Inhibitors er-findungsgemäßen kompensiert werden können.

Eine weitere bevorzugte Randbedingung für die Gestaltung eines erfindungsgemäßen Inhibitors kann vorteilhafterweise die hydrophobe Seitenkette der Aminosäure L108 darstellen. An einer komplementären Stelle kann dann typischerweise ein Inhibitor ebenfalls hydrophobe Fragmente aufweisen. Weiterhin kann der Inhibitor an Wechselwirkungen mit dem Tryptophanrest von W214, bspw. durch Wasserstoffbrücken am Indolstickstoff oder der bereits vorgenannten Aromatenwechselwirkung, beteiligt sein. Darüber hinaus kann ggf. auch ein Kontakt des Inhibitors mit der Carboxylgruppe von D249 vorliegen, vorzugsweise in Form einer Salzbrücke zwischen bspw. einer positiven Ladung des Inhibitors und der Carboxylgruppe.

Eine Salzbrücke kann vorteilhafterweise auch von der positiven Ladung am NZ-Atom von K257 zu einer korrespondierenden negativen Ladung und/oder Teilladung des Inhibitors vorliegen. Bei Inhibitoren, die vorzugsweise im äu-ßeren, nahe der Komplexoberfläche gelegenen Bereich der Furche andocken, wäre eine Wechselwirkung von funktionellen Gruppen des Inhibitors mit den negativen Ladungen von E292 wünschenswert. Auch hier würde der Inhibitor eine komplementäre positive Ladung und/oder Teilladung in Form einer Salzbrücke mit E292 ausbilden. Sofern ein Inhibitor mit der Eigenschaft, an der äußeren Oberfläche des Calpain-Komplexes im Bereich der Furche eingesetzt werden soll, kann ein inhibitorischer Ligand bspw. auch in Kontakt mit der Aminosäure Y264 stehen. Hierdurch kann vorteilhafterweise eine Blockade des an der katalytischen Reaktion beteiligten Aminosäure N286 und H262 gegeben sein. Der Tyrosinrest Y264 kann typischerweise Wechselwirkungen mit dem Inhibitor sowohl über mindestens eine Wasserstoffbrückenbindung, die an der charakteristischen Hydroxylgruppe ansetzt, als auch über seinen aromatischen Charakter bzw. seine Hydrophobizität mit entsprechenden Funktionen am Inhibitor eingehen. Für den Fall, daß der Inhibitor tief in die Furche hineinragt, wäre eine Wechselwirkung mit dem Aminosäurerest S236 der Subdomäne IIb, insbesondere eine Wasserstoffbrücke mit demselben, besonders wünschenswert.

Insbesondere umfaßt die vorliegende Erfindung solche Liganden, die an eine Raum- oder Kristallform, die durch die Strukturkoordinaten nach Figur 10 dargelegt ist, binden können.

Erfindungsgemäße Kristallformen zeichnen sich auch dadurch aus, daß sie als dreidimensionale Struktur, charakterisiert durch Strukturkoordinaten für jedes einzelne, die Struktur aufbauende Atom, Bestandteil einer symmetrischen Anordnung in einem Kristall sind. Dabei ist es bevorzugt, daß eine erfindungsgemäße Kristallform, die mindestens ein Polypeptid mit mindestens einer katalytischen Sub-Domäne enthält, nach Überlagerung mit den in Fig. 10 aufgelisteten Strukturkoordinaten für die mindestens eine an der katalytischen Reaktion beteiligte Sub-Domäne eine Standardabweichung (rms) von weniger als 2,5 Å, vorzugsweise von weniger als 2 Å, aufweist.

Ein weiterer Gegenstand der vorliegenden Erfindung sind Kristalle, die Kristallformen, wie durch die Ansprüche 1 bis 23 beansprucht, nach symmetrischen Gesetzmäßigkeiten angeordnet, aufweisen. Hierunter fallen Kristalle all jener Kristallformen, die gemäß vorliegender Erfindung offenbart werden. Hierbei kann es sich um native Kristalle, Derivatkristalle oder Cokristalle handeln. Erfindungsge-mäße native Kristalle weisen im wesentlichen eine symmetrische Anordnung von mindestens einem Polypeptid, das mindestens eine an der Katalyse der Calpain-Reaktion beteiligte Sub-Domäne enthält, ggf. in Kombination mit Calcium-Ionen als Bestandteil einer Kristallform, auf. Hierbei kann es sich bei der im kristallisierten Polypeptid enthaltenen, katalytischen Sub-Domäne sowohl um aktive als auch um inaktive Mutanten derselben handeln. Inaktive Mutanten sind dann ganz besonders bevorzugt, wenn sie die Struktur einer oder der beiden Sub-Domänen von Calpain im wentlichen beibehalten.

Ein weiterer Gegenstand der vorliegenden Erfindung sind Verfahren zur Identifizierung einer Verbindung, die die Eigenschaft besitzt, als Substrat, Pseudosubstrat, Aktivator, Inhibitor oder allosterischer Effektor von Calpain oder einer Mutante von Calpain, insbesondere humanem Calpain, ganz besonders humanem m-Calpain, zu wirken. Insbesondere bevorzugt ist ein solches Verfahren dann, wenn die Verbindung mit Ligandenfunktion an einen Strukturbereich einer der beiden katalytischen Sub-Domänen, insbesondere im Bereich des aktiven Reaktionszentrums, bindet. Ein solches Verfahren ist dadurch gekennzeichnet, daß (a) eine Kristallform nach den Ansprüchen 1 bis 23 erhalten wird, wobei die Kristallform in Form ihrer Strukturkoordinaten vorliegt, (b) die Strukturkoordinaten der Kristallform in drei Dimensionen dargestellt werden, (c) und die sterischen Eigenschaften und/oder funktionellen Gruppen einer Verbindung mit Ligandenfunktion so gewählt werden, daß Wechselwirkungen zwischen der Verbindung und den Haupt- und/oder Seitenketten des Polypeptids, das das aktive Zentrum bildet, möglich werden. Nach Maßgabe dieser Wechselwirkungen werden erfindungsgemäß geeignete Liganden, insbesondere geeignete inhibitorische Liganden, die die inaktive Calpain-Struktur mit einer Furche zwischen den Subdomänen konservieren, ermittelt.

Die Darstellung der Strukturkoordinaten einer erfindungs-gemäßen Kristallform erfolgt vorzugsweise durch graphische Darstellung mit Hilfe entsprechender Computerprogramme auf einem Computerbildschirm. Anhand der, bezogen auf potentielle Liganden, komplementären Anordnung der Haupt- und Seitenketten der Kristallform, beispielsweise im aktiven Zentrum eines Calpains, kann nicht-automatisiert nach Erfahrung des Operators geeignete Liganden mit entsprechenden chemischen und/oder sterischen Eigenschaften identifiziert, am Bildschirm konstruiert und schließlich deren Bindungsverhalten simuliert werden.

Vorzugsweise aber erfolgt die Auswahl geeigneter Liganden jedoch automatisiert dadurch, daß Computerdatenbanken, die eine Vielzahl von Verbindungen enthalten, durchsucht werden. Die Suche wird auf die zuvor erfolgende Charakterisierung von geometrischen, chemischen und/oder physikalischen Eigenschaften für die gewünschten Calpain-Liganden gestützt. Zu durchmusternde Datenbanken enthalten natürlich auftretende wie auch synthetische Verbindungen. Beispielsweise können die in der CCDC (Cambridge Crystal Data Center, 12 Union Road, Cambridge, GB) gespeicherten Verbindungen für eine derartige Suche herangezogen werden. Aber auch die bei Tripos (s. Zitat a.a.O.) erhältlichen Datenbanken, nämlich Aldrich, May-bridge, Derwent World Drug Index, NCI und/oder Chapman & Hall können durchsucht werden. Die folgenden Computerprogramme können für eine derartige Durchmusterung eingesetzt werden: insbesondere das Programm "Unity", "FLEX-X" (Rarey et al. J. Mol. Biol. 261, 470-489, 1996), "Cscore" (Jones et al., J. Mol. Biol. 245, 43, 1995) aus der Sybyl Base-Umgebung des Tripos-Programmpakets.

Im folgenden wird die Durchführung eines erfindungsgemä-ßen Verfahrens zur Computer-gestützten Identifizierung potentieller Liganden näher beschrieben. Zunächst muß der gewünschte Bindungsbereich eines Liganden in einer erfin-dungsgemäßen Kristallform definiert werden. Abhängig von der gewünschten Wirkung des Liganden kann es sich um einen Aktivator oder Inhibitor handeln, der an eine regulatorische Region bindet oder um einen Liganden für das aktive Zentrum, typischerweise dann mit inhibitorischen Eigenschaften. Der Bindungsbereich wird durch entsprechende Parameter, beispielsweise Atomabstände, Wasserstoffbrük-ken-Bindungspotentiale, hydrophobe Bereiche und/oder Ladungen, charakterisiert und auf dieser Basis Randbedingungen für die chemischen, physikalischen und/oder geometrischen Eigenschaften des Liganden definiert. Vorzugsweise handelt es sich bei dem Bindungsbereich um eine Region im Bereich des sauren "Loops" in der Domäne III eines Calpains oder um eine Bindung eines Liganden an oder in die Furche zwischen Sub-Domäne IIa und Sub-Domäne IIb. Ganz besonders bevorzugt sind an der Bindung mindestens eine der bereits zuvor spezifizierten Aminosäuren, insbesondere mit den vorgenannten Seitenketten derselben, beteiligt. Daher wird auch für ein vorliegendes erfindungs-gemäßes Verfahren zur Identifizierung von Verbindungen auf die vorangegangene Offenbarung zum Erfindungsgegenstand "Verbindung" gemäß Ansprüchen 24 bis 34 vollinhaltlich Bezug genommen. Computerprogramme identifizieren in entsprechenden Datenbanken dann solche Verbindungen, die die zuvor eingeführten Bedingungen erfüllen. Hierbei ist es besonders bevorzugt, das Programmpaket Sybil Base (Tripos, 1699 South Hanley Road, St. Louis, Missouri, USA) zu verwenden. Besonders bevorzugt ist es dabei, daß die zu durchsuchende Datenbank Verbindungen unter Angabe ihrer jeweiligen dreidimensionalen Strukturen zur Verfügung stellt. Sollte dies nicht gegeben sein, wird für ein erfindungsgemäßes Verfahren vorzugsweise in einem Verfahrensschritt (c1) ein Computerprogramm eingesetzt werden, das vor der Prüfung, ob die vorgebenen Randbedingungen von einem Liganden erfüllt sind, zunächst deren dreidimensionale Struktur berechnet (z.B. das Programm "CONCORD" aus der Sybyl-Umgebung von Tripos Inc.).

Typischerweise wird in einem Verfahrensschritt (d1) das Wechselwirkungspotential zwischen einer identifizierten Verbindung, beispielsweise im Rahmen einer automatisierten Suche einer Verbindung aus einer Computerdatenbank, und dem gewünschten Bindungsbereich in einer Kristallform ermittelt. Ganz besonders bevorzugt ist ein erfindungsge-mäßes Verfahren dann, wenn es zur Identifizierung von Verbindungen, die an eine Kristallform mit den Strukturkoordinaten der Fig. 10 andocken sollen, dient. Die Stärke der gemäß Verfahrensschritt (d1) ermittelten Wechselwirkung zwischen einer Verbindung aus einer Computerda-tenbank und einer erfindungsgemäßen Kristallform geben Anhaltspunkte für deren Eignung, als Liganden eingesetzt werden zu können.

Nicht automatisiert gestaltet sich ein Verfahren zur Identifizierung geeigneter Verbindungen mit Ligandencha-rakter wie folgt. Eine Gerüstverbindung als Ausgangapunkt der Identifizierung wird in den durch die zu identifizie-rende Verbindung auszufüllenden Raum im Innern oder an der Oberfläche der Kristallform, z. B. in das katalytische Zentrum einer erfindunsgemäßen Kristallform, manuell eingefügt. Für den nach Einfügung der Gerüststruktur noch verbliebenen Raum wird nach Fragmenten gesucht, die in Wechselwirkung mit der umgebenden Kristallform treten können und sich an die Gerüststruktur anlagern lassen. Diese Suche nach geeigneten Fragmenten erfolgt also nach Maßgabe der geometrischen und/oder physikochemischen Gegebenheiten der dreidimensionalen Struktur. Die Suche nach geeigneten Fragmenten kann bspw. als automatisierte Computersuche unter Vorgabe entsprechender Randbedingungen geführt werden. Etwaige durch den Operator und/oder durch die Computersuche ermittelten Fragmente werden nach Maßgabe chemischer Gesetzmäßigkeiten an die Ausgangsge-rüststruktur des Startmodells graphisch angelagert und nach jedem derartigen Schritt das Wechselwirkungspotential mit dem Zielstrukturbereich in der Kristallform errechnet. Die Vorgehensweise erfolgt so lange, bis das Wechselwirkungspotential zwischen der zu identifizieren-den Verbindung und dem Zielstrukturbereich optimiert ist.

Die Vorgehensweise der Schritte (c), (c1), (d) und (d1) kann zyklische so lange wiederholt werden, bis eine Verbindung oder eine Verbindungsklasse in bezug auf ihr Bindungsverhalten, berechnet nach einem Wechselwirkungspotential, das dem jeweiligen Computerprogramm als Algorithmus zugrundeliegt, optimiert ist. Die durch eine relativ grobe Charakterisierung des Bindungsbereichs der Kristallform zunächst erhaltene große Zahl an potentiell bindungsfähigen Verbindungen kann durch weitergehende Vorgaben physikalische-chemischer oder sterischer Charakteristika an die gewünschte Zielverbindung zunehmend reduziert werden.

Insbesondere bietet sich hierfür auch eine sinnfällige Kombination der nicht-automatisierten und der automatisierten Suchverfahren nach geeigneten Verbindungen an. So etwa könnte eine zunächst durch automatisierte Computersuche aus Computerdatenbanken identifizierte Verbindung nicht-automatisiert durch Anlagerung von Fragmenten mit geeigneten funktionellen Gruppen verbessert werden.

Schließlich ist es im Rahmen der vorliegenden Erfindung bevorzugt, die durch derartige erfindungsgemäße Verfahren per automatisierter Computersuche erhaltenen Verbindungen zu synthetisieren oder, falls bereits synthetisiert und zugänglich, einer chemischen Bibliothek zu entnehmen und in einem geeigneten biologischen Testsystem auf ihre biologische Wirksamkeit hin zu untersuchen. Abhängig vom Ergebnis des biologischen Testsystems, bei dem es sich z. B. um einen Ligandenbindungsassay oder um einen Enzymaktivitätstest handeln kann, können dann weitere chemische Modifikationen der zuvor ermittelten Verbindung oder der Verbindungsklase vorgenommen werden. Hierbei kann sich dann insbesondere die Anwendung von Programmpaketen zur Identifizierung geeigneter Fragmente, die gegen vorhandene Fragmente an der zuvor identifizierten Verbindung ausgetauscht oder an dieselbe zusätzlich angelagert werden könnten, als sinnvoll erweisen.

Ein weiterer Gegenstand der vorliegenden Erfindung sind gleichfalls Verfahren zur Identifizierung einer Verbindung mit der Eigenschaft, als Substrat, Pseudosubstrat, Aktivator oder Inhibitor, d. h. als Ligand von Calpain wirken zu können, wobei in einem solchen erfindungsgemä-ßen Verfahren in einem Verfahrensschritt (a) ein biologisches Testsystem vorangestellt wird, anhand dessen ein sog. "Screening" nach geeigneten Zielverbindungen durchgeführt wird. Auch hier können bevorzugt ein Bindungsas-say oder ein Enzymaktivitätstest als biologisches Testsystem dienen. In den weiteren Verfahrensschritten werden zunächst gemäß (b) solche Verbindungen (beispielsweise aus einer Bibliothek chemischer Verbindung) identifiziert, die ein positives Ergebnis im biologischen Test gezeigt haben. Diese Verbindungen, beispielsweise inhibierend oder aktivierend, werden in bezug auf ihre bspw. geometrischen und/oder chemischen Eigenschaften, insbesondere in bezug auf ihre dreidimensionale Struktur, charakterisiert (Verfahrensschritt (c)). Sofern die dreidimensionale Struktur der als Treffer im biologischen Test ermittelten Verbindungen nicht a priori bekannt ist, kann dieselbe durch Methoden der Strukturaufklärung, nämlich Röntgenkristallographie und/oder NMR-Spektroskopie, oder auch durch Modellierungen oder z.B. semi-quantenchemische Berechnungen ermittelt werden. In die gemäß Verfahrens-schritt (d) als dreidimensionale Struktur dargestellten atomaren Strukturkoordinaten einer erfindungsgemäßen Kristallform werden dann gemäß (e) die im Rahmen der Verfahrensschritte (b) und (c) erhaltenen Verbindungen eingefügt. Hierbei kann es sich um Verbindungen handeln, die an das aktive Zentrum oder eine für die Regulation des aktiven Zentrums relevanten Abschnitt in der Kristallform binden. Das Einfügen der Verbindung in die Kristallform kann manuell nach der Erfahrung des Operators erfolgen oder aber auch automatisiert, indem mit Hilfe entsprechender Computerprogramme ("Dock" Kuntz et al., 1982, J. Mol. Biol. 161, 269-288, Sybyl/Base "FLEX-X", s. Zitat a.a.O.) eine Positionierung des Liganden mit der stärkstmöglichen Wechselwirkung zwischen Ligand und dem Zielstrukturbereich ermittelt wird (Verfahrensschritt (el)).

Indem eine derart erhaltene Verbindung graphisch in Kombination mit der in der Kristallform vorliegenden Struktur dargestellt wird, können weitere Verfahrensschritte erfolgen, die die Wirksamkeit der Zielverbindung verbessern. Insbesondere kann eine derart, bereits als geeignet identifizierte Verbindung als Basis ("template") für noch wirksamere Verbindungen, z. B. Verbindungen mit noch höherer Bindungskonstante, dienen. In diesem Zusammenhang können die bereits gemäß Ansprüchen 35 bis 40 beschriebenen Verfahren und Ansätze zum Einsatz kommen. Bevorzugt ist eine Vorgehensweise, die insoweit zyklisch ist, als nach dem "Screening" im biologischen Testsystem eine strukturelle Darstellung erfolgt und mit Hilfe von Compu-termethoden auf der Basis der im biologischen Testsystem erhaltenen Ergebnisse wirksamere Verbindungen ermittelt werden, die schließlich wiederum als Ausgangspunkt für den nächsten Zyklus, an dessen Beginn ein biologisches Testsystem steht, dienen. Biologische Testsysteme (in vitro oder in vivo) können Aussagen über die Qualität der Verbindung, z.B. als Inhibitor der biologischen Reaktion, also bspw. als Inhibitor der Protease-Reaktion, oder über die Bindungskonstante, die Toxizität oder die Metaboli-sierungseigenschaften oder ggf. über das Membrandurch-trittsvermögen der Verbindung etc., gemacht werden.

Schließlich werden im Rahmen der vorliegenden Erfindung alle solchen Verbindungen beansprucht, die als Ergebnis eines Verfahrens nach einem der Ansprüche 35 bis 42 erhalten werden.

Ein weiterer Gegenstand der vorliegenden Erfindung sind Verfahren zur Herstellung einer Raum- oder Kristallform mit mindestens einem Polypeptid nach einem der Ansprüche 1 bis 23, wobei in einem Verfahrensschritt (a) zunächst das Polypeptid in einem Expressionssystem überexprimiert, synthetisiert oder isoliert wird, (b) das gemäß (a) erhaltene Polypeptid in einem geeigneten Puffersystem gelöst wird und **(c)** die Kristallisierung, bspw. durch Dampfdiffusionsverfahren, eingeleitet wird. Typischerweise wird gemäß Verfahrensschritt (b) eine konzentrierte oder hochkonzentrierte Lösung des (der) Polypeptids/e vorliegen. Erfolgt die Kristallisierung des mindestens einen Polypeptids zu erfindungsgemäßen Kristallen, die erfindungsgemäße Kristallformen aufweisen, mit dem Ziel, die Kristalle nachfolgend zur Röntgenstrukturanalyse einzusetzen, so folgt nach der Kristallisierung die Sammlung von Röntgendiffraktionsdaten, die Bestimmung der Einheitszellkonstanten und der Symmetrie sowie die Berechnung der Elektronendichtekarten, in die das (die) Polype-tid/e hineinmodelliert werden.

Ein weiterer Gegenstand der vorliegenden Erfindung sind Verfahren zur dreidimensionalen Darstellung einer Kristallform unbekannter Struktur mit mindestens einem Polypeptid, das mindestens eine an der Katalyse beteiligte Sub-Domäne eines Proteins aus der Familie der Calpaine enthält. Ein solches Verfahren ist dadurch gekennzeichnet, daß die Kristallform mit unbekannter Struktur auf der Basis einer erfindungsgemäßen Kristallform mit bekannter Struktur, beispielsweise auf Basis der in Fig. 10 festgehaltenen Strukturkoordinaten, ermittelt wird. Hierbei gibt es verschiedene Möglichkeiten, bekannte Strukturkoordinaten erfindungsgemäßer Kristallformen zur Strukturaufklärung von Polypeptiden oder Polypeptidkom-plexen mit bislang unbekannten 3D-Strukturen (Zielstruktur), die jedoch mit der bekannten erfindungs-gemäßen Kristallform gewisse Homlogien zeigen, einzusetzen.

Eine Möglichkeit ist in diesem Zusammenhang die Verwendung von Phaseninformation, die aus bekannten Aus-gangsstrukturkoordinaten, beispielsweise den Strukturkoordinaten gemäß Fig. 10, entnommen werden können. Hierzu wird die Phaseninformation, die im Falle einer bekannten 3D-Struktur einer erfindungsgemäßen Kristallform vorliegt bzw. berechnet werden kann, eingesetzt, um eine unbekannte Struktur, die sich vorzugsweise gegenüber der bekannten Struktur nur durch nicht wesentliche konformationelle Abweichungen unterscheidet (als Beispiele zu nennen wären Zielstrukturen, an die erstmals ein Ligand oder ein anderer Ligand als in der Ausgangsstruktur gebunden ist, oder Derivate, z.B. Zielstrukturen, die Mutanten der Ausgangsstruktur sind) zu lösen. Hierzu wird die Phaseninformation der gesamten oder eines Teils der bekannten Struktur mit den für die Kristallform unbekannter Struktur gesammelten Intensitäten der Reflexe kombiniert und aus dieser Kombination eine Elektronendichtekarte für die Kristallform unbekannter Struktur berechnet. Diese Methode wird als "molekulares Ersetzen" ("molecular replace-ment") bezeichnet. Vorzugsweise wird das molekulare Ersetzen mit dem Programmpaket X-PLORE (Brünger, Nature 355, 472-475, 1992) durchgeführt.

Eine weitere Möglichkeit, vorliegende erfindungsgemäße Kristallformen zur Strukturaufklärung von strukturell verwandten Sequenzen bzw. beim Vergleich der Primärstruk-turen zumindest tw. homologen Polypeptidketten einzusetzen, besteht erfindungsgemäß darin, daß (a) die Primärsequenz eines Polypeptids unbekannter 3D-Struktur mit einer Primärsequenz eines Polypeptids, das mindestens eine der beiden katalytischen Sub-Domänen eines Calpains aufweist (insbesondere aber ein Calpain), verglichen wird und im Rahmen dieses Vergleichs homologe Abschnitte zwischen dem Polypeptid unbekannter Struktur und der Primärsequenz eines Calpains, dessen Raum- oder vorzugsweise Kristallform bekannt ist, identifiziert werden, (b) die homologen Abschnitte in Anlehnung an die bekannte 3D-Struktur modelliert wird und schließlich gemäß Verfahrensschritt (c) mit Hilfe geeigneter Computerprogramme die modellierte 3D-Struktur des Polypeptids in Hinblick auf ihre sterischen Verhältnisse optimiert wird.

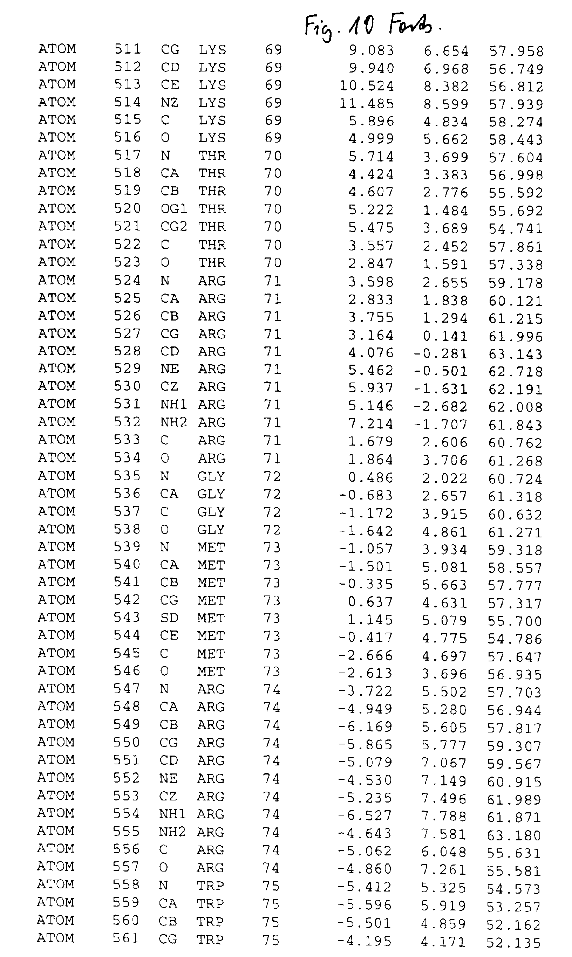

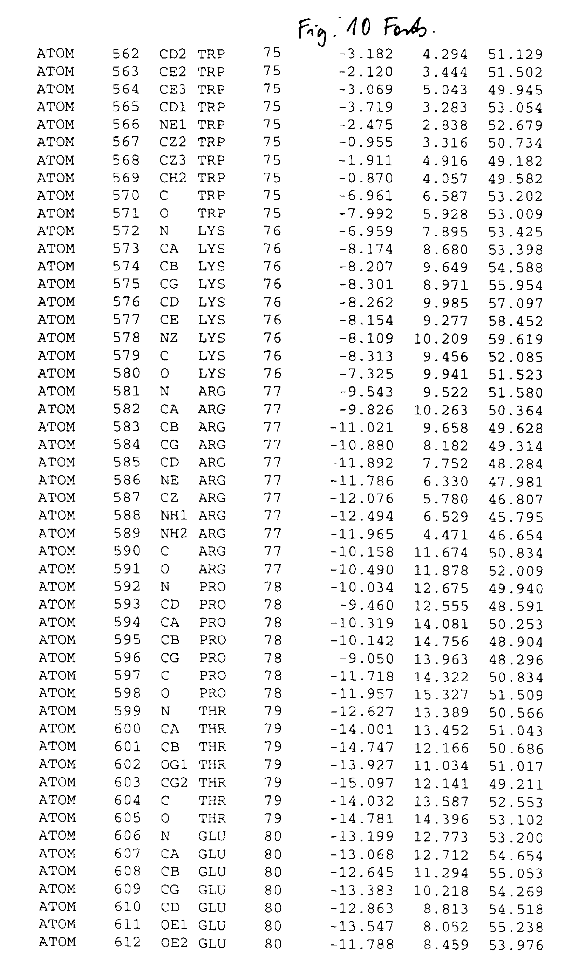

Das sog. "Alignment" der Primärsequenzen von zu vergleichenden Polypeptiden unbekannter bzw. bekannter 3D-Struktur gemäß (a) stellt eine zentrale Aufgabe für das Homologie-Modelling dar. Hierbei werden die ausgerichteten korrespondierenden Aminosäuren verschiedenen Kategorien zugeordnet, nämlich Positionen mit identischen, ähnlichen, entfernt ähnlichen oder unähnlichen Aminosäuren. In diesem Zusammenhang wird dabei auch die Figuren 7 und 8 bzw. auf die Beschreibung dieser Figuren verwiesen. Besondere Berücksichtigung müssen beim "Alignment" ggf. Insertionen oder Deletionen zwischen den zu vergleichenden Primärsequenzen finden. Die gemäß Verfahrensschritt (c) erfolgende Optimierung der auf der Grundlage der bekannten 3D-Struktur modellierten Zielstruktur kann durch die Methoden der Molekulardynamik-Simulation "molecular dyna-mics" oder durch Energieminimisierung (z.B. Sybil Base von Tripos, s. Zitat a.a.O.) erfolgen.

Besonders bevorzugt ist ein erfindungsgemäßes Verfahren zur Aufklärung von Kristallformen unbekannter Struktur dann, wenn es sich bei der Kristallform bekannter Struktur um ein m- oder µ-Calpain handelt und die Kristallform eines n-Calpains z.B. durch "Molekulares Ersetzen" oder durch "Homologie-Modelling" aufgeklärt werden soll. Aber auch die umgekehrte Vorgehensweise ist möglich oder aber auch die Bestimmung einer µ-Calpainstruktur auf der Basis einer bekannten m-Calpain-Kristallform oder umgekehrt. Gleichfalls kann auf der Basis einer erfindungsgemäßen bekannten Calpain-Kristallform eines Wirtsorganismus eine Kristallform für einen Calpain-Komplex eines anderen Wirtsorganismus ermittelt werden.

Damit also können Strukturkoordinaten von erfindungsgemä-ßen Krisnallformen durch Homologie-Modelling als Struk-turmodelle für sequentiell homologe Polypeptide unbekannter 3D-Strukturen dienen. Im Rahmen des Homologie-Modellings kommen Programmpakete zum Einsatz, insbesondere kann mit dem Insight II Modellierungspaket (Molecular Simulations Inc.) ein derartiges Modelling durchgeführt werden.

Schließlich wird im Rahmen der vorliegenden Erfindung auch die Verwendung von Inhibitoren und/oder Aktivatoren der katalytischen Aktivität von Calpain, insbesondere von humanem Calpain, ganz besonders von humanem m-Calpain, nach einem der Ansprüche 24 bis 34 oder erhalten aus einem Verfahren nach einem der Ansprüche 35 bis 42 zur Herstellung eines Arzneimittels, zur Verwendung als Arzneimittel oder als Wirkstoff der in einer pharmazeutischen Zusammensetzung enthalten ist, offenbart. In einer pharmazeutischen Zusammensetzung wird sich ein erfindungsge-mäßer Calpain-Aktivator oder -Inhibitor mit mindestesn einem weiteren Wirkstoff und/oder die pharmazeutische Zusammensetzung wird als Arzneimittel in eine dem Fachmann geläufigen Formulierung eingearbeitet sein. Die Formulierung wird dabei insbesondere vom Verabreichungsweg abhängen. Dieser kann oral, rektal, intranasal oder parenteral, insbesondere subkutan, intravenös, oder intramuskulär, sein. Pharmazeutische Zusammensetzung, die einen derartigen Inhibitor und/oder Aktivator enthalten, können die Verabreichungsform eines Puders, einer Suspension, einer Lösung, eines Sprays, einer Emulsion oder einer Creme haben.

Ein erfindungsgemäßer Inhibitor und/oder Aktivator kann mit einem pharmazeutisch akzeptablen Trägermaterial mit neutralem Charakter (wie z. B. wäßrigen oder nicht-wäßrigen Lösungsmitteln, Stabilisatoren, Emulgatoren, Detergentien und/oder Additiven und ggf. weiteren Farb-oder Geschmacksmitteln kombiniert sein. Die Konzentration eines erfindungsgemäßen Inhibitors und/oder Aktivators in einer pharmazeutischen Zusammensetzung kann zwischen 0,1% und 100% variieren, abhängig insbesondere von dem Verabreichungsweg. Ein pharmazeutische Zusammensetzung oder ein Arzneimittel, enthaltend einen erfindungsgemäßen Inhibitor und/oder Aktivator von, kann insbesondere zur Behandlung von ischämischen Zuständen, von Muskeldystrophie und/oder Tumorerkankungen dienen.

Die vorliegende Erfindung wird durch die nachfolgenden Figuren näher erläutert.

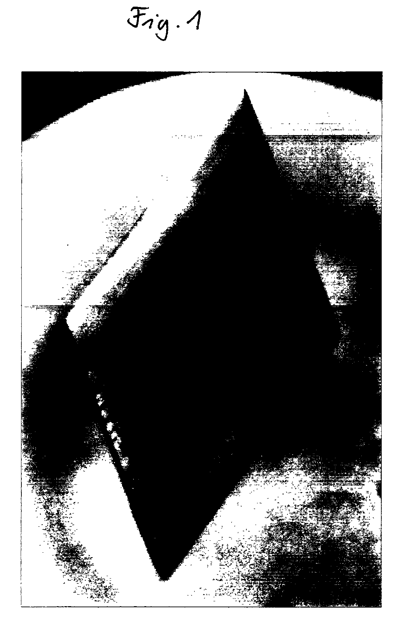

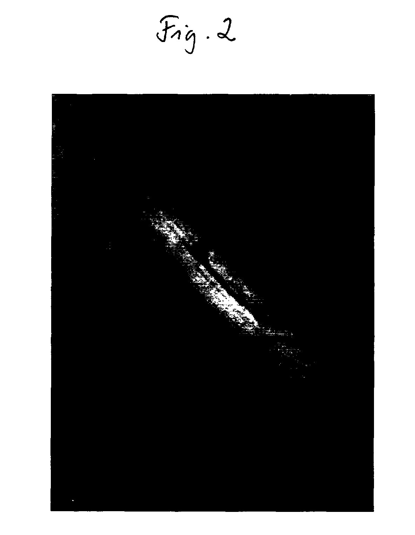

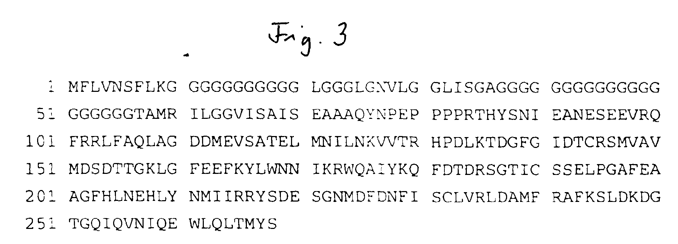

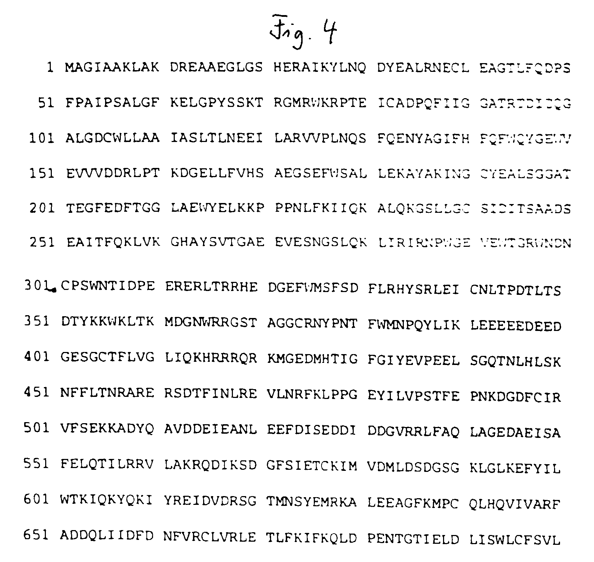

Fig. 1 stellt einen erfindungsgemäßen Kristall des Kristalltyps 1 mit einer erfindungsgemäßen Kristallform des humanen m-Calpains (kleine und große Untereinheit) mit dem Aussehen eines rhombusartigen Plättchens und den Einheitszellkonstanten a=64,78 Å, b=133,25 Å, c=77,53 Å und β=102,07° dar. Der abgebildete Kristall hat eine Größe von ca. 1mm x 1mm x 0,1mm.

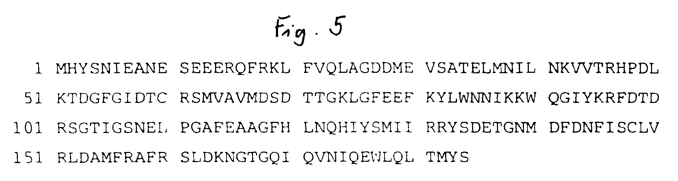

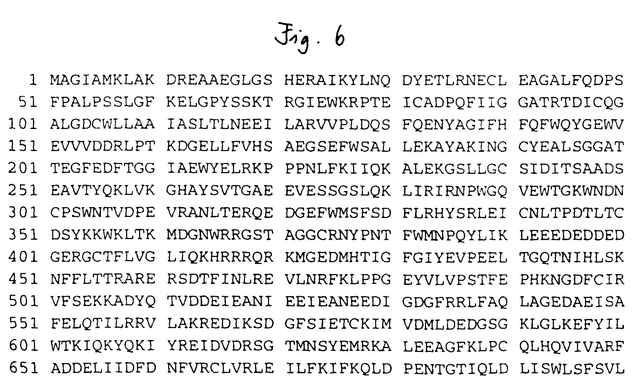

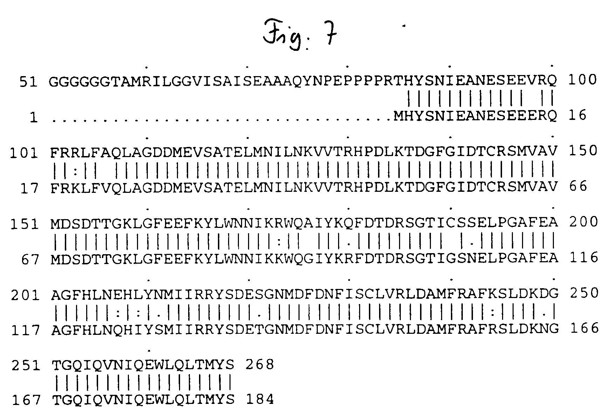

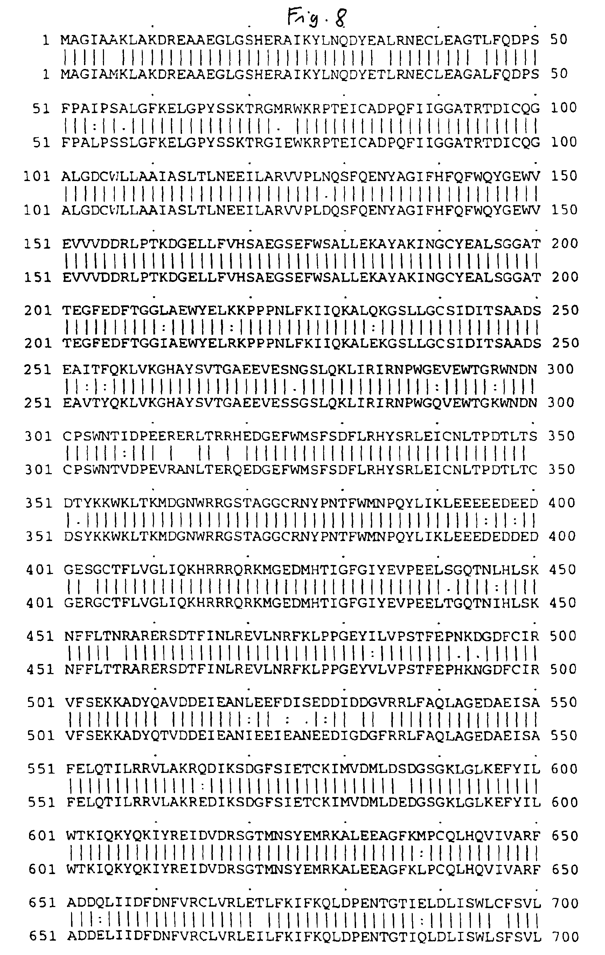

Fig. 2 zeigt einen Kristall des Kristalltyps 2, mit ebenfalls einer Kristallform, aufweisend eine große und eine kleine Untereinheit des humanen m-Calpains, mit plättchen- bis prismaartiger Morphologie. Er ist gekennzeichnet durch Einheitszellkonstanten a=51,88 Å, b=169,84 Å, c=64,44 Å und β=95,12°. Der in Fig. 2 dargestellte Kristall zeigt eine Größe von ca. 1mm x 0,2mm x 0,1mm.

Fig. 3 stellt die Aminosäuresequenz der kleinen Untereinheit von humanem m-Calpain (30 kDa-Untereinheit) dar. Die Aminosäuresequenz ist im Ein-Buchstaben-Code angegeben.

Fig. 4 stellt die Aminosäuresequenz der großen Untereinheit (80 kDa-Untereinheit) von humanem m-Calpain dar. Auch hier ist die Aminosäuresequenz im Ein-Buchstaben-Code angegeben.

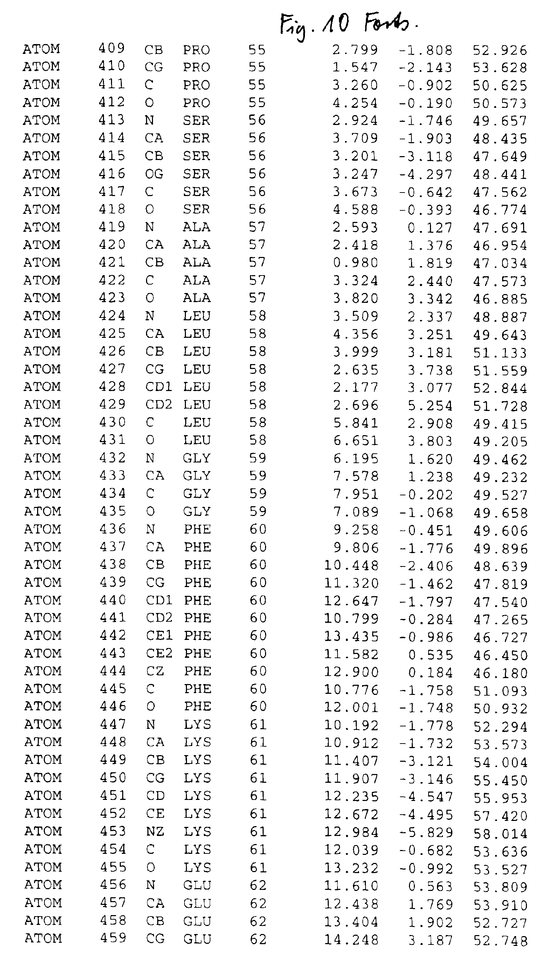

Fig. 5 stellt die Aminosäuresequenz (Ein-Buchstaben-Code) der kleinen Untereinheit vom m-Calpain der Ratte (Spezies: Rattus Norvegicus) dar.

Fig. 6 zeigt die Aminosäuresequenz der großen Untereinheit vom m-Calpain (80 kDa-Untereinheit) der Ratte (Spezies: Rattus Norvegicus) im Ein-Buchstaben-Code.

Fig. 7 stellt einen Vergleich der Aminosäuresequenzen zwischen den kleinen Untereinheiten von m-Calpain der Ratte bzw. der humanen Form dar. Die für den Vergleich herangezogenen Sequenzen entsprechen den in den Fig. 3 und 5 gezeigten Sequenzen, wobei die jeweils obere Reihe mit der humanen und die untere Reihe mit der Rattense-quenz korrespondiert. Liegen an den jeweils entsprechenden Positionen identische Aminosäuren vor, so sind sie durch einen Strich markiert, ähnliche Aminosäuren durch Doppelpunkte und nur entfernte Ähnlichkeiten der Seitenketten von korrespondierenden Aminosäuren durch einen Punkt. Bei diesem Vergleich ergaben sich eine Sequenzi-dentität von 91,85% und eine Sequenzähnlichkeit von 94,57%. Sequenzlücken in einer der beiden Vergleichsse-quenzen treten nicht auf. Es bestehen keine Identitäten und/oder Ähnlichkeiten zwischen Aminosäuren an den folgenden korrespondierenden Positionen (nur die humane Sequenzposition genannt): V98, A106, A176 und C190.

In Fig. 8 ist ein Vergleich der Aminosäuresequenzen der großen Untereinheit von m-Calpain des Menschen bzw. der Ratte dargestellt. Die jeweils obere Zeile entspricht der humanen Sequenz, wie sie auch in Fig. 4 dargestellt ist, die jeweils untere Zeile der Ratten-Sequenz, wie aus Fig. 6 ersichtlich. Die Erläuterungen zu Fig. 7 gelten im vorliegenden Fall analog. Sequenzdifferenzen zwischen der humanen und der Ratten-Sequenz sind an den Positionen (im folgenden jeweils nur Angabe der Position in der humanen Sequenz): A6, A34, T54, R74, E311, E313, R314, R317, H319, S350, S403, N456, A511, F523, I525, D531, V534, S586, T671 und C696 festzustellen. Der Prozentsatz identischer Aminosäuren beträgt 93%, und der Prozentsatz äh-licher Aminosäuren als Ergebnis dieses Vergleichs 96,86%. Sequenzlücken bei einer der beiden Sequenzen konnten bei dem Sequenzvergleich nicht beobachtet werden.

Fig. 9 bildet die Aminosäuresequenzen der großen Untereinheit von humanem m-Calpain (80 kDa-Untereinheit) sowie die Aminosäuresequenz der kleinen Untereinheit von humanem m-Calpain (30 kDa), jeweils im Ein-Buchstaben-Code, in Kombination mit strukturellen Angaben ab. Die unter den jeweiligen Aminosäuren angegebene Zeichen (Zylinder oder Pfeile) repräsentieren die von den derart gekennzeichneten Aminosäuren jeweils eingenommene Sekundärstruktur. Hierbei stehen die Zylinder für Aminosäuren, die eine Helixstruktur einnehmen, und Pfeile für solche Aminosäuren, die Bestandteil von Strängen mit sog. β-Konformation sind. Die Zuordnung der in einer Konformation mit Sekundärstruktur vorliegenden Aminosäuren erfolgt abhängig von den Torsionswinkeln der Hauptkette um das Cα-Atom der jeweiligen Aminosäure, wobei für Einzelheiten auf Lehrbücher der Biochemie, beispielsweise auf Lehnin-ger, Nelson & Cox, Prinzipien der Biochemie, Spektrum Akademischer Verlag GmbH, 1998, verwiesen wird. Jeweils rechts von den Sekundärstruktur-Symbolen ist nach eindeutiger Nomenklatur (α für Helix, β für einen Strang in β-Konformation, dann fortlaufende Numerierung der Sekundärstruktur und schließlich in römischen Zahlen die Domänen-bezeichnung) die Bezeichnung der jeweiligen Sekundärstruktur angegeben. Oberhalb von der Aminosäuresequenz im Ein-Buchstaben-Code finden sich gegenläufig ausgerichtete schwarze Pfeile, die die Domänengrenzen markieren. Funktionell wichtige Reste sind durch oberhalb derselben angeordnete Symbole bezeichnet (so Aminosäuren aus dem katalytischen Reaktionszentrum oder weitere wichtige Aminosäuren aus den Sub-Domänen IIa und IIb durch rote bzw. schwarze Dreiecke, saure Aminosäuren der Schalterschleife in der Domäne III und die benachbart gelegenen positiv geladenen Aminosäuren der Sub-Domäne IIb durch rote bzw. blaue Kreise). Die an der Ca-Bindung der Domäne VI der kleinen Untereinheit beteiligten Aminosäuren sind durch schwarze Kreise gekennzeichnet.

Nach einer entsprechend Sorimachi et al. (s. a.a.O.) modifizierten Unterteilung der beiden den Calpain-Komplex bildenden Polypeptidketten unter Berücksichtigung der-strukturellen Gegebenheiten lassen sich für das humane m-Calpain die folgenden Domänengrenzen bestimmen. Große Untereinheit: Domäne I (M1 bis E16), Linker-Bereich zwischen Domäne I und Domäne II (G17 bis A92), Sub-Domäne IIa (T93 bis G209), Sub-Domäne IIb (G210 bis N342) , Linker-Bereich zwischen Domäne II und Domäne III (L343 bis K355), Domäne III (W356 bis A511), Linker-Bereich zwischen Domäne III und Domäne IV (V512 bis D529), Domäne IV (I530 bis L700). Kleine Untereinheit: Domäne V (M1 bis E94) und Domäne VI (S95 bis S268).

Im folgenden werden die einzelnen Sekundärstrukturen der großen und kleinen Untereinheit, beginnend am N-Terminus der großen Untereinheit, beschrieben.

Bei der großen Untereinheit (80 kDa-Untereinheit) liegt eine helikale Struktur zwischen den Aminosäuren 4 und 15 (Helix α1I) in der Domäne I (in grün) vor. Aminosäure 17 markiert den Beginn der Domäne II (Sekundärstrukturen der Domäne IIa in gelb), wobei sich zunächst die Aminosäuren 31 bis 44 in einer α-helikalen Konfirmation befinden (α1II). Die nächste Sekundärstruktur, ebenfalls eine α-Helix, beginnt mit Aminosäure D104 und läuft bis zur Aminosäure N118 (a2II). Sie wird unmittelbar gefolgt von einer weiteren α-Helix (α3II), beginnend mit Aminosäure E118 - V125. Ab Aminosäure I138 nimmt die große Untereinheit mehrfach die Konformation von β-Faltblattsträgen an, nämlich β1II (I138 - Q145, E148 - D156, P159 bis K161 und E164 - L166 (β4II)). Eine Helix kann zwischen den Aminosäuren F176 und G190 (α4II) und Y192 - S196 (α5II) beobachtet werden. Schließlich findet sich als letzte Sekundärstruktur in der Domäne IIa eine Helix von Aminosäure T200 - T208.

Die nun folgenden Sekundärstrukturen der Domäne IIb sind rot gekennzeichnet. Mit G210 wird der Beginn der Domäne IIb definiert, wobei von I211 bis L217 ein β-Faltblattstrang (β5II) verläuft. Danach nimmt ab Aminosäure N223 das Polypeptid helikale Struktur bis Q233 (α7II) an. Ab L237 bis I242 liegt die Konformation eines β-Strangs (β6II) vor. Weitere Sekundärstrukturen in der Domäne IIb sind zwei β-Faltblattstränge von H262 bis S274 und S277 bis N286. Hierauf folgen in der Domäne IIb folgende Sekundärstrukturelemente: α-Helix von P309 bis T316, ein β-Faltblatt-Strang von G322 bis M326, eine α-Helix von S327 bis R333 und schließlich von S336 bis N342 ein ein β-Faltblattstrang.

Für die Domäne III sind mit blauer Markierung die folgenden Sekundärstrukturen hervorgehoben: ein β-Faltblatt-strang von K357 bis W365 (βlIII), ein β-Faltblattstrang von Q386 bis L391 (β₂III), ein β-Faltblatt-Strang von C405 bis K414 (β₃III), ein weiterer β-Faltblatt-Strang von T428 bis E435 (β₄III), eine α-Helix von S449 bis N456 (α₁III), und schließlich vier β-Faltblattstränge von R469 bis L477 (β₅III), G480 bis F489 (β₆III), G495 bis E504 (β₇III) und schließlich D508 bis V512 (β₈III).

Die Domäne IV, eine Calcium-Bindungsdomäne, ist dadurch gekennzeichnet, daß sie ausschließlich α-helikale Sekundärstrukturen aufweist. In entsprechender Reihenfolge sind die folgenden α-Helices zu nennen (gelb markiert): I530 bis A542 (α₁IV), S549 bis L561 (α₂IV), E575 bis D585 (α₃IV), G593 bis V616 (α₄IV), N623 bis G535 (α₅IV), P639 bis F650 (α₆IV), D658 bis D680 (α₇IV) und schließlich D690 bis L700 (α₈IV) .

In der kleinen Untereinheit sind die ersten 84 Aminosäuren aus der Domäne V in der Elektronendichtekarte auf Grund starker Flexibilität nicht definiert, weswegen die entsprechenden Strukturdaten nicht vorliegen. In der Domäne VI, beginnend mit S95, die gleichfalls eine Calcium-Bindungsdomäne darstellt, sind ebenfalls ausschließlich α-helikale Strukturelemente zu finden. Im einzelnen handelt es sich um die folgenden α-helikalen Sequenzabschnitte: S95 bis L108 (α₁VI), S116 bis R130 (α₂VI), G140 bis D152 (α₃VI), G160 bis D182 (α₄VI), C190 bis G202 (α₅VI), L209 bis S218 (α₆VI), D225 bis D247 (α₇VI) und schließlich N257 bis Y267 (α₈VI).

Figur 10 listet die Koordinaten der einzelnen Atome der beiden Untereinheiten von humanem m-Calpain auf. Die Koordinaten geben die Struktur der beiden Untereinheiten in einem x-, Y-, z-Koordinatensystem wieder. Die Reihenfolge der Atome in Figur 10 wird durch deren Zugehörigkeit zu Aminosäuren einer bestimmten Sequenzposition definiert. Hierbei erfolgt die Auflistung der Aminosäuren vom N- zum C-Terminus zunächst für die große, dann für die kleine Untereinheit von humanem m-Calpain. Da die an Position 1 des N-Terminus der langen Untereinheit sitzende Aminosäure Methionin M1 wegen typischerweise an den Termini zu beobachtender starker konformationeller Flexibilität keine Elektronendichte in der Elektronendichtekarte erkennen läßt, wird in Figur 10 zu Anfang die Aminosäure A2 geli-stet. In Figur 10 werden entsprechend der international üblichen Nomenklatur (Bernstein et al., J. Mol. Biol. 112, 535ff., 1977, einschließlich der dort zitierten Veröffentlichungen) die Strukturkoordinaten sämtlicher Atome einer erfindungsgemäßen Kristallform (als Bestandteil eines Kristalls) von humanem m-Calpain dargestellt, abgesehen von Wasserstoffatomen, die sich in der Elektronendichtekarte nicht durch entsprechende Elektronendichten manifestieren. In Figur 10 sind auch die Positionen der Sauerstoffatome der für die Kristallform ermittelten Was-sermoleküle enthalten.

In Figur 11 findet sich eine schematisierte und dem Fachmann in dieser Form geläufige Darstellung der dreidimensionalen Struktur der beiden Untereinheiten von humanem m-Calpain, wobei die strukturelle Konformation des Komplexes in Abwesenheit von Calcium abgebildet ist. Hierbei handelt es sich um eine sog. "Ribbon"-Darstellung, die nur das Rückgrat der Polypeptidketten berücksichtigt und die jeweils am Cα-Atom ansitzenden Seitenketten bzw. deren Konformation nicht wiedergibt. Die Positionen des Rückgrats der beiden Polypeptidketten mit den die Struktur bestimmenden, freien Torsionswinkeln um die Cα-Atome jeder in der Elelktronendichtekarte erfaßten Aminosäure von humanem m-Calpain werden durch den Bandverlauf in dem "Ribbon"-Diagramm nachgezeichnet. Dabei sind in dieser Darstellungsweise helikale Strukturen als Helices und β-Faltblattstränge als Pfeile markiert, während Sequenzbe-reiche ohne derartige Sekundärstrukturelemente als Faden eingezeichnet sind. Im vorliegenden Fall sind die einzelnen Domänen der kleinen und großen Untereinheit farbig gekennzeichnet. Die jeweils eingezeichneten Sekundärstrukturelemente korrespondieren im übrigen mit den strukturellen Angaben, die in Figur 9 der die Primärsequenz der beiden Polypeptide zugeordnet sind.

Der m-Calpain-Komplex hat die Form einer flachen ovalen Scheibe. Die oberen und unteren Pole (gemäß der Refe-renzorientierung der Figur 11) werden von den beiden katalytischen Subdomänen IIa und IIb bzw. dem Calmodulinähnlichen Domänenpaar aus der großen bzw. kleinen Untereinheit gebildet. Die Domäne III und die N-terminalen Domänen I (gr. Untereinheit) und V (kleine Untereinheit) verbinden die beiden Calmodulin-artigen Domänen mit den beiden katalytischen Sub-Domänen. Die insgesamt zu beobachtende Ähnlichkeit der Kristallformen von m-Calpain in der Röntgenstrukturanalyse für jede der beiden Kristalltypen (1) und (2) läßt vermuten, daß die Struktur von calciumfreiem m-Calpain, wie in Figur 11 gezeigt, unabhängig von der spezifischen Packung im Kristall ist.

Die Aminosäurekette der großen Untereinheit beginnt mit einer sog. "Ankerhelix" (α1I) der Domäne I (in grün), die in einer halbrunden Kavität der Domäne VI positioniert ist. Von dort läuft die Aminosäurekette geradewegs zur Domäne IIa. Die Ankerhelix bewirkt infolge ihrer Interaktionen mit der Domäne VI unter anderem, daß beide Untereinheiten des m-Calpainkomplexes zusammengehalten werden. Bei der Aminosäure Glyl9 der großen Untereinheit (Zählweise für humanes m-Calpain, siehe Figur 4) kontaktiert die Aminosäurekette der großen Untereinheit die Sub-Domäne IIa (in gelb), wobei sich die Polypeptidkette in diesem Bereich zu einer α-Helix (α1II) und verschiedenen "Turn"-Strukturen zur Ausbildung einer äußeren polaren Oberfläche des Komplexes auffaltet.

Von der Aminosäure T93 sind die Aminosäuren der langen Polypeptidkette am Strukturbereich der katalytischen Domäne beteiligt, die topologisch mit der katalytischen Domäne der Protease Papain verwandt ist. Allerdings bestehen erhebliche Unterschiede zur katalytischen Domäne von Papain bereits darin, daß die beiden beim Calpain charak-teristischerweise separierten Sub-Domänen sowohl bezüglich der Länge als auch ihrer Konformation erheblich von der korrespondierenden Struktur bei der Protease Papain abweichen.

Aus der in Figur 11 gewählten Perspektive (Referenzposition des Komplexes) bildet die Domäne IIa die linke Hälfte der katalytischen Furche, die sich am Zwischenraum der Subdomäne IIa und IIb (in rot) aus Aminosäuren beider Sub-Domänen zusammensetzt. In der Figur 11 sind die im wesentlichen an der katalytischen, d.h. proteolytischen, Reaktion beteiligten Aminosäuren mit der Position ihrer Seitenketten, allerdings ohne Darstellung der Wasserstoffatome, eingezeichnet. Dabei handelt es sich um die Aminosäuren Cys105 und Trp106 der Sub-Domäne IIa und His262, Asn286, Trp288 und Pro287 der Subdomäne IIb.

An dem konformationellen "Scharnier" zwischen Gly209 und Gly210 der großen Untereinheit tritt die Polypeptidkette in die faßartig strukturierte Sub-Domäne IIb über, wo sie ein typisches 6-strängiges β-Faltblatt (Stränge β₅II bis β₁₀II) bildet. Die faßatige Struktur wird durch die Super-sekundärstruktur erreicht, d.h. die Anordnung der Sekundärstrukturelemente. Insbesondere erwähnenswert ist die Folge der Aminosäuren Asn286, Pro287 und Trp288 mit ihren besonderen Konformationen, wobei Asn286 an der katalytischen Reaktion beteiligt ist. Vom Strang β₁₀II aus verläuft die Polypeptidkette zunächst in Richtung Sub-Domäne IIa, bevor sie über eine offene Schleifenstruktur (einen sog. Linker-Bereich) Bestandteil der Domäne III (in blau) wird.

Die Domäne III besteht im wesentlichen aus zwei gegenläufigen β-Faltblättern, wobei jedes β-Faltblatt vier antiparallele Stränge aufweist. Dies führt zu einer kompakten Tertiärstruktur, die β-sandwichartig ist. Die Topologie dieser Domäne erinnert entfernt an das TNFα-Monomer oder einige Virusoberflächenproteine. Bemerkenswert sind die basischen Aminosäuren His415 bis His427 in der Domäne III, die eine im Zentrum des Calpainkomplexes liegende Schleife bilden. Ebenso auffällig ist die gegenüber Lösungsmittel exponierte und negativ geladene β₂III/β₃III-Schleife, die zehn saure Aminosäuren innerhalb des elf Aminosäuren umfassenden Abschnitts Glu392 bis Glu402 aufweist. Diese Schleife ist kristallographisch gut erfaßt. Sie ist räumlich nahe an der Helix α₇II der Sub-Domäne IIb und der offenen Schleife von der Subdomäne IIb angeordnet und interagiert elektrostatisch mit den zahlreichen positiven Ladungen dieser beiden Abschnitte der Sub-Domäne IIb.

Von der Domäne III verläuft die Aminosäurekette entlang der Calmodulin-artigen Domäne IV in einer ausgestreckten ("extended") Konformation, wodurch mehrere saure Aminosäuren in direktem Kontakt mit dem Lösungsmittel stehen. Hierbei handelt es sich um einen weiteren, langen Linker-Bereich (in magenta) ohne charakteristische Sekundärstruktur, der bis an die unterste Position der Domäne IV (in gelb) nach der hier gewählten Referenzperspektive reicht. Bei der Aminosäure Ile530 beginnt die Tertiär-struktur der Domäne IV, die weitgehend mit der Faltung der aus dem Stand der Technik bekannten Strukturen der isolierten Domänen VI des Calpains der Ratte und des Schweins bekannt sind. Beide Domänen IV und VI zeigen Ähnlichkeit zu anderen Calcium-Bindungsdomänen, nämlich "Calmodulin"-Domänen, mit EF-Hand-Motiv. Die Domäne IV (in gelb) weist als Sekundärstrukturelemete acht α-Helices auf, die durch charakteristische Linker-Bereiche verbunden sind, wodurch fünf der wohlbekannten Superse-kundär-Strukturelemente, die als EF-Hände bezeichnet werden, gebildet werden (Tooze & Branden, Introduction into Protein Structure, Garland Publishing Inc., Dec. 1998, 2. Auflage).

Der N-terminale Teil des Polypeptids, das die kleine Calpain-Untereinheit bildet, ist reich an Glycinresten und läßt keine für die Strukturaufklärung verwendbare Elektronendichte erkennen. Ab Aminosäure Thr85 der Domäne V kann der kleinen Untereinheit eine dreidimensionale Struktur zugeordnet werden (gem. Figur 11 in rot), allerdings liegt die Polypeptidkette hier nicht in einer der beiden typischen Sekundärstrukturen vor. An der gegenüber Lösungsmittel exponierten Oberfläche der Kristallform faltet sich die Polypeptidkette zurück und läßt dort mit der a-Helix alVI das erste Sekundärstrukturelement der Domäne VI (in orange) erkennen.

Die Domäne VI weist gleichfalls (wie Domäne IV) fünf EF-Hand-Motive auf und bildet zusammen mit der Domäne IV der großen Untereinheit ein quasi symmetrisches Heterodimer, da die Domäne VI in struktureller Nachbarschaft zur Domäne IV liegt, nämlich auf der linken Seite der in Figur 11 gewählten Perspektive der kleinen Untereinheit. Hierbei sind die Helices α₆VI, α₇VI und α₈VI und der Linker-Bereich zwischen α₇VI und α₈VI (α₇VItα₈VI) an den symmetrischen Interdimerkontakten beteiligt.

Die beiden Domänen IV und VI sind nicht primär an der Regulation der katalytischen Aktivität von Calpain beteiligt, da die Bindung von Calcium-Ionen keine strukturellen Veränderungen hervorruft, wie durch einen Vergleich mit den nach dem Stand der Technik bekannten Kristallformen der Domäne VI von Ratten-Calpain mit gebundenem Calcium erkennbar wird. Die Calmodulin-artigen Domänen können daher primär strukturelle Aufgaben übernehmen. Die vorliegenden Untersuchungen lassen auch vermuten, daß die Calcium-Bindung an die Domänen IV und VI zu einer Disso-ziierung der beiden Untereinheiten eines Calpains, insbesondere eines m-Calpains, führt.

Die katalytische Domäne wird durch die beiden Sub-Domänen IIa und IIb gebildet. In der Calcium-freien Kristallform, wie in Figur 11 dargestellt, liegt keine katalytisch aktive Konformation vor, vielmehr ist eine deutliche Furche zwischen den beiden vorgenannten Sub-Domänen erkennbar. In der in Figur 11 abgebildeten dreidimensionalen Struktur liegen daher die beiden katalytisch wirksamen Seitenketten von Cys105 (SG) und ND1 von His262 8,5° auseinander, wobei jedoch für die Katalyse die Imidazol-Seitenkette von His262 in die Nähe von Cys105 gebracht werden und gleichzeitig auch die Wasserstoffbrückenbindung zwischen His262 NE2 und Asn286 ND2 geschlossen werden muß. Diese Furche zwischen den Sub-Domänen kann dadurch geschlossen werden kann, daß die Sub-Domäne IIb um 50° rotiert und 12 Å gegen die Sub-Domäne IIa translatiert wird. Erst nach einer derartigen Bewegung kann Calpain seine katalytische Aktivität entfalten. Im Zuge der konformationellen Aktivierung von Calpain kann schließlich auch die Indolgruppe von Trp288 der großen Untereinheit ihre entsprechende Schutzfunktion gegenüber Wassermolekülen aus dem Lösungsmittel ausüben und damit eine ungestörte Proteolyse des Substrats sicherstellen.

Physiologisch wird die proteolytische Aktivität von Calpain nur in Gegenwart von Calcium gewährleistet. Wie bereits zuvor erwähnt, bleibt die Bindung von Calcium an die Domänen IV und VI des Calpain-Komplexes aber ohne regulatorische Auswirkung auf die Struktur der katalytischen Domäne. Vielmehr erscheint eine Anlagerung von Calcium-Ionen an die sauren Reste des sauren "Loops" der Domäne III für eine Zusammenlagerung der Sub-Domänen IIa und IIb zur Ausbildung eines aktiven Reaktionszentrums als entscheidender Calcium-Bindungsplatz. Hierdurch wird ein Konformationswechsel ausgelöst, der dann auch regulatorische Auswirkungen hat.

Der saure "Loop" (β₂IIItβ₃III) weist, wie bereits zuvor erwähnt, zehn negativ geladene Seitenketten auf, die infolge der elektrostatischen Abstoßung voneinander wegzei-gen. Dieser saure "Loop" steht in unmittelbarem Kontakt mit der amphipatischen α₇II-Helix der Sub-Domäne IIb und der offenen Schleife ("Loop") der Sub-Domäne IIb, mit den Lysin-Resten K226, K230, K234, K354, K355 und K357. Zwischen einigen der vorgenannten sauren und basischen Ami-nosäureseitenketten bilden sich direkte Salzbrücken über die Domänengrenzen hinweg, da sich die von den entsprechenden Seitenketten getragenen negativen bzw. positiven elektrostatischen Potentiale gegenseitig anziehen. Die Bindung von einem oder mehreren Calcium-Ionen, bspw. unter entsprechenden physiologischen Bedingungen, die nach einer Ca-Freisetzung unter anderem die Calpain-Aktivität erhöhen, an dieser sauren Schleife sorgt mindestens teilweise für eine Ladungskompensierung. Vorzugsweise binden die sauren Aminosäuren des sauren "Loops" mehr als ein Calcium-Ion, z.B. zwei oder drei Calcium-Ionen.

Die Bindung mindestens eines Calcium-Ions an dieser Stelle kompensiert die außerordentlich starke Anhäufung von negativen Ladungen in diesem Strukturbereich und führt gleichzeitig auch zu einer kompakteren Faltung im Bereich des sauren Loops, da die elektrostatische Abstoßung der negativ geladenen Seitenketten in diesem Strukturbereich reduziert wird. Eine Erniedrigung der elektrostatischen Wechselwirkung zwischen dem sauren "Loop" und den zuvor genannten basischen Aminosäuren erlaubt es auch, daß sich die Sub-Domäne IIb aus ihrer in Figur 11 dargestellten fixierten Position löst und sich auf die Sub-Domäne IIa zubewegt, wodurch die zwischen den Sub-Domänen in Abwesenheit von Calcium (wie in Figur 11 dargestellt), was den inaktiven Zustand der Protease simuliert, vorhandene Furche geschlossen wird.

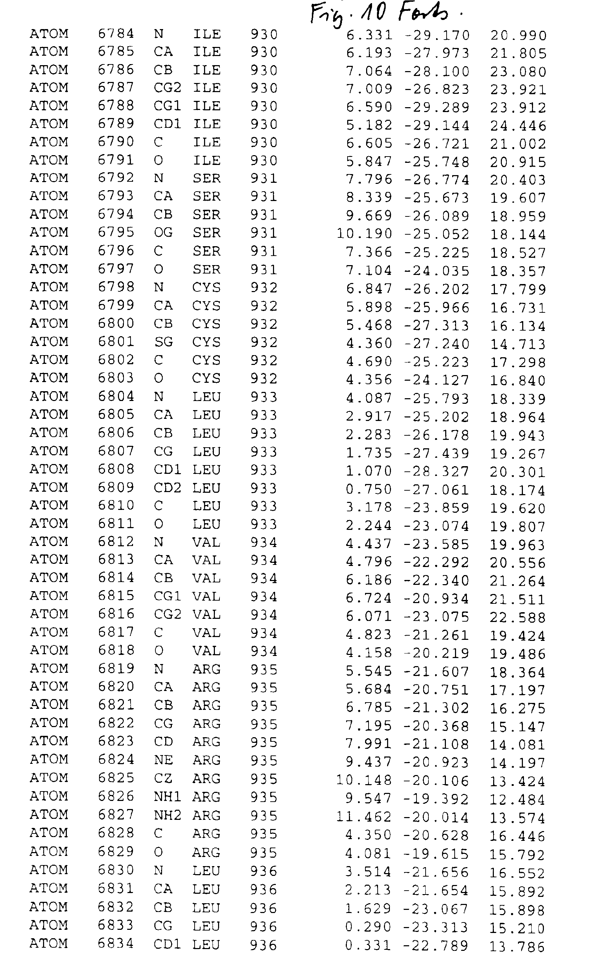

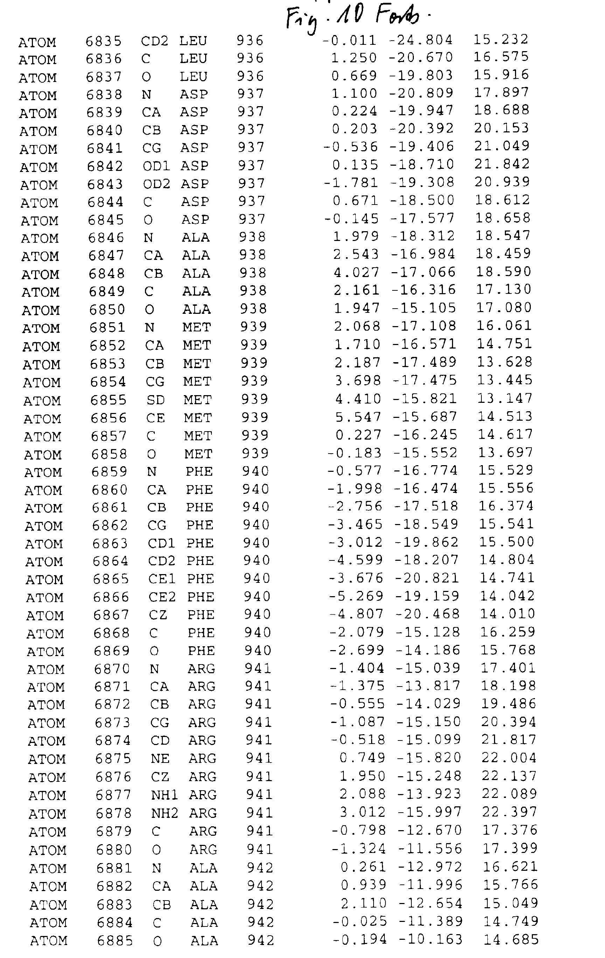

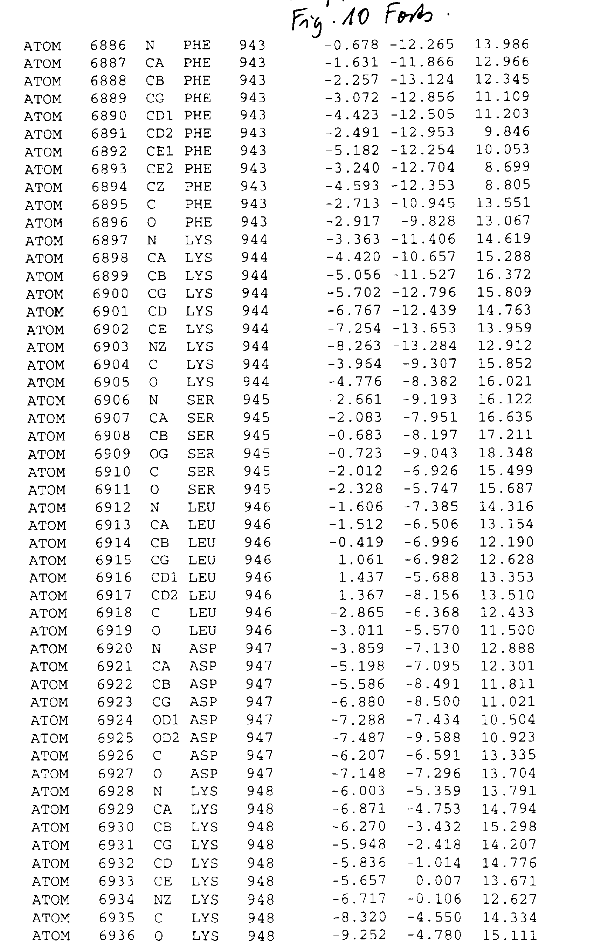

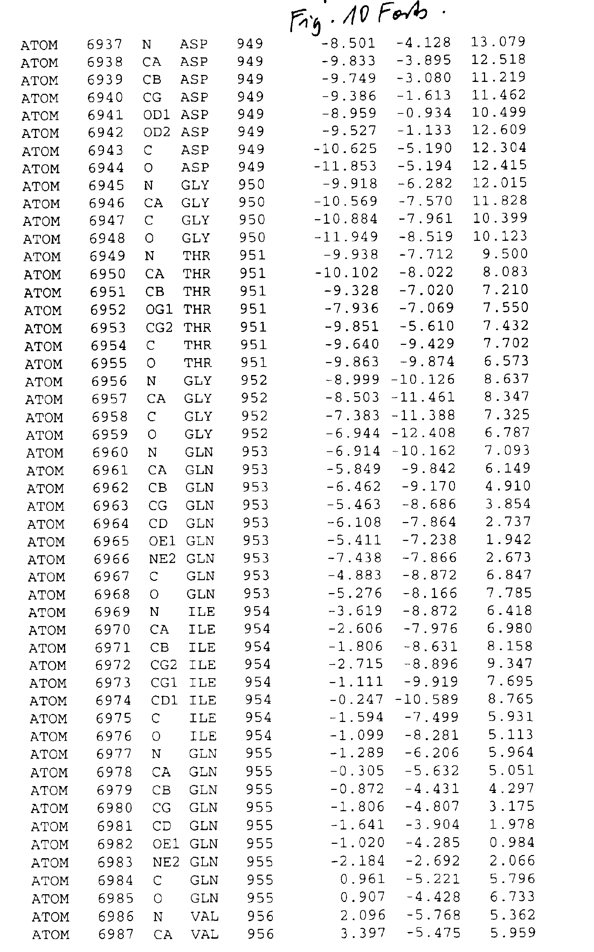

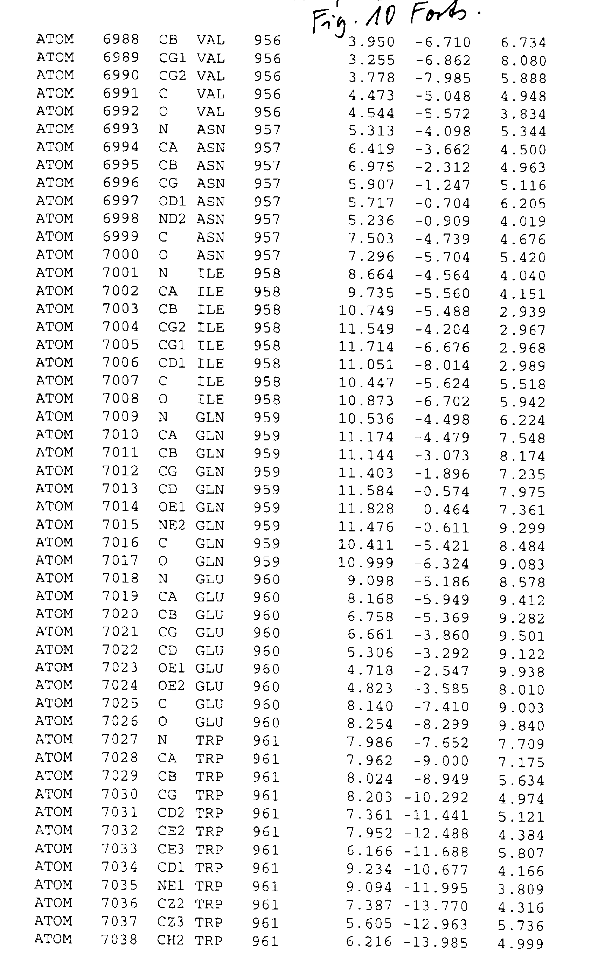

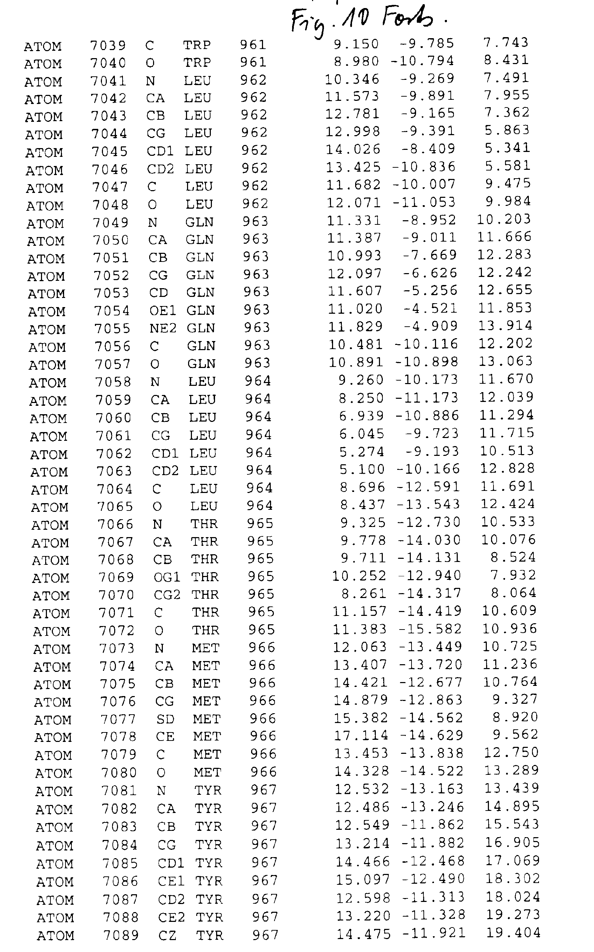

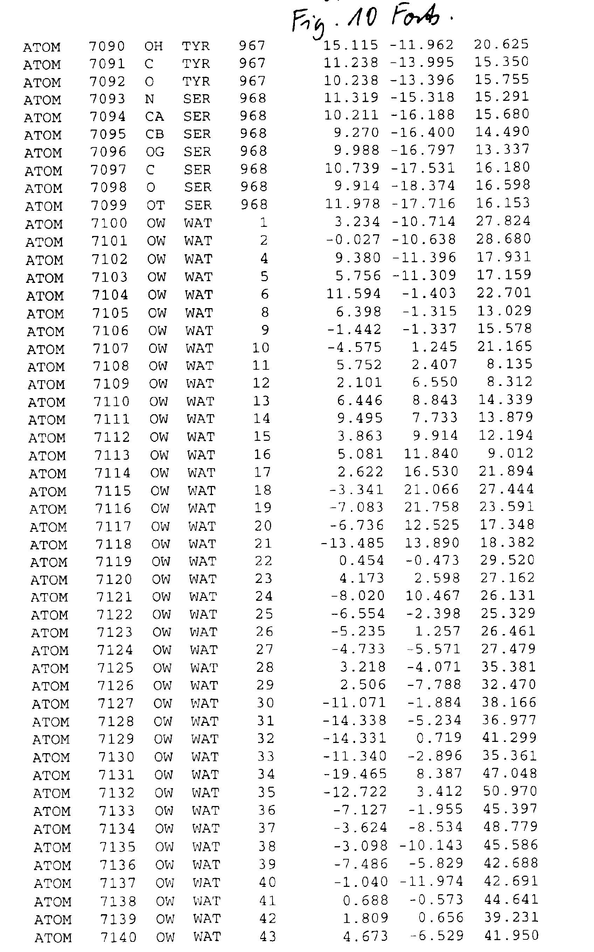

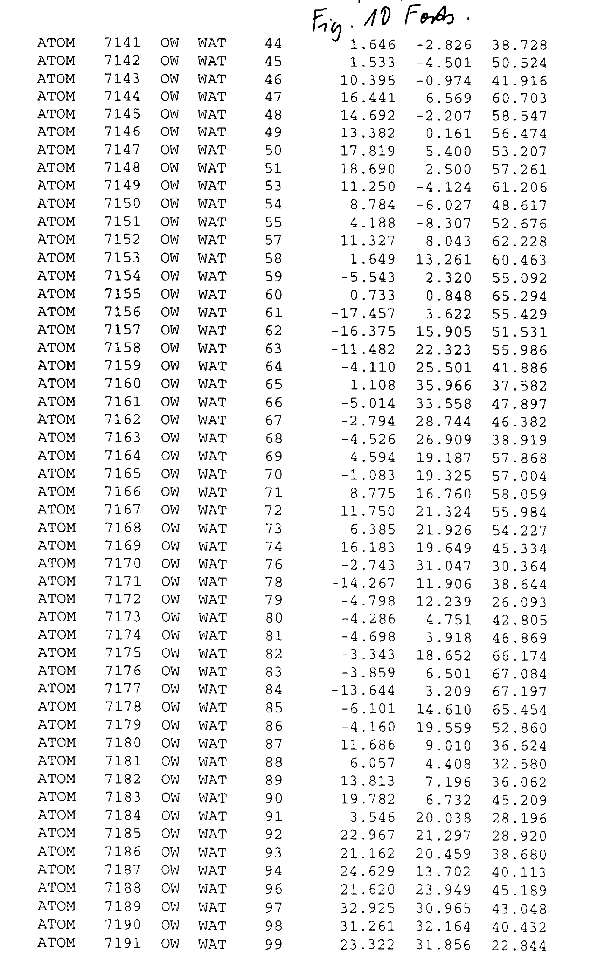

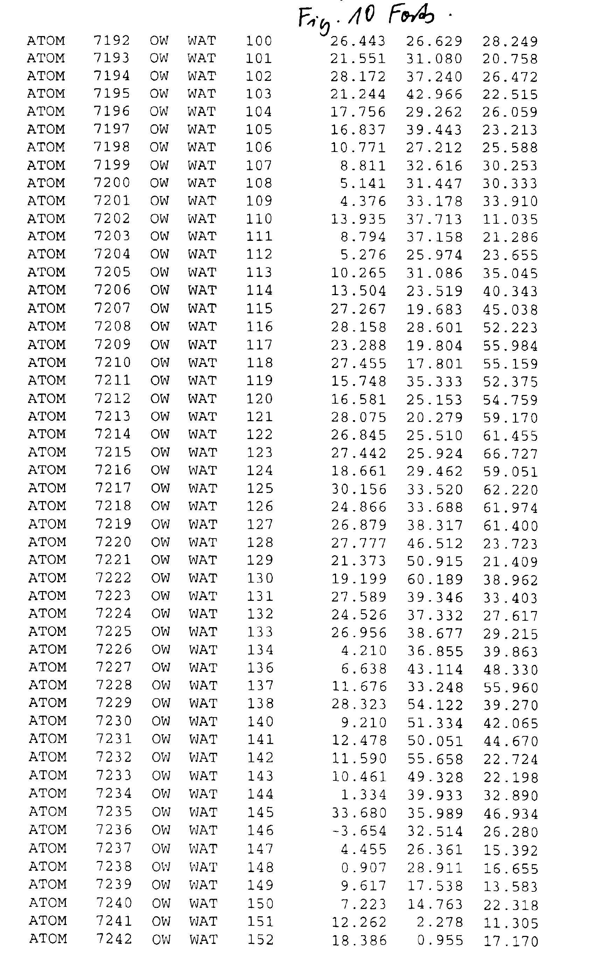

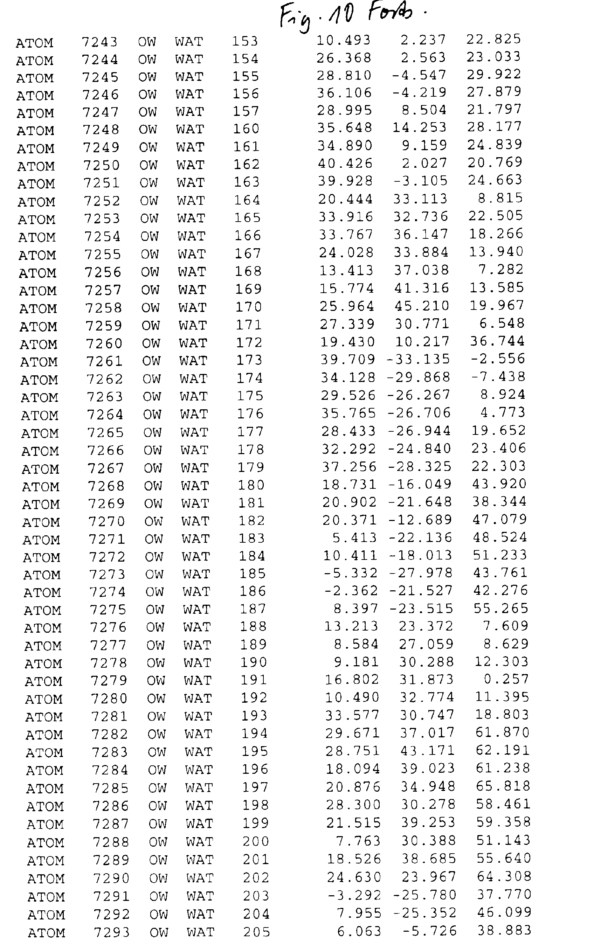

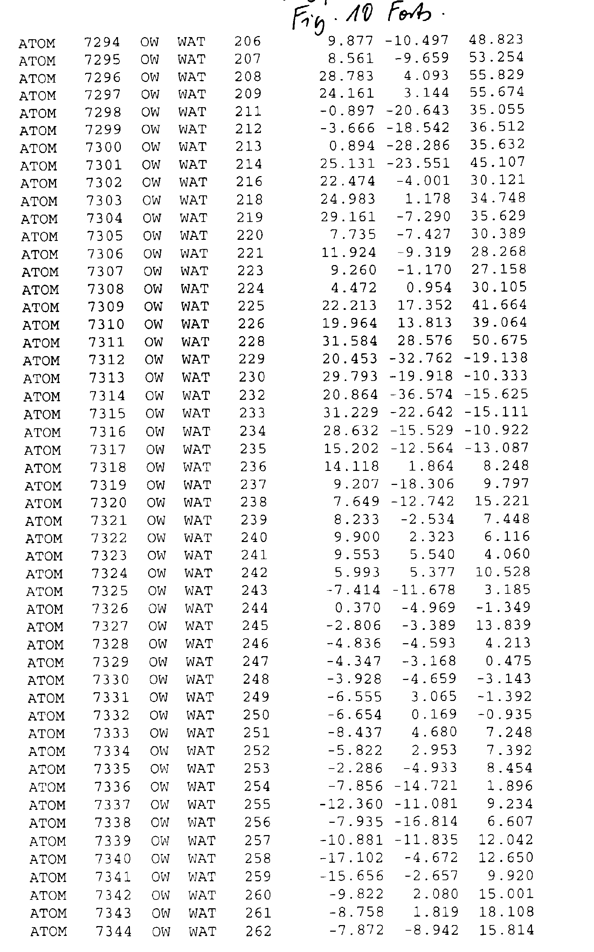

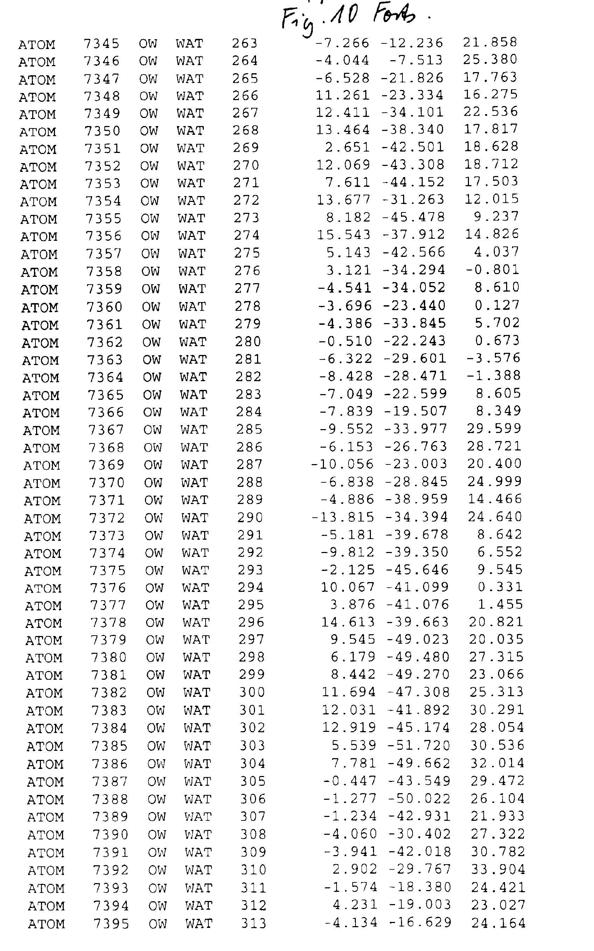

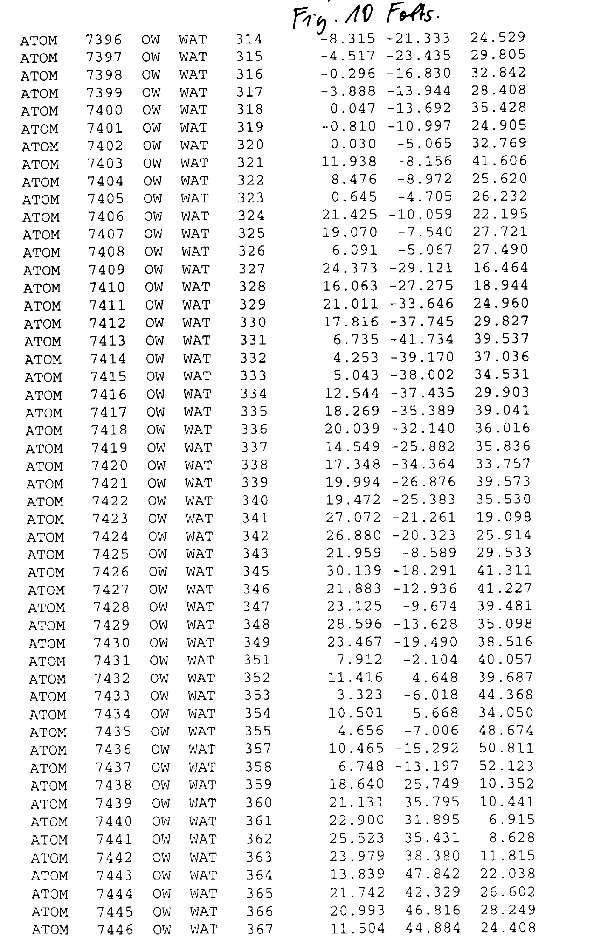

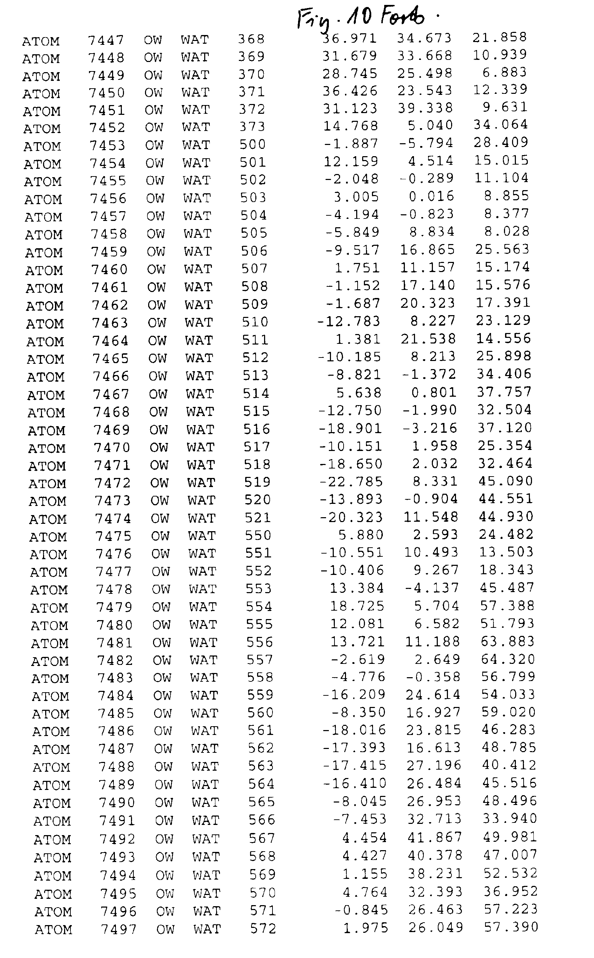

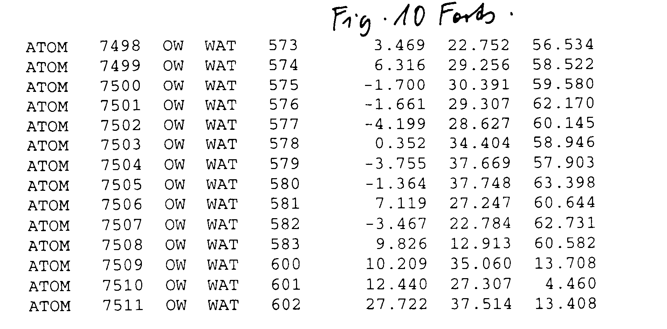

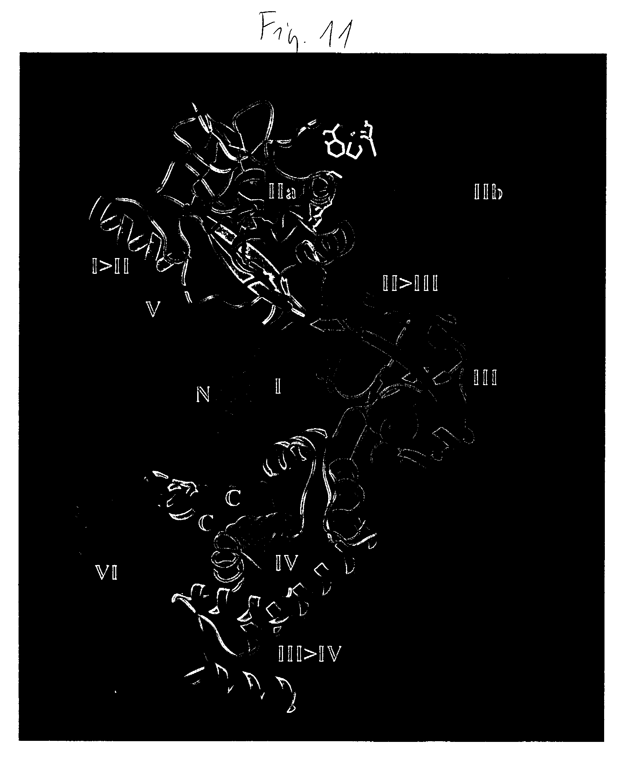

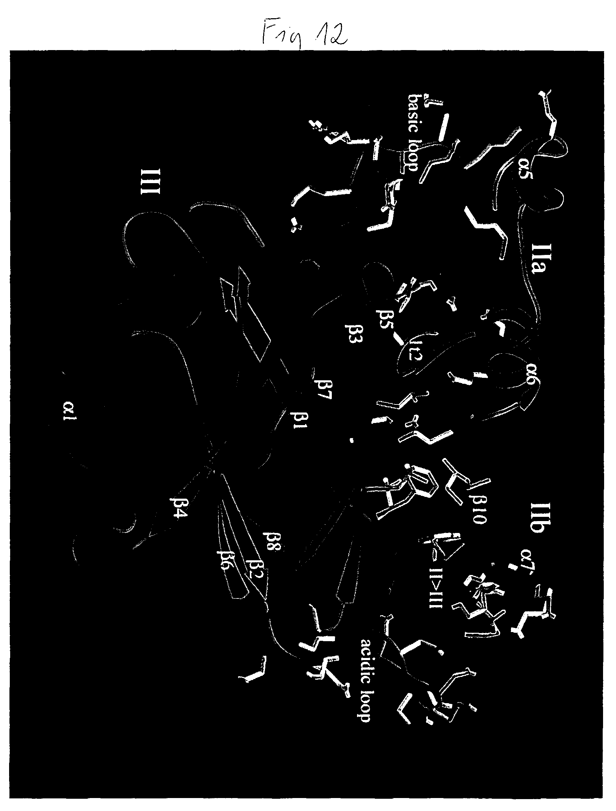

Ein derart ausgelöster Konformationswechsel der Subdomäne IIb, der den Komplex aus der inaktiven in die aktive Form überführt, wird auch durch die, in Figur 12 dargestellt, hydrophobe Region zwischen den β-Strängen β₅II und β₁₀II des β-Faltblatts de Domäne IIb und den Strängen β₃III, β₅III und β₇III des β-Faltblatts der Domäne III erlei-chert. Die Ansammlung der hydrophoben Aminosäureseiten-ketten in diesem Bereich erlaubt eine Gleitbewegung der Sub-Domäne IIb in Richtung auf die Sub-Domäne IIa. Gleichzeitig ist die Sub-Domäne IIa durch ihre zahlreichen polaren Wechselwirkungen mit der Domäne III gegenüber derselben fest positioniert und damit fixiert.

Während die m-Calpaine im Bereich des sauren "Loops" zehn negative Ladungen aufweisen, sind in dem korrespondierenden Strukturbereich bei µ-Calpainen nur acht negative Ladungen als Folge der Primärsequenz angeordnet. Daher benötigen m-Calpaine eine stärkere Ladungskompensierung in diesem Bereich als µ-calpaine, die typischerweise niedrigere Calcium-Spiegel oder kein Calcium für den Konformationswechsel und die Aktivierung der katalytischen Region benötigen. Ein entsprechendes Muster wird im übrigen in dem benachbarten basischen Bereich von µ-Calpainen beobachtet. Basische Aminosäuren an den Positionen 226 und 357 der großen Untereinheit treten nur bei m-Calpainen auf, so daß bei den µ-Calpainen einer geringeren Anzahl an sauren Aminosäuren im sauren "Loop" auch eine geringere Anzahl an strukturell benachbarten basischen Aminosäuren gegenübersteht.

Schließlich können weitere Verbindungen die Interaktion zwischen dem sauren "Loop" der Domäne III und den basischen Aminosäuren der Sub-Domäne IIb bei m- und/oder µ-Calpainen reduzieren. So können vorzugsweise saure Phospholipide, wie z.B. phosphorylierte Phosphatidylinosito-le, die für die Aktivierung erforderlichen Calcium-Konzentrationen senken, indem sie mit den basischen Aminosäuren der Sub-Domäne IIb in Wechselwirkung treten und damit das Wechselwirkungsstärke zwischen den basischen und suren Aminosäuren reduzieren. Somit sind insbesondere saure Phospholipide als Liganden an eine vorliegend beanspruchte Raum- und/oder Kristallform erfindungsgemäß bevorzugte aktivierende Liganden. In einem anderen bevorzugten Fall weisen erfindungsgemäße Liganden positive Ladungen und/oder Teilladungen auf, die die negativen Ladungen des sauren "Loops" kompensieren und dadurch die Sub-Domäne IIb für den Calpain-aktivierenden Konformationswechsel freigeben.

Auch Figur 12 gibt eine schematisierte "Ribbon"-Darstellung einer erfindungsgemäßen Kristallform mit ihrer dreidimensionalen Struktur wieder, und zwar fokus-siert auf einen Ausschnitt der großen Untereinheit von humanem m-Calpain in Abwesenheit von Ca-Ionen. Die Domäne III (in blau) zeichnet sich durch β-Faltblatt-Strukturen mit jeweils gegenläufig ausgerichteten β-Faltblattsträngen aus, wobei insbesondere in der vorliegenden Abbildung auf den Kontaktbereich der Domäne III mit den Strukturelementen der Sub-Domänen IIa und IIb abgestellt wird. In gelb sind in Figur 12 daher Teile der Helices α₅II und α₆II bzw. die Helix α₇II der Domäne IIb (in rot) mit der Positionierung von Seitenketten ausgewählter Aminosäuren dargestellt. Insbesondere ist die Anordnung der sauren Aminosäuren in der sog. sauren "Schleife" der Domäne III hervorgehoben. Diese Ladung wird, wie aus Figur 12 deutlich erkennbar, zumindest tw. durch positive Ladungen und/oder Teilladungen von Seitenketten der in der Helix α₇II angeordneten Aminosäuren kompensiert. In der Mitte der Figur 12 sind jene Aminosäure-Seitenketten mit entsprechender Konformation abgebildet, die an hydrophoben oder polaren Wechselwirkungen im Grenzbereich zwischen den Sub-Domänen IIa und IIb bzw. der Domäne III beteiligt sind. Auf der linken Seite der Figur 12 (ebenfalls entsprechend der Referenzposition der Figur 11) wird durch die Abbildung der basischen Aminosäuren der Charakter der basischen Schleife der Domäne III dargestellt.

Die vorliegende Erfindung wird durch das nachfolgende Ausführungsbeispiel näher erläutert.

### 1. Überexpression, Reinigung und Charakterisierung von humanem m-Calpain

Humanes m-Calpain voller Länger, das am N-Terminus der großen Untereinheit die Sequenz Gly-Arg-Arg-Asp-Arg-Ser aufweist, worauf die native Sequenz, beginnend mit Metl, folgt, wurde in einem Baculovirus-Expressionssystem ex-primiert und gereinigt, wie es dem Stand der Technik entspricht, und sich detailliert bei Masumoto et al. (J. Biochem. 124, 957-961, 1998) beschrieben findet. Der Inhalt der vorzitierten Veröffentlichung ist auch vollständig Bestandteil der Offenbarung der vorliegenden Erfindung. Insbesondere sind die bei Masumoto et al. zu den Materialien, zur Präparation des Baculovirus-Transfervektors mit humanem Calpain der großen bzw. der kleinen Untereinheit, zu den Zellkulturen, zur Präparation des rekombinanten Virus, zur Expression von humanem m-Calpain und zur Reinigung des rekombinaten humanen m-Calpains und zu allen weiteren experimentellen Vorge-hendsweisen zur Überexpression, Reinigung und Charakterisierung von humanem m-Calpain offenbarten Angaben Bestandteil der Beschreibung der vorliegenden Erfindung.

### 2. Proteinkristallisierung

Vor der Kristallisierung wurde das Protein bis zu einer Konzentration von näherungsweise 14 mg/ml aufkonzentriert, und es wurde der Puffer gegen 10 mM Tris/HCL pH 7,5, 50 mM NaCl, 1 mM EDTA und 1 mM DTE gewechselt. Hierbei wurde die Proteinkonzentration durch Absorptionsspektroskopie unter Verwendung eines molaren Extinktionskoeffizienten bei 280 nm von A=17,2 (10mg⁻¹ ml⁻¹ cm⁻¹⁾ bestimmt. Bei 20% Polyethylenglykol (PEG) 10000, 0,1 M He-pes/NaOH (pH 7,5) wurden kleine Kristalle mit maximal 100 µm in der längsten Abmessung beobachtet. Allerdings ergab sich auch unter diesen Bedingungen nur in 5 bis 10% der Versuchsansätze ein derartiges Kristallwachstum. Durch Hinzufügung von Isopropanol und Guanidiniumchlorid konnten die Kristallgröße und das Kristallwachstum verbessert werden. Gleichwohl konnten Kristalle, die eine für die Röntgenstrukturanalyse ausreichende Größe aufwiesen, nur durch sogenanntes "Makro-Seeding" der kleinen Kristalle unter Verwendung der Technik des hängenden und sitzenden Tropfens mit entsprechenden Dampfdiffusionsmethoden erhalten werden. Tropfen von 6,6 µl, bestehend auf 4 µl der Proteinlösung, 2 µl der Präzipitanzlösung (100 mM He-pes/NaOH pH 7,5, 15 % PEG 10000, 2,2 % Isopropanol und 0,6 µl von 1 M Guanidiniumchlorid wurden gegen 400 µl der Präzipitanzlösung bei 20° C ins Gleichgewicht gebracht.

Hierbei wurden zwei verschiedene Kristalltypen erhalten, wobei beide die Raumgruppe P2₁ aufwiesen.

### 3. Röntgendiffraktion

Die gezüchteten Kristalle waren ungeeignet für eine Expo-sition gegenüber Röntgenstrahlen bei Raumtemperatur. Daher wurde ein entsprechender Kryo-Schutzpuffer eingesetzt. Hierzu wurden die Kristalle aus den Tropfen mit Hilfe einer "Schleife", als "Kryo-Loop" bezeichnet, entnommen und in 20 µl des Reservoirpuffers tranferiert. 10 µl von 88%igem Glycerin wurden langsam hinzugefügt, um den Kristall an die Kryo-Puffer-Bedingungen zu gewöhnen. Die equilibrierten Kristalle wurden in Röhrchen gesetzt und mit einem Gasfluß aus einem Kryostaten mit flüssigem Stickstoff blitzartig abgekühlt (Oxford Cryo Systems). Die Röntgenbeugungsmuster wurden bei 100 K auf einem MARCCD-Detektor an einer BW6-"Beamline" des deutschen Elektron-Synchrotrons in Hamburg (DESY) unter Verwendung von monochromatischem Röntgenlicht nach Standardverfahren gesammelt. Eine entsprechende Beschreibung der Standardverfahren befindet sich bei Helliwell (Macromolecular Crystallography with Synchrotron Radiation, Cambridge University Press, Cambridge 1992), die gänzlich Bestandteil der vorliegenden Offenbarung ist.

### 4. Bestimmung der Einheitszellkonstanten und der Auflösung

Für die beiden während der Kristallisierung erhaltenen Kristallarten konnte ein divergentes Auflösungsvermögen bestimmt werden. Während sich für die Kristalltyp 1 Reflexe bis zu einer Auflösung von 2,8 Å beobachten ließen, ergab sich für die Kristalltyp II ein Auflösungsvermögen von max. 2,1 Å. Nach der Sammlung der Daten des Diffraktionsmusters konnten die Zellkonstanten der Einheitszelle, nämlich die Dimensionen der Einheitszelle und die Winkel in der Einheitszelle, sowie die Orientierung der Kristalle bestimmt werden. Daraus ergab sich auch die Symmetrie der Einheitszelle.

### (a) Kristalltyp 1

Die Kristalltyp 1 stellt sich makroskopisch als ein rhom-busartiges Plättchen dar und weist die Zellkonstanten a=64,78 Å, b=133,25Å, c=77,53 Å und β=102,07° auf. Es handelt sich bei diesen Kristallen um solche mit monokli-ner Raumgruppe P2₁. Die Kristalle wachsen bis zu einer maximalen Größe von 1,0mm x 1,0 mm x 0,1 mm. Es wurde ein 3,0 Å-Datensatz mit über 360 Stellungen (Expositionszeit jeweils 50 sec) gesammelt. Der Vₘ-Wert beträgt 3,4 Å³/Da. Zur näheren Erläuterung dieser Angabe wird hierfür auf Matthews (J. Mol. Biol. 33, 491-497, 1968) verwiesen. Es handelt sich um ein Heterodimer in der asymmetrischen Einheit, entsprechend einem Lösungsmittelanteil von 61 Volumen-%. Insgesamt wurden 223726 Reflexe aufgezeichnet, was wiederum 25010 Individualreflexen ("Unique reflecti-ons") entsprach (R_{merge} = 5,6%) entspricht. Es läßt sich errechnen, daß 96,9% der theoretisch möglichen Indivi-dualreflexe gesammelt wurden. Weitere Angaben zu Kristalltyp 1 finden sich in Tabelle 1.

### (b) Kristalltyp 2

Der Kristalltyp 2 hat ein plättchen- bis prismaähnliches Erscheinungsbild, mit Einheitszellkonstanten von a=51,88 Å, b=169,84 Å, c=64,44 Å und β=95,12°. Die Kristalle wachsen bis zu einer maximalen Größe von 1,0 mm x 0,2 mm x 0,1 mm und zeigen eine Auflösung bis zu 2,1 Å. Weitere Angaben zu Kristalltyp 2 sind in Tabelle 1 dargestellt und folgen unter 5..

### 5. Gewinnung der Phaseninformation

Da zur Berechnung von Elektronendichtekarten Strukturfak-toren mit Amplituden- und Phaseninformation herangezogen werden müssen, das Beugungsmuster aber nur die Amplitu-deninformation anhand der für jeden Reflex gemessenen Intensität liefert, müssen Methoden zur Ermittlung der Phaseninformation eingesetzt werden. Im vorliegenden Fall wurden hierfür Schwermetallatom-Derivate herangezogen und die Methode der multiplen anormalen Dispersion (MAD: Hendrickson & Latman, Acta Crystallographica B26, 136-143, 1970; Hendrickson & Teeter, Nature 290, 107-113, 1981; Bijvoet, Nature 173, 888-891, 1954) eingesetzt. Im Rahmen der MAD-Messungen wurden Daten für den Goldderi-vatkristall bei Wellenlängen von λ=1,0092 Å (Absorptionskante von Gold), λ=1,004 Å und bei der weiter entfernten Wellenlänge von λ=1,100 Å gesammelt. Auch für den Quecksilberderivat-Kristall wurden drei MAD-Messungen bei verschiedenen Einstrahl-Wellenlängen vorgenommen (λ=1,0399 Å (Absorptionskante von Quecksilber), λ=1,0392 Å und bei der weiter entfernten Wellenlänge von λ=1,100 Å. Im vorliegenden Fall wurde die Phaseninformation aus den Messungen für das Quecksilber-Derivats mit der Amplitu-deninformation des Goldderivats zur Berechnung einer Elektronendichtekarte für die Kristallform kombiniert.

Um Kristalle zu erhalten, die Kristallformen mit eingelagerten Schwermetall-Ionen aufwiesen, wurden Gold- und Quecksilber-Derivate durch sogenanntes "Soaking" von nativen Kristallen mit 5 mM Goldtriethylphosphin bzw. mit Phenylquecksilber hergestellt.

### (a) Goldderivat

Kristalle mit Kristallformen mit eingelagertem Goldderivat streuten bis zu einer Auflösung von 2,3 Å. Ein entsprechender Datensatz mit 330 Stellungen (Expositionszeit 50 sec pro Stellung) wurde gesammelt. Nach Maßgabe des VₘWertes von 2,61 Å³/Da wurde ein Heterodimer pro asymmetrischer Einheit in den Kristallen bestimmt, was wiederum einem Lösungsmittelanteil von 53 Volumen-% im Kristall entspricht. Insgesamt wurden 400860 Reflexe aufgezeichnet und diese zu 47236 Individualreflexen (R_{merge} = 4,5 %) zu-sammengefaßt, was 94,8 % der theoretisch möglichen Reflexe entspricht (Tabelle 1). Fünf Goldpositionen wurden in den anormalen Differenz-Pattersonkarten identifiziert. Die Positionen für das Gold- ebenso wie für das Quecksil-ber-Derivat wurden mit dem Programm MLPHARE aus dem CCP4-Programm-paket (s. Zitat a.a.O) verfeinert.

### (b) Das Phenylquecksilber-Derivat

Bei diesem Derivat wurden Reflexe bis 2,4 Å gesammelt. Für das Phenylquecksilber-Derivat wurden 421730 Reflexe mit DENZO (Otwinowski und Minor, 1993, DENZO: Film Processing for Macromolecular Crystallography, Yale University, New Haven, Connecticut) indiziert, skaliert und zu 48363 Individualreflexen (R_{merge} = 4,7 %), d.h. 93% aller möglichen Individualreflexe, zusammengefaßt. Es wurden hierbei drei Positionen für Quecksilber in den anormalen Differenz-Pattersonkarten ("anomalous") bestimmt. Außerdem erfolgte eine Reduzierung mit SCALEPACK (Otwinowski und Minor, 1993, s.o.). Die Daten wurden wei-terprozessiert mit den entsprechenden Programmen der CCP4 Programmfolge (Collaborative Computational Project No. 4, 1994, Acta Crystallog., Sec. D50, 760-763.)

### 6. Bau des Strukturmodells:

Für den Bau des Strukturmodells wurden die Strukturfak-toramplituden des Goldderivats mit den Phasen des Phenyl-quecksilber-Derivats versehen, wodurch eine Elektronendichtekarte für das Goldderivat errechnet werden konnte, die eine gute Konturierung bis zu einer Auflösung von 2,4 Å zeigte. Es wurde die Methode nach La Fortelle und Bri-cogne [Methods Enzymol. 276, 472-494 (1997), (SHARP)] eingesetzt. Auf diese Weise konnten 90% der Aminosäuren des kristallisierten Proteins in die Elektronendichtekarte modelliert werden. Hierbei sind die ersten 84 Aminosäuren der kleinen Untereinheit nicht berücksichtigt, da keine ausreichende Elelktronendichte beobachtet werden konnte. Das Modellieren der beiden Aminosäureketten, d.h. Polypetide, der großen und der kleinen Untereinheit in die Elektronendichtekarte erfolgte an einer Silicon Gra-phics "Workstation" (Indigo) mit Hilfe des Software-Pakets TurboFRODO (Roussel und Cambileau, Silicon Gra-phics, Mountainview, Kalifornien, USA (1998)).

### 7. Verfeinerung des Strukturmodells

Dieses modellierte Teilmodell wurde kristallographisch verfeinert. Hierzu wurde das Proteinmodell mit Hilfe einer verbesserten phasenkombinierten Fourier-Transformation vervollständigt. Zur Berechnung der Elektronendichtekarten und zur Durchführung der kristallographischen Verfeinerung wurden die Software-Pakete REFMAC aus der CCP4-Programmreihe und X-PLORE (Brünger, X-PLORE Version 3.1, A System for X-Ray Crystallography and NMR Spectroscopy, Yale University Press, New Haven, Connecticut (1993)) eingesetzt. Um das kristallographische Modell zu vervollständigen, wurden schließlich Wassermoleküle in die Elektronendichtekarte eingesetzt und die individuellen atomaren Temperaturfaktoren verfeinert. Im Endmodell wurde ein R-Faktor von 20,6%, freier R-Faktor = 26,6%) für 38.544 Reflexe erreicht. In diesem Endmodell waren die Aminosäuren Ala2-Leu700 der großen Untereinheit sowie die Aminosäuren Thr85-Ser268 der kleinen Untereinheit wohl definiert. Das Endmodell zeigte 7097 Atome (außer Wasserstoffatomen), 5 Goldionen und 352 Wassermo-leküle. Die abschließenden Standard(rms)-Abweichungen von den entsprechenden Standardbindungslängen und Standard-winkeln betrugen 0,007 Å und 1,179°. 85% aller Hauptket-ten-Torsionswinkel liegen in den erlaubten oder erweiterten erlaubten Bereichen der Ramachandran-Auftragung.

**Tabelle 1:**

| **Konstante** | **Kristalltyp I** | **Kristalltyp II** |
|---|---|---|
| | | (Gold-Derivat) |
| Raumgruppe | P2₁ | P2₁ |
| Kristallmorphologie | Plättchen | Stab/rechtwinklige Würfel |
| Einheitszellkonstanten | a = 64,78 Å | a = 51,88 Å |
| | b = 133,25 Å | b = 169,84 Å |
| | c = 77,53 Å | c = 64,44 Å |
| | β = 102,07° | β = 95,12° |
| Vₘ(Å³Da⁻¹) | 3,14 | 2,61 |
| Heterodimere pro asymmetrischer Einh. | 1 | 1 |
| Geschätzter Lösungsmittelanteil (%) | 61 | 53 |
| Beugungsgrenze (Ä) | 3,0 | 2,3 |
| Rotationswinkel zwischenden Expositions stellungen | 0,5 | 0,4 |
| Expositionszeit pro Stellung | 50 s | 50 s |
| Gesamtrotation des Kristalls bei der Datensammlung | 180° | 132° |
| Zahl der gemessenen Reflexe | 223726 | 400680 |
| Zahl der Individual reflexe | 25010 | 47236 |
| ^{R}merge^{a} | 0,056 | 0,045 |
| Vollständigkeit des Datensatzes | 96,9% | 94,8% |
| Vollständigkeit des Datensatzes in der äußersten Kalotte des inversen Raums | 95,2% | 92,2% |

## Patentansprüche

1. Raumform mindestens eines Polypeptids, dadurch gekennzeichnet, daß mindestens ein Polypeptid in der Raumform mindestens eine an der Katalyse beteiligte (Sub)-Domäne eines Proteins aus der Familie der neutralen Ca-aktivierten Cystein-Proteinasen (Calpaine) enthält.

2. Raumform mindestens eines Polypeptids nach Anspruch 1, dadurch gekennzeichnet, daß die neutrale Ca-aktivierte Cystein-Proteinase ausgewählt ist aus der Gruppe, bestehend aus Isozymen aus der Familie der ubiqitär exprimierten Calpaine und aus Isozymen aus der Familie der Gewebe-spezifisch exprimierten Calpaine (n-Calpaine).

3. Raumform mindestens eines Polypeptids nach Anspruch 1 oder 2, dadurch gekennzeichnet, daß die neutrale Ca-aktivierte Cystein-Proteinase ein Isozym aus der Gruppe der m- oder µ-Calpaine ist.

4. Raumform mindestens eines Polypeptids nach einem der vorgenannten Ansprüche, dadurch gekennzeichnet, daß das Calpain humanen Ursprungs ist.

5. Raumform mindestens eines Polypeptids nach einem der vorgenannten Ansprüche, dadurch gekennzeichnet, daß mindestens ein Polypeptid der Raumform die Aminosäuresequenz der Sub-Domäne IIa und/oder die Aminosäuresequenz der Sub-Domäne IIb eines m-Calpains enthält.

6. Raumform mindestens eines Polypeptids nach einem der vorgenannten Ansprüche, dadurch gekennzeichnet, daß mindestens ein Polypeptid der Raumform die Aminosäuresequenz der (Sub)-Domänen IIa oder IIb, III und/oder IV eines Calpains enthält.

7. Raumform nach einem der vorgenannten Ansprüche, dadurch gekennzeichnet, daß die Raumform eine Kristallform ist, wobei die Kristallform mindestens ein Polypeptid, enthaltend mindestens eine an der Katalyse beteiligte (Sub)-Domäne eines Proteins aus der Familie der neutralen Ca-aktivierten Cystein-Proteinasen (Calpaine), pro asymetrischer Einheit aufweist.

8. Kristallform mindestens eines Polypeptids pro asymmetrischer Einheit nach Anspruch 7, dadurch gekennzeichnet, daß die Kristallform Metall-Ionen enthält.

9. Kristallform mindestens eines Polypeptids pro asymmetrischer Einheit nach einem Ansprüche 7 oder 8, dadurch gekennzeichnet, daß die Kristallform Ca- und/oder Schwermetall-Ionen enthält.

10. Kristallform mindestens eines Polypeptids pro asymmetrischer Einheit nach einem der vorgenannten Ansprüche 7 bis 9, dadurch gekennzeichnet, daß die Metall-Ionen in der räumlichen Nachbarschaft von Cystein- oder Histidinresten mindestens eines Polypeptids angelagert sind.

11. Kristallform mindestens eines Polypeptids pro asymmetrischer Einheit nach einem der vorgenannten Ansprüche 7 bis 10, dadurch gekennzeichnet, daß die Kristallform mindestens eine Verbindung, ausgewählt aus der Gruppe, bestehend aus Substrat-, Pseudosubstrat-, Aktivator- und Inhibitor-Molekülen, enthält.

12. Kristallform mindestens eines Polypeptids pro asymmetrischer Einheit nach Anspruch 11, dadurch gekennzeichnet, daß die Verbindung ein Di- oder Oligopeptid ist.

13. Kristallform mindestens eines Polypeptids pro asymmetrischer Einheit nach Anspruch 11 oder 12, dadurch gekennzeichnet, daß die Verbindung ein chemisch modifiziertes Di- oder Oligopeptid ist.

14. Kristallform mindestens eines Polypeptids pro asymmetrischer Einheit nach einem der vorgenannten Ansprüche 7 bis 13, dadurch gekennzeichnet, daß die Kristallform zwei verschiedene Polypeptide als Heterodimer in der asymmetrischer Einheit aufweist.

15. Kristallform mindestens eines Polypeptids pro asymmetrischer Einheit nach einem der vorgenannten Ansprüche 7 bis 14, dadurch gekennzeichnet, daß mindestens ein Polypeptid eine Aminosäuresequenz, wie in Figur 3, 4, 5 oder 6 dargestellt, enthält.

16. Kristallform mindestens eines Polypeptids pro asymmetrischer Einheit nach einem der vorgenannten Ansprüche 7 bis 15, dadurch gekennzeichnet, daß die asymmetrische Einheit ein Heterodimer aufweist, das ein Polypeptid (1) mit einer Aminosäuresequenz, wie in Figur 3 dargestellt, und ein Polypeptid (2) mit einer Aminosäuresequenz, wie in Figur 4 dargestellt, enthält.

17. Kristallform mindestens eines Polypeptids pro asymmetrischer Einheit nach einem der vorgenannten Ansprüche 7 bis 16, dadurch gekennzeichnet, daß die Raumgruppe des die Kristallform aufweisenden Kristalls monoklin, tetragonal, orthorhombisch, kubisch, triklin, hexagonal oder trigonal/rhombo-hedral ist.

18. Kristallform mindestens eines Polypeptids pro asymmetrischer Einheit nach einem der vorgenannten Ansprüche 7 bis 17, dadurch gekennzeichnet, daß die Raumgruppe des die Kristallform aufweisenden Kristalls P2₁ ist.

19. Kristallform mindestens eines Polypeptids pro asymmetrischer Einheit nach einem der vorgenannten Ansprüche 7 bis 18, dadurch gekennzeichnet, daß die Einheitszelle des die Kristallform enthaltenden Kristalls Zellkonstanten von ungefähr a = 64,9 Å, b = 134,0 Å, c = 78,0 Å und β = 102,4° oder a = 51,8 Å, b = 171,4 Å, c = = 64,7 Å und β = 94,8° (innerhalb der aufweist.

20. Kristallform mindestens eines Polypeptids pro asymmetrischer Einheit nach einem der vorgenannten Ansprüche 7 bis 19, dadurch gekennzeichnet, daß die an der Katalyse beteiligte Calpain-Sub-Domäne IIa (Sequenzabschnitt T93 bis G209) und/oder IIb (Sequenzabschnitt G210 bis N342) des mindestens einen Polypeptids pro asymmetrischer Einheit die Strukturkoordinaten nach Figur 10 für die vorgenannten Aminosäuren aufweist.

21. Kristallform mindestens eines Polypeptids pro asymmetrischer Einheit nach einem der vorgenannten Ansprüche 7 bis 20, dadurch gekennzeichnet, daß mindestens ein Polypeptid pro asymmetrischer Einheit die Strukturkoordinaten nach Figur 10 für die große Untereinheit (A2 bis L700) aufweist.

22. Kristallform mindestens eines Polypetids pro asymmetrischer Einheit nach einem der vorgenannten Ansprüche 7 bis 21, dadurch gekennzeichnet, daß mindestens ein Polypeptid pro asymmetrischer Einheit die Strukturkoordinaten nach Figur 10 für die große Untereinheit (A2 bis L700) und mindestens ein anderes Polypeptid die Strukturkoordinaten nach Figur 10 für die kleine Untereinheit (T85 bis S268) aufweist.

23. Kristallform mindestens eines Polypetids pro asymmetrischer Einheit nach einem der vorgenannten Ansprüche 7 bis 22, dadurch gekennzeichnet, daß der Kristall mit der Kristallform durch Röntgenstrukturanalyse Reflexe bis zu einem Bragg-Index von mindestens d = 3,0 Å streut.

24. Verbindung, mit der Eigenschaft, als Substrat, Pseu-do-Substrat, Aktivator oder Inhibitor einer neutralen Ca-aktivierten Cystein-Proteinase (Calpain) zu wirken, dadurch gekennzeichnet, daß die Verbindung in Wechselwirkung mit den Haupt- und/oder Seitenketten von Aminosäuren der katalytischen Domäne oder von Aminosäuren eines für die Regulation des aktiven Zentrum relevanten Abschnitts mindestens eines Polypeptids der Kristallform treten.

25. Verbindung nach Anspruch 24, dadurch gekennzeichnet, daß die Verbindung mit der Struktur der Haupt-und/oder Seitenketten der katalytischen Domäne oder eines für die Regulation des aktiven Zentrum relevanten Abschnitts mindestens eines Polypeptids in einer Raum- oder Kristallform, wie gemäß einem der Ansprüche 1 bis 23 erhalten, in Wechselwirkung treten.

26. Verbindung nach Anspruch 24 oder 25, dadurch gekennzeichnet, daß die Verbindung mit mindestens einer Aminosäure des Sequenzabschnitts β2tβ3, des sauren Loops", des mindestens einen Polypeptids in einer Raum- oder Kristallform, wie gemäß einem der Ansprüche 1 bis 23 erhalten, in Wechselwirkung tritt.

27. Verbindung nach Anspruch 26, dadurch gekennzeichnet, daß die Verbindung mindestens eine positive Ladung und/oder mindestens eine positive Teilladung aufweist und eine Interaktion zwischen der α₇II-Helix und dem Sequenzabschnitts β2tβ3 im wesentlichen verhindert.

28. Verbindung nach Anspruch 26 oder 27, dadurch gekennzeichnet, daß die Verbindung ein Aktivator der katalytischen Aktivität eines Calpains ist.

29. Verbindung nach Anspruch 24 oder 25, dadurch gekennzeichnet, daß die Verbindung die Wechselwirkung zwischen dem Abschnitt α₇II-Helix und dem Sequenzabschnitt β2tβ3 des mindestens einen Polypeptids in einer Raum- oder Kristallform, wie gemäß einem der Ansprüche 1 bis 23 erhalten, im wesentlichen verstärkt.

30. Verbindung nach Anspruch 29, dadurch gekennzeichnet, daß die Verbindung mit mindestens einer der Aminosäuren 226L, 230L, 234L, 354L, 355L und/oder 357L in Wechselwirkung tritt.

31. Verbindung nach Anspruch 29 oder 30, dadurch gekennzeichnet, daß die Verbindung ein Inhibitor der katalytischen Aktivität des Polypeptids ist.

32. Verbindung nach Anspruch 24 oder 25, dadurch gekennzeichnet, daß die Verbindung mit mindestens einer der Aminosäuren der Sub-Domäne(n) IIa und/oder IIb interagiert.

33. Verbindung nach Anspruch 32, dadurch gekennzeichnet, daß der Inhibitor die Rotations- und/oder Translationsbewegung Sub-Domäne IIb gegenüber der Sub-Domäne IIa blockiert, indem er sich in die Furche zwischen den beiden Sub-Domänen einlagert.

34. Verfahren zur Identifizierung einer Verbindung mit der Eigenschaft, als Substrat, Pseudo-Substrat, Aktivator oder Inhibitor einer neutralen Ca-aktivierten Cystein-Proteinase (Calpain) zu wirken, dadurch gekennzeichnet, daß die
(a) eine Raum- oder Kristallform nach einem der Ansprüche 1 bis 23 erhalten wird,
(b)die Strukturkoordinaten der Raum- oder Kristallform dreidimensional dargestellt werden,
(c) sterische Eigenschaften und/oder funktio nelle Gruppen einer Verbindung so gewählt werden, daß Wechselwirkungen zwischen der Verbindung und den Haupt- und/oder Seitenketten des Polypeptids in dem Bin dungsbereich entstehen und
(d) die gemäß (c) erhaltene Verbindung in das aktive Zentrum der katalytischen Sub-Domäne(n) oder in einen für die Regulation des aktiven Zentrum relevanten Abschnitt des Polypeptids eingefügt wird.

35. Verfahren nach Anspruch 34, dadurch gekennzeichnet, daß in einem Verfahrensschritt (c1) die dreidimensionale Struktur der Verbindung ermittelt wird.

36. Verfahren nach Anspruch 34 oder 35, dadurch gekennzeichnet, daß in einem Verfahrensschritt (d1) die Stärke der Wechselwirkung zwischen der Verbindung und mindestens einem Polypeptid, wie gemäß einer Raum- oder Kristallform nach einem der Ansprüche 1 bis 23 erhalten, bestimmt wird.

37. Verfahren nach einem der Ansprüche 34 bis 36, dadurch gekennzeichnet, daß gemäß Verfahrensschritt (b) alle oder ein Teil der Strukturkoordinaten aus Figur 10 dargestellt werden.

38. Verfahren nach einem der Ansprüche 34 bis 37, dadurch gekennzeichnet, daß die Verfahrensschritte (c), (c1), (d) und (d1) zyklisch so lange wiederholt werden, bis die gemäß (d1) erhaltene Stärke der Wechselwirkung zwischen Verbindung und der Haupt-und/oder Seitenkette des mindestens einen Polypeptids in einer Raum- oder Kristallform, wie gemäß einem der Ansprüche 1 bis 23 erhalten, optimiert wird.

39. Verfahren nach einem der Ansprüche 34 bis 38, dadurch gekennzeichnet, daß in einem Verfahrensschritt (d2) die Eigenschaften der Verbindung in einem biologischen Testsystem ermittelt werden.

40. Verfahren zur Identifizierung einer Verbindung, mit der Eigenschaft als Substrat, Pseudosubstrat, Aktivator oder Inhibitor einer neutralen Ca-aktivierten Cystein-Proteinase (Calpaine) zu wirken, dadurch gekennzeichnet, daß
(a) ein biologische Testsystem für ein Substrat, Pseudosubstrat, Aktivator und/oder Inhibitor von Calpain etabliert wird,
(b) eine als Substrat, Pseudosubstrat, Aktivator und/oder Inhibitor von Calpain wirkende Verbindung durch ein biologisches Testsystem gemäß (a) ermittelt wird,
(c) die Konformation der Verbindung bestimmt wird,
(d) die Strukturkoordinaten mindestens eines Polypeptids aus einer Raum- oder Kristallform nach einem der Ansprüche 1 bis 23 dargestellt werden und
(e) die gemäß (b) und (c) erhaltene Struktur der Verbindung in die gemäß (d) erhaltene Struktur des aktiven Zentrums der katalytischen Sub-Domäne(n) oder eines für die Regulation des aktiven Zentrum relevanten Abschnitts des Polypeptids eingefügt wird.

41. Verfahren nach Anspruch 40, dadurch gekennzeichnet, daß in einem Verfahrensschritt (e1) Art und/oder Stärke der Wechselwirkung zwischen der Verbindung und der Raum- oder Kristallform mindestens eines Polypeptids bestimmt werden.

42. Verbindung als Substrat, Pseudosubstrat, Aktivator oder Inhibitor, dadurch gekennzeichnet, daß sie aus einem Verfahren nach einem der Ansprüche 34 bis 41 erhalten wird.

43. Verfahren zur Herstellung einer Kristallform mindestens eines Polypeptids nach einem der Ansprüche 1 bis 23, dadurch gekennzeichnet, daß
(a) das Polypeptid in einem Expressionssystem überexprimiert wird,
(b) das gemäß (a) erhaltene Polypeptid in einem geeigneten Puffersystem gelöst wird und
(c) die Kristallisierung durch bspw. Dampfdiffusi-onsverfahren eingeleitet wird.

44. Verfahren zur Darstellung einer 3-dimensionalen Struktur eines Polypeptids oder eines Komplexes unbekannter Struktur, enthaltend mindestens ein Polypeptid, das mindestens eine an der Katalyse beteiligte Domäne eines Proteins aus der Familie der neutralen Ca-aktivierten Cystein-Proteinasen (Calpaine) enthält, dadurch gekennzeichnet, daß die unbekannte Struktur des Polypeptids oder Komplexes auf der Basis einer bekannten Raum- oder Kristallform nach einem der Ansprüche 1 bis 23 ermittelt wird.

45. Verfahren nach Anspruch 44, dadurch gekennzeichnet, daß die Strukturkoordinaten, wie in Figur 10 dargestellt, eingesetzt werden.

46. Verfahren nach Anspruch 44 oder 45, dadurch gekennzeichnet, daß
(a) die Primärsequenz eines Polypeptid unbekannter 3D-Struktur mit der Primärsequenz eines Polypeptids bekannter Kristallform verglichen wird,
(b) die 3D-Struktur des Polypeptids unbekannter Struktur in Anlehnung an die Kristallform homologer Abschnitte modelliert wird, und
(c) mit Hilfe entsprechender Computer-Programme energetische Optimierungen der gemäß (b) modellierten Struktur durchgeführt werden.

47. Verfahren nach einem der Ansprüche 44 bis 46, dadurch gekennzeichnet, daß das Polypeptid unbekannter Struktur ein Isozym aus der Familie der n-Calpaine oder ein Isozym eines m- oder µ-Calpains ist.

48. Verfahren zur Identifizierung eines Substrats, Pseu-dosubstrats, Aktivators oder Inhibitors einer neutralen Ca-aktivierten Cystein-Proteinase (Calpain) unbekannter 3D-Struktur, dadurch gekennzeichnet, daß
(a) die unbekannte 3D-Struktur des Polypeptids nach einem Verfahren nach einem der Ansprüche 44 bis 47 ermittelt wird und
(b) mit Hilfe eines Verfahrens nach einem der Ansprüche 34 bis 41 eine Verbindung, mit der Eigenschaft als Inhibitor, Pseudosubstrat, Aktivator oder Substrat des Polypeptids unbekannter 3DStruktur zu wirken, ermittelt wird.

49. Verwendung von Inhibitoren und/oder Aktivatoren der katalytischen Aktivität einer neutralen Ca-aktivierten Cystein-Proteinase (Calpain) nach einem der Ansprüche 24 bis 33, Anspruch 42 oder erhalten aus einem Verfahren nach Anspruch 48 zur Verwendung als Arzneimittel.

50. Verwendung von Verbindungen nach Anspruch 49 zur Behandlung von ischämischen Zuständen, Muskeldystrophie und/oder Tumorerkrankungen.
